(19) Europäisches Patentamt
European Patent Office
Office européen des brevets

(11) **EP 4 314 339 B1**

(12) **EUROPEAN PATENT SPECIFICATION**

(45) Date of publication and mention of the grant of the patent:
**18.06.2025 Bulletin 2025/25**

(21) Application number: **23717230.9**

(22) Date of filing: **04.04.2023**

(51) International Patent Classification (IPC):
***C12Q 1/6869*** *(2018.01)*

(52) Cooperative Patent Classification (CPC):
(C-Sets available)
**C12Q 1/6855; C12Q 1/6869** (Cont.)

(86) International application number:
**PCT/GB2023/050891**

(87) International publication number:
**WO 2023/194714 (12.10.2023 Gazette 2023/41)**

# (54) CHIMERIC ARTEFACT DETECTION METHOD

CHIMÄRES ARTEFAKTDETEKTIONSVERFAHREN

PROCÉDÉ DE DÉTECTION D'ARTÉFACT CHIMÉRIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **04.04.2022 GB 202204903**

(43) Date of publication of application:
**07.02.2024 Bulletin 2024/06**

(73) Proprietor: **Oxford University Innovation Limited**
**Oxford, Oxfordshire OX2 0JB (GB)**

(72) Inventors:
- **PHILPOTT, Martin**
  **Oxford Oxfordshire OX2 0JB (GB)**
- **CRIBBS, Adam**
  **Oxford Oxfordshire OX2 0JB (GB)**
- **OPPERMANN, Udo**
  **OX2 0JB Oxford (GB)**

(74) Representative: **J A Kemp LLP**
**80 Turnmill Street**
**London EC1M 5QU (GB)**

(56) References cited:
**WO-A1-2017/079593**
**WO-A1-2020/136438**
**WO-A1-2021/247593**
**WO-A1-2022/118027**
**WO-A2-2005/003291**

- **LEBRIGAND KEVIN ET AL: "High throughput error corrected Nanopore single cell transcriptome sequencing", NATURE COMMUNICATIONS, vol. 11, no. 1, 12 August 2020 (2020-08-12), XP093047897, Retrieved from the Internet <URL:https://www.nature.com/articles/s41467-020-17800-6.pdf> DOI: 10.1038/s41467-020-17800-6**
- **KARST SØREN M ET AL: "High-accuracy long-read amplicon sequences using unique molecular identifiers with Nanopore or PacBio sequencing", NATURE METHODS, vol. 18, no. 2, 11 January 2021 (2021-01-11), pages 165 - 169, XP037359604, ISSN: 1548-7091, DOI: 10.1038/S41592-020-01041-Y**
- **LAURAE MACCONAILL ET AL: "Unique, dual-indexed sequencing adapters with UMIs effectively eliminate index cross-talk and significantly improve sensitivity of massively parallel sequencing", BMC GENOMICS, BIOMED CENTRAL LTD, LONDON, UK, vol. 19, no. 1, 8 January 2018 (2018-01-08), pages 1 - 10, XP021252292, DOI: 10.1186/S12864-017-4428-5**
- **CATHERINE CATHERINE BURKE ET AL: "A method for high precision sequencing of near full-length 16S rRNA genes on an Illumina MiSeq", PEERJ, 20 September 2016 (2016-09-20), XP055493145, Retrieved from the Internet <URL:https://www.ncbi.nlm.nih.gov/pmc/articles/PMC5036073/pdf/peerj-04-2492.pdf> [retrieved on 20180717]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **DU MEIJIE ET AL: "Unique dual indexing PCR reduces chimeric contamination and improves mutation detection in cell-free DNA of pregnant women", TALANTA, ELSEVIER, AMSTERDAM, NL, vol. 217, 14 April 2020 (2020-04-14), XP086156529, ISSN: 0039-9140, [retrieved on 20200414], DOI: 10.1016/J.TALANTA.2020.121035**

Remarks:
The complete document including Reference Table(s) and the Sequence Listing(s) can be downloaded from the EPO website

(52) Cooperative Patent Classification (CPC): (Cont.)

C-Sets
**C12Q 1/6855, C12Q 2525/191, C12Q 2535/122, C12Q 2537/165, C12Q 2563/179;**
**C12Q 1/6869, C12Q 2525/191, C12Q 2535/122, C12Q 2537/165, C12Q 2563/179**

## Description

### Field of the invention

The disclosure relates to methods for detecting chimeric artefact polynucleotides produced during amplification of a mixed sample of polynucleotides and to associated methods of preparing a library of polynucleotides, libraries of polynucleotides produced by such methods, and sets of oligonucleotides and kits that are useful for performing the method of detecting chimeric artefacts.

### Background to the Invention

The inclusion of Unique Molecular Identifiers (UMI) in polynucleotide libraries creates a distinct identity for each input molecule. The UMIs are used to correct sampling and PCR amplification bias within both short-read and long-read sequencing experiments. However, PCR and sequencing errors make it difficult to distinguish true UMIs from artefactual ones. As such, bioinformatics methods have been established to try to account for these errors.

The use of UMI sequences pre-dates next-generation sequencing, but the first protocols to include UMIs prior to short-read sequencing library generation were for iCLIP, a method for studying RNA and protein interactions. Despite short-read sequencing having a reportedly low error rate (~0.1%), the inclusion of UMIs prior to library construction can improve accuracy across almost all next-generation sequencing methods, including bulk RNA sequencing, single-cell sequencing and genomic DNA sequencing. However, short-read Illumina sequencing is restricted to a maximum amplicon size of ~500bp, which limits long range and assay resolution information.

The advent of third generation sequencing technologies is revolutionising genomics research as they enable the exploration of genomes at unprecedented resolution. While long read sequencing technologies can sequence native RNA, they may require PCR-based library methods to overcome limitations on sample size. However, the relatively high error rates of Oxford Nanopore Technologies (ONT) (5-25%) and Pacific Biosciences (PacBio) (~13%) have made it difficult to identify true UMI sequences. Furthermore, the high levels of PCR cycles required to generate a long-read sequencing library increases PCR amplification bias, the formation of chimerical molecules (Brakenhoff, R.H. et al. Chimeric cDNA clones: a novel PCR artifact. Nucleic Acids Res 19, 1949 (1991)), and heteroduplex molecules (Ruano, G. & Kidd, K.K. Modeling of heteroduplex formation during PCR from mixtures of DNA templates. PCR Methods Appl 2, 112-116 (1992)). Only recently has it been shown possible to correct UMIs sequenced using long-read technologies. This can be accomplished at the single-cell level by sequencing using a combination of short read Illumina sequencing to identify consensus long-read UMIs, concatemeric consensus sequencing, or by incorporating dimer amidites into the UMI regions to aid error correction (Lebrigand, K. et al. High throughput error corrected Nanopore single cell transcriptome sequencing. Nat Commun 11, 4025 (2020); Philpott, M. et al. Nanopore sequencing of single-cell transcriptomes with scCOLOR-seq. Nat Biotechnol 39, 1517-1520 (2021)). However, both methods are still error prone and do not account for chimeric artefacts. More recently, the inclusion of recognisable internal patterns into UMIs have also been explored as a potential solution for obtaining high-accuracy genomic sequencing (Karst, S.M. et al. High-accuracy long-read amplicon sequences using unique molecular identifiers with Nanopore or PacBio sequencing. Nat Methods 18, 165-169 (2021)). While this approach has been shown to aid the analysis of metagenomic DNA, the method is not easily adaptable for transcriptomic analysis.

### Summary of the Invention

The technical information set out below may, in some respects, go beyond the disclosure of the invention, which is defined by the appended claims. The additional technical information is provided to place the invention in a broader technical context and to illustrate possible related technical developments. Such additional technical information which does not fall within the scope of the appended claims is not part of the invention. Accordingly, any aspects, embodiments and examples of the present disclosure which do not fall under the scope of the appended claims do not form part of the invention and are merely provided for illustrative purposes.

In a first aspect, the invention provides a method for detecting chimeric artefact polynucleotides produced during amplification of a mixed sample of RNA molecules, the method comprising (i) capturing RNA molecules of the sample on a set of capture polynucleotides, wherein the capture polynucleotides each comprise a sample RNA capture region, one of a mixed pool of first identifier sequences, and a PCR handle sequence; (ii) performing reverse transcription of captured sample RNA molecules using a template switch reverse polymerase and primed using the capture polynucleotides to generate cDNA polynucleotides having 3’ end non-templated nucleotides; (iii) annealing a set of template switch oligonucleotides (TSOs) to the 3’ end non-templated nucleotides, wherein the TSOs each comprise one of a mixed pool of second identifier sequences, wherein the first identifier sequences and/or the second identifier sequences each comprise a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two nucleotide substitutions; (iv) amplifying the cDNA using the capture polynucleotide PCR handle sequences and TSO PCR handle sequences to generate a library of amplified cDNA flanked by first and second identifier sequences; (v) sequencing the library; (vi) identifying pairings of first and second identifier sequences flanking the library cDNA molecules; (vii) counting the library cDNA molecules having identified pairings of first and second identifier sequences and identifying pairings that are under-represented in the library; and/or identifying pairings as mismatch pairings when they comprise (a) a first identifier sequence in common with another pairing in the library having a different second identifier sequence to the mismatch pairing; and/or (b) a second identifier sequence in common with another pairing in the library having a different first identifier sequence to the mismatch pairing; and (viii) identifying a library cDNA molecule that is (a) flanked by an underrepresented pairing of first and second identifier sequences; and/or (b) flanked by a mismatch pairing of first and second identifier sequences; as a chimeric artefact polynucleotide.

The methods of the invention make use of a library of polynucleotides generated from a mixed sample, wherein the library polynucleotides include an identifier sequence flanking the sample polynucleotide sequence at both ends.

In a further aspect, the invention provides a method of producing a library of cDNA molecules of mixed sequence from RNA molecules in a sample, the method comprising: (i) capturing RNA molecules of the sample on a set of capture polynucleotides, wherein the capture polynucleotides each comprise a sample RNA capture region, one of a mixed pool of first identifier sequences, and a PCR handle sequence; (ii) performing reverse transcription of captured sample RNA molecules using a template switch reverse polymerase and primed using the capture polynucleotides to generate cDNA polynucleotides having 3' end non-templated nucleotides; (iii) annealing a set of template switch oligonucleotides (TSOs) to the 3' end non-templated nucleotides, wherein the TSOs each comprise one of a mixed pool of second identifier sequences, wherein the first identifier sequences and/or the second identifier sequences each comprise a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two nucleotide substitutions; and (iv) amplifying the cDNA using the capture polynucleotide PCR handle sequences and TSO PCR handle sequences to generate a library of amplified cDNA flanked by first and second identifier sequences.

The libraries may further be sequenced.

In a further aspect, the invention provides a library produced by the method.

The invention makes use of a library of polynucleotides of mixed sequence from a sample. The sample polynucleotide sequences in the library are flanked by a first identifier sequence at one end and a second identifier sequence at the other end, wherein the identifier sequences are from a pool of mixed first identifier sequences and a pool of mixed second identifier sequences. Each identifier sequence comprises a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two, or at least three, nucleotide substitutions.

The methods described herein conveniently make use of sets of template switch oligonucleotides (TSOs) to provide an identifier sequence at one or more ends of the library polynucleotides. The inventors have developed in particular sets of identifier sequences, for inclusion for example in TSOs, that provide improved efficiency of sequencing error detection in the identifies sequences. Hence, in a further aspect, the invention provides an array of template switch oligonucleotides (TSOs). The TSOs comprise an identifier sequence, wherein the array comprises a pool of different identifier sequences. Each identifier sequence of the pool comprises a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two, or at least three, nucleotide substitutions.

The TSOs as described above may be used in a method of adding an identifier sequence to an array of polynucleotides, the method comprising:

(i) reverse transcribing the polynucleotides using a template switch reverse transcriptase to generate double stranded polynucleotides having 3' end non-templated nucleotides; and
(ii) annealing a set of template switch oligonucleotides (TSOs) as described above to the 3' end non-templated nucleotides.

In a further aspect, the invention provides a kit for generating a library of polynucleotides. The kit comprises a set of capture polynucleotides. Each capture polynucleotide comprises an RNA capture region, one of a mixed pool of first identifier sequences in the set of capture polynucleotides, and a PCR handle sequence. The kit further comprises a set of template switch oligonucleotides (TSOs). Each TSO comprises a PCR handle sequence, one of a mixed pool of second identifier sequences and a 3' end template switching sequence that can hybridize to 3' non-templated nucleotides added to a cDNA strand by a reverse transcriptase. Each identifier sequence of the pool in the capture polynucleotides and/or the TSOs comprises a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two, or at least three, nucleotide substitutions.

The disclosure will now be described in more detail, by way of example and not limitation, and by reference to the accompanying drawings. Many equivalent modifications and variations will be apparent, to those skilled in the art when given this disclosure.

The present disclosure includes the combination of the aspects and preferred features described except where such a combination is clearly impermissible or is stated to be expressly avoided. As used in this specification and the appended items, the singular forms "a", "an", and "the" include plural referents unless the content clearly dictates otherwise. Thus, for example, reference to "a polynucleotide" includes two or more such polynucleotides.

Section headings are used herein for convenience only and are not to be construed as limiting in any way.

## Description of the Figures

**Fig. 1 - Homotrimeric nucleoside phosphoramidite UMI-tagging approach for long-read transcriptomic sequencing.** a) A cDNA is flanked by 12 homotrimer Unique Molecular Identifier (UMI) on each side of the molecule, with a SMART primer at the extremity of the library used for amplification. b) Two sets of primers are synthesised, one containing a polyT sequence, UMI and SMART primer, the second is a template switch oligonucleotide that contains a UMI and SMART primer. c) The library is amplified with a minimum of 25 PCR cycles. d) The library is sequenced following the addition of the adapter molecules and motor protein using the standard PCR free Nanopore library. e) Both UMI sequences are identified and then added to the read name. f) Errors are corrected computationally and the inclusion of both UMIs allows the user to remove PCR artefacts. g) Pair UMI detection approach to identify chimeric artefacts. h) Simulated data showing the difference between the deduplicated UMIs and ground truth with respect to sequencing error rate. i) Error correction using UMI-tools or mcl-UMI with respect to sequencing error j) The number of artefactual chimeric reads identified using the dual UMI error correction approach shown in g.

**Fig. 2 - Error profiling of short and long-read RNA sequencing using homotrimeric UMIs.** a, Qscore profiles for bulk RNA sequencing reads generated following Illumina, ONT and PacBio sequencing. A bulk RNA library was generated using primers that contain a Common Molecular Identifier (CMI) synthesised using homotrimers. b, Sequencing was then performed using Illumina, PacBio and ONT sequencing platforms. For each platform, the frequency of CMIs that do not contain any errors, the frequency of CMIs that can be error corrected to the CMI sequence and the percent of CMIs in which at least one error remains is plotted. c, The relationship between the recovery of CMIs following the application of different homotrimer error thresholds. d, The relationship of the hamming distance between the sequenced CMI and the expected CMI and the percent of reads is shown for libraries sequenced using the Illumina, PacBio and ONT with flow cells R9.4.1 and flow cells R10.4. e, The LSK-109 R9.4.1 sequencing data was deduplicated using both UMI-tools and homotrimer correction methodology and compared to assuming that no CMI is included within the library. The accuracy of each method was measured by comparing the measured gene count to the ground truth count of 1. A filtering threshold of 3 errors within the UMI was applied for the homotrimer deduplication. f, A cDNA ONT library was generated from mixed human:mouse RNA at a 50:50 ratio and homotrimeric UMIs were used to remove PCR artefacts. The frequency of chimeric PCR artefacts before and after the removal of those reads using mclUMI. a and e, show one representative sample. c shows one sample of one experiment performed. b, and d show one representative sample of three performed.

**Fig. 3 - Homotrimer UMIs improve differential expression analysis for long-read sequencing.** Scatter plot of the log2 fold changes obtained from read counting versus the log2 fold change for homotrimer UMI correction and counting for genes (a) and transcripts (b). Scatter plot of the log2 fold changes obtained from randomly collapsing each sequenced trimer UMI and then applying UMI-tools deduplication versus the log2 fold changes obtained from homotrimer UMI correction and counting for genes (c) and transcripts (d). Red points indicate the overlapping significant genes/transcripts and blue points indicate genes/transcripts that were disconcordantly significantly differentially expressed.

**Fig. 4 - UMI errors from sequencing or PCR increase UMI counting.** a, Ideal UMI collapsing example: This figure illustrates an ideal scenario where transcripts (two blue and two green) are levelled with unique molecular identifier barcodes (UMIs) and PCR amplification is performed. Due to PCR amplification bias, longer transcripts have a lower amplification rate than shorter ones. The sequenced reads are then grouped together based on the set of UMIs and then collapsed within those groups to match the original number of transcripts. b, Increased UMI counting occurs due to errors: In real situations, errors occur during PCR amplification and sequencing, which can lead to increased UMI counts. As shown in this example, an error occurs within one of the UMIs which results in a higher count of unique UMIs than the actual number of input transcripts in the final library. This phenomenon can affect downstream analysis and should be taken into consideration otherwise they will lead to false positives when performing differential expression analysis.

**Fig. 5 - Figure 2: Computational UMI demultiplexing strategies alone are insufficient to correct errors.** To assess the efficiency of computational UMI demultiplexing strategies in correcting sequencing errors, monomer base UMIs were simulated with increasing error rates and calculated the fold change between the ground truth and the output of applying MCL-umi error correction. The results show that even with the application of MCL-umi error correction, computational strategies alone are unable to fully correct sequencing errors, as evidenced by the increasing deviation from the ground truth with higher error rates.

**Fig 6 - Improved UMI deduplication using homotrimer blocks of nucleotides.** (a) Schematic showing attachment of 3' and 5' UMIs to mRNA. **(b)** Homotrimer blocks of nucleotides are used to synthesise UMIs, which enables efficient error correction through a majority vote between the trimer nucleotide blocks. This approach does not require knowledge of the original oligonucleotide, and the trimers are resilient to base pair errors (example 1) and insertions/deletions (example 2). Errors are removed before collapsing the sequence to a single-base and then performing downstream analyses. **(c)** UMIs were simulated with increasing error rates and modelled the correction of trimer sequences using the majority vote approach as described in (b). To handle homotrimer errors more robustly, a new model was subsequently developed, which is described within the methods section "homotrimer correction".

**Fig. 7 - Comparison of Homotrimer and monomer UMI for demultiplexing.** The effectiveness of homotrimer UMIs versus monomer UMIs was evaluated for demultiplexing by simulating base UMIs with increasing error rates and calculated the fold change between the ground truth and the output of applying computational error correction. The findings demonstrate that homotrimer UMIs outperform both uncorrected monomer UMIs and monomer UMIs corrected using computational methods. At very high error rates, the demultiplexing accuracy of homotrimer UMIs can be further improved by combining them with computational error correction techniques such as MCL-umi.

**Fig. 8 The majority vote method is improved using a set coverage solution. (a)** A cumulative plot showing the fraction of genes that have been collapsed to a UMI count of less than or equal to the value shown on the x-axis by either the majority vote approach or the set cover-based optimization method. Only genes with at least 2 mapped reads were considered (n=3,428). Maximal UMI counts returned by majority vote and set cover optimization are 245 and 72, respectively. **(b)** A scatter plot comparing UMI counts obtained using the majority vote approach (x-axis) to counts returned by the greedy set cover algorithm (y-axis). Only genes with at least 2 mapped reads were considered (n=3,428). To simplify visualization, genes with large majority counts were excluded (x,y) = (83, 22), (91, 24), (96, 31), (106, 32), (113, 33) and (245, 72).

**Fig. 9 Illustration of removal of chimeric reads using the Rarity and RarityCR methods. a,** UMI count tables at a given genomic locus. It includes a count table of UMIs at 3' end, a count table of UMIs at 5' end, and a count table of UMIs obtained by merging UMIs at 3' end 5' ends. Non-corrected UMIs represent UMIs of trimer nucleotide blocks in their original form, while corrected UMIs represent UMIs of collapsing trimer nucleotide blocks. **b** and **c** show the Rarity and RarityCR methods, respectively. The Rarity and RarityCR methods are incorporated into the MCL-umi software tool.

**Fig. 10 Simulated data demonstrates the effectiveness of using dual UMIs for chimeric artefact removal.** To evaluate the effectiveness of dual UMIs in removing chimeric artifacts, these events were simulated and the ability of dual UMIs attached to each cDNA end to accurately identify and remove these artifacts was tested. During simulated PCR amplification, it was assumed that there was a 2% probability of artefactual translocations occurring between two reads. The goal was to evaluate the efficacy of using trimer UMIs at both ends to detect these artefactual translocations. The trimer UMIs were collapsed at both ends of each read into monomer UMIs and compared them to the original pairs of UMIs made during library preparation. A read was considered a real chromosomal translocation if its collapsed UMI pair was no more than 2 edit distance away from any UMI pairs in the prepared library; otherwise, it was considered a real PCR artifact. **(a)** The ability to detect chimeric artefacts using umiRarity This was compared to detecting artefacts only considering using UMIs at the 3' end of the transcript (referred to as "control"). It was shown that considering UMIs at both ends is necessary for improving the performance of detecting chimeric artefacts. **(b)** It was found that the threshold-based match strategy accurately identified the real and artefactual molecules, with the number of estimated molecules closely mirroring the ground truth. The simulation suggests that the use of trimer UMIs can effectively remove PCR-amplified real and artefactual translocation molecules, which is an important consideration for translocation detection.

**Fig. 11 Empirical evaluation of transcript counting with Common Molecular Identifiers (CMIs). (a)** An Ideal CMI collapsing example: In this scenario, transcripts (two blue and two green) are levelled with a common molecular identifier barcode (CMIs; labelled as red) and amplified via PCR. During transcripts grouping, all transcripts are labelled with the same common sequence. Therefore, following demultiplexing, each transcript should receive a count of one for every instance of detection. **(b)** Increased counts result from the introduction of errors: This figure illustrates the effect of the errors within the CMI sequence. Any error introduced during PCR or sequencing creates a new CMI (labelled as yellow), resulting in an increase in transcript counts. This allows empirical evaluation of the effect of errors on the counting of transcripts, providing valuable insights into the accuracy of transcript quantification.

**Fig. 12 Read quality control following sequencing by Illumina, PacBio and ONT.** The read quality outputs from FASTQC for Illumina, PacBio and ONT (old chemistry: LSK110 R9.4.1 and new kit14 chemistry LSK114 R10.4).

**Fig. 13 Quality score following sequencing by Illumina, PacBio and ONT.** The Qscore of each read as a relationship between the number of errors measured within the CMI, sequencing across Illumina, PacBio and ONT (old chemistry: LSK110 R9.4.1 and new kit14 chemistry LSK114 R10.4) technologies. **a,** Qscore represented as a density plot for the different sequencing platforms. **b,** The Qscore relationship with the number of errors detected within the CMI across the different sequencing platforms.

**Fig. 14(a)** Percent of CMIs that are correctly sequenced and then error corrected using homotrimer correction across Illumina, PacBio and ONT sequencing platforms. **(b)** Barcode assignment using homotrimer barcodes before and after majority vote correction.

**Fig. 15 Evaluation of CMI accuracy across Illumina, PacBio and legacy ONT chemistry.** The accuracy of sequencing was evaluated for legacy ONT chemistry, the percentage of CMIs with a Hamming distance between the expected and the sequenced CMI was measured. The results are shown for Illumina, PacBio, and ONT legacy chemistry sequencing. Data from Illumina and PacBio is the same as in Fig. 14a.

**Fig. 16 Improved basecalling accuracy using super accuracy guppy basecalling for ONT technology.** Percent of CMIs that are correctly sequenced and then error corrected using homotrimer correction using either high accuracy guppy basecalling or super accuracy basecalling for the LSK114 chemistry and R10.4 flow cells.

**Fig. 17(a)** Percent of genes with an accurate CMI count following increased PCR cycles of the same sequencing library. **(b)** CMI counts plotted for each transcript showing the numbers of counts per transcript (the ground truth count for each transcript should be equal to 1).

**Fig. 18(a)** Percent of genes with an accurate CMI count following counting using UMI-tools correction and homotrimer error correction. **(b)** The number of artefactual human-mouse chimeric reads removed using the dual UMI error correction approach (mcl-UMI). RM82 sarcoma cells were treated with DMSO or SGC-CLK-1 for 24 hours.

**Fig. 19** TLE5 read counts showing the expression for DMSO and SGC-CLK1 following the application of UMI-tools or homotrimer correction.

**Fig. 20 Analysis of differential gene expression in RM82 Ewing's sarcoma cells treated with DMSO and CLK-1 inhibitor and sequenced using the ONT platform. (a)** A PCR plot showing the variance for cells treated with either DMSO or CLK-1 inhibitor. **(b)** A volcano plot showing the log2 fold change and -log10 padj values for cells treated with DMSO or CLK-1 inhibitor, analysed without the inclusion of a UMI during analysis. **(c)** A volcano plot showing the log2 fold change and -log10 padj values for cells treated with DMSO or CLK-1 inhibitor and analysed using the homotrimer corrected UMI. These results demonstrate the utility of homotrimer correction in identifying differentially expressed genes and removal of false positive transcripts.

**Fig. 21 Analysis of differential gene expression in RM82 Ewing's sarcoma cells treated with DMSO and CLK-1 inhibitor and sequenced using the Illumina platform. (a)** A PCR plot showing the variance for cells treated with either DMSO or CLK-1 inhibitor. **(b)** This scatter plot compares the log2 fold changes obtained from randomly collapsing each sequenced trimer UMI with those obtained from homotrimer UMI correction. **(c)** A volcano plot showing the log2 fold change and -log10 padj values for cells treated with DMSO or CLK-1 inhibitor, analysed without the inclusion of a UMI during analysis. **(d)** A volcano plot showing the log2 fold change and -log10 padj values for cells treated with DMSO or CLK-1 inhibitor and analysed using the homotrimer corrected UMI. These results demonstrate the utility of homotrimer correction in identifying differentially expressed genes and removal of false positive genes.

**Fig. 22 Go analysis of the differentially expressed genes between DMSO and CLK-1 inhibitor.** Go analysis was performed for data that was not corrected using UMIs **(a)** and differentially regulated genes following homotrimer correction **(b).**

**Fig. 23** Human Jurkat and mouse 5TGM1 cells were mixed at a 50:50 ratio and approximately 3000 cells were taken for encapsulation and cDNA synthesis using 10X chromium followed by nanopore sequencing.

**Fig. 24(a)** A UMAP of 10X chromium data showing the integration, clustering and annotation of human and mouse cells following 30 and 35 cycles of PCR. **(b)** A density plot for the 10X chromium data showing the log10 density of the number of UMIs following 30 and 35 cycles of PCR. The dotted line shows the maximum density for each condition.

**Fig. 25 Quality metrics for 10X chromium single-cell sequencing libraries amplified using 30 and 35 cycles of PCR.** The number of counts, features and number of UMIs for 10X Chromium libraries PCR amplified for 30 cycles **(a)** and 35 cycles **(b).** Each dot represents a single-cell following filtering. **(c)**The log10 genes per UMI plotted as a density.

**Fig. 26(a)** A UMAP showing the expression of ENSMUST00000034966 between libraries amplified following 30 and 35 PCR cycles. **(b)** A UMAP showing the expression of ENST00000532223 between libraries amplified following 30 and 35 cycles of PCR.

**Fig. 27(a)** A drop-seq library showing the percent of reads that have an accurate CMI following amplification of the same library using 20, 25, 30 and 35 cycles of PCR before and after homotrimer correction. **(b)** barnyard plots showing the expression of mouse and human cells following 30 and 35 cycles of PCR.

**Fig. 28 The number of discordant bases across the full length of the homotrimer UMI.** The number of errors per read between the sequenced CMI and the ground truth CMI following 30 and 35 PCR cycles.

**Fig. 29 Number of features and UMAP clustering for the integrated analysis of homotrimer drop-seq UMIs following 30 and 35 cycles of PCR. (a)** The number of features detected within the mouse and human cells for monomer (left panel) and homotrimer (right panel) UMIs following 30 and 35 PCR cycles. UMAP plots showing the integration, clustering and annotation of libraries amplified following 30 and 35 PCR cycles for monomer **(b)** and homotrimer **(c)** UMIs. **(d)** UMAP plots showing the expression of a non-significant gene ENSMUST0000037023 in monomers (left panels) and homotrimer corrected (right panels) following 30 and 35 cycles of PCR. Even though this gene is not considered significantly different between 30 and 35 cycles in both the monomer and homotrimer datasets, there is generally an overall increase in background counts following 35 cycles of PCR in the monomer dataset that is not apparent within the homotrimer dataset.

**Fig. 30** UMAP plot showing the transcript expression of ENST00000330494 following monomer based UMI-tools demultiplexing **(a)** and homotrimer based demultiplexing **(b).**

**Fig 31** Schematic showing the use of annealed oligonucleotide strand pairs.

## Detailed Description of the Invention

*Detecting amplification chimeric artefacts*

Chimeric artefacts can arise during amplification when a polymerase used for the amplification falls off the polynucleotide strand that is being copied, reattaches to a different strand, and continues copying. The invention provides means and methods for detecting such chimeric artefacts by adding identifier sequences at both ends of sample polynucleotide (or, where appropriate, polynucleotides attached to sample analytes) and using the pair of identifier sequences flanking a sample polynucleotide sequence in amplified polynucleotides to detect the chimeric artefacts.

Including identifier sequences at both ends of indexed sample polynucleotides means that each library/indexed polynucleotide has a distinct pair of identifier sequences. In some cases, the pool(s) of identifier sequences used may be large enough that essentially every sample polynucleotide that was indexed by the addition of the identifier sequences at each end can be expected to have a unique pair of flanking sequence identifiers. More typically, however, the pool size(s) will be chosen to ensure that each identical copy of a polynucleotide in the initial sample, or each polynucleotide in the initial sample having at least the same terminal sequence adjacent to one of the identifier sequences in the indexed polynucleotide (e.g. each polynucleotide of a particular species and having the same terminal splicing position) has a unique pair of flanking identifier sequences. Hence, in some cases, the sample polynucleotide sequence, or a portion thereof, may be used together with the identifier sequences added at each end to uniquely identify sample polynucleotides or the flanking sequence identifier pairs associated therewith. For example, in some cases up to 100 nucleotides at the end where a sequence identifier is added may be used. Typically between about 30 and about 100 may be used. In addition, or alternatively, an additional identifier sequence, such as a barcode sequence, as described elsewhere herein, or the PCR handle sequence, or a portion thereof, may be used together with the identifier sequences added at each end to uniquely identify sample polynucleotides or the flanking sequence identifier pairs associated therewith. For example, the PCR handle sequence, or a portion thereof, may be used when the first and second PCR handle sequences are different from each other (all first identifier sequences are included at the same end as a PCR handle sequence having a common first PCR handle sequence, and all second identifier sequences are included at the same end as a PCR handle sequence having a common second PCR handle sequence). The first and second identifier sequence pools themselves may comprise the same or different identifier sequences, as described elsewhere herein.

The method may comprise identifying pairings (or unique pairings) of first and second identifier sequences in the indexed/library polynucleotides (optionally also using additional sequences as described above). The number of amplified polynucleotides sharing each identified pairing is counted. Rare or underrepresented pairings that have fewer copies than is expected, based for example on the extent or number of rounds of amplification and/or the identity of the sample polynucleotide (e.g. polynucleotide species that are expected to more abundant compared to the frequency of the identifier sequence pairing), may be identified as chimeric artefact polynucleotides or suspected chimeric artefact polynucleotides. Such polynucleotide sequences may, where appropriate, be removed from further sample/library analysis.

An alternative or complementary approach, which may be used along with or in combination with the method described above, involves identifying "mismatch pairings" of identifier sequences flanking a sample polynucleotide sequence in the library. A pairing is a mismatch pairing if each identifier sequence of the pair is also present in a different identified pairing, optionally in combination with additional matching sequences elements as described above. Hence, a pairing may be identified as a mismatch pairing if it comprises (a) a first identifier sequence in common with another pairing in the library having a different second identifier sequence to the mismatch pairing; and (b) a second identifier sequence in common with another pairing in the library having a different first identifier sequence to the mismatch pairing. An amplified sample polynucleotide flanked by a mismatched (and/or underrepresented) pairing of first and second identifier sequences may be identified as a chimeric artefact polynucleotide. Such polynucleotide sequences may, where appropriate, be removed from further sample/library analysis. In some cases, a mismatch pairing is identified if either one of the two identifier sequences is also found in a more abundant pairing of two identifier sequences.

*Identifier sequences*

The methods described herein make use of, and arrays of polynucleotides described herein include, identifier sequences. An identifier sequence is a polynucleotide sequence tag or index that can be used to distinguish different polynucleotides or groups of polynucleotides, either alone or combination with other identifier sequences or other sequence elements. Means and methods are described for adding an identifier sequences at both ends of polynucleotides, typically of unknown sequence, in a mixed sample. This double-indexing is particularly useful for detecting chimeric artefacts produced during amplification of the indexed polynucleotides and the production of libraries for sequencing. However, the identifier sequences added at one or both ends of the sample polynucleotides can also in some cases have additional functions as are known in the art. For example, identifier sequences are typically added to or included in a polynucleotide to capture information about the polynucleotide or an analyte associated with the polynucleotide. For example, an identifier sequence could indicate the source of a polynucleotide/analyte, for example a particular sample (e.g. a cell, or cell nucleus), or a spatial position, or can be used to count the number of original analytes after downstream amplification.

In some cases, an identifier sequence may be added to a polynucleotide used to capture analytes/polynucleotides in a sample, for example when generating a library of sample analytes. The identifier sequence in the capture polynucleotide is copied or amplified together with the captured polynucleotide such that the captured polynucleotide is "tagged" or "indexed" by the identifier sequence.

Often a benefit of using an identifier sequence is that it allows different samples, molecules or analytes to be mixed together physically for more efficient processing, such as amplification and sequencing. The identifier sequence is used later to distinguish different molecules, their source, or copies thereof.

An identifier sequence may allow polynucleotides having a shared source to be identified. Typical examples of polynucleotides having a shared source that can be tagged with an identifier sequence include the polynucleotides associated with or captured on a single micro-particle or micro-bead, or in a single well or discrete position on a surface, or in a sample or part of a sample (e.g. contacted with the same).

An identifier sequence used to tag/index multiple analytes from a shared source, such as a single cell, may be referred to as a "barcode" (BC) sequence.

An identifier sequence added to analytes to identify or count single molecules or single capture events, e.g. prior to an amplification step, may be referred to as a "unique molecular identifier" (UMI) sequence.

An identifier sequence may also in some cases be used to determine an amplification and/or sequencing error rate.

Identifier sequences have conventionally consisted of a string of single nucleotides, generated randomly, for example using split-and-pool synthesis or degenerate polynucleotide synthesis, as described further elsewhere herein.

In some cases, the identifier sequences may have the features described in WO 2022/118027, for example in the claims of that application, or use homodimer nucleotide blocks as described therein and exemplified in the Examples. Instead of consisting of a string of single nucleotides, the identifier sequences comprise a series of discrete nucleotide blocks. Typically the nucleotide blocks are two or three nucleotides in length, but longer nucleotide blocks can also be used, as described elsewhere herein. An identifier sequence could also be built up from multiple nucleotide blocks of different sizes. Typically, however, each nucleotide block in the same or an equivalent position in each identifier sequence is the same length. Each discrete nucleotide block has one of a pool of known sequences.

The identifier sequence, e.g. of an individual polynucleotide, may comprise or consist of any combination of the same or different nucleotide blocks within the limitations described herein. However, the ability to differentiate between identifier sequences is improved by using a pre-determined and limited number of different nucleotide block sequences, either across the full identifier sequence or at each same or equivalent position of the identifier sequence across different polynucleotides. The sequence of each nucleotide block can be compared across different polynucleotides, i.e. because they are at the same known or otherwise identifiable position in each polynucleotide. These pre-defined nucleotide block sequences may be referred to herein as a nucleotide block pool or nucleotide block sequence pool.

All of the nucleotide block sequences within the pre-defined nucleotide block pool differ from every other nucleotide block sequence within the pool by at least two nucleotide substitutions. This provides a number of advantages. One advantage is that a single nucleotide substitution in one or more nucleotide blocks as a result of an amplification or sequencing error will not directly result in mis-identification or allocation of the sequence. All single substitutions in a nucleotide block can be detected by comparing the sequenced nucleotide blocks to the known nucleotide block sequence pool. Moreover, since single errors in each nucleotide block can always be detected, the error rate across the whole identifier sequence can be used to determine the overall accuracy of the polynucleotide sequence. This determined error rate can in turn be used to correctly identify a higher proportion of the identifier sequences and/or to identify the correct identifier sequences with higher confidence. Importantly, it is not necessary that the full sequence of individual identifier sequences is known *ab initio* in order to identify amplification/sequencing errors. Hence, this feature allows for diversity in the identifier sequences coupled with error detection and improved identifier sequence identification.

Identifier sequences of this type are particularly useful when it is desirable to generate a diversity of sequence identifiers for tagging different samples/analytes/polynucleotides, whilst increasing the ability to distinguish between different identifier sequences, particularly when errors may be introduced during amplification and/or sequencing, e.g. when using full-length single molecule sequencing or "long-read" sequencing methods.

Typically the same pre-determined nucleotide block pool is used at the equivalent position in an identifier sequence across an array of polynucleotides. Any one of the nucleotide blocks from the pool can be used at a given position in the identifier sequence of any one analytes/polynucleotide. The same or different pre-determined nucleotide block pools may be used at other positions within the identifier sequence. This may in part depend on the method used to generate and synthesise the identifier sequence. If the identifier sequence is generated by repeated rounds of degenerate polynucleotide synthesis from a single mixed pool of nucleotide blocks, then generally the whole identifier sequence will be generated from the same single mixed pool of nucleotide block sequences. Hence, in some cases the same pre-determined nucleotide block pool may be used to generate all of the nucleotide blocks of the identifier sequence of the polynucleotides.

The sequence of each of the nucleotide blocks is otherwise not particularly limited unless otherwise provided herein and provided that the different nucleotide block sequences can be distinguished when sequenced.

In some cases, the nucleotide block sequences of any one or more pools of nucleotide blocks used to generate an identifier sequence are selected such that each nucleotide block sequence differs from each other nucleotide block sequence in the pool by at least three nucleotide substitutions. In this case, any nucleotide block comprising any two single nucleotide substitutions can still be identified. This may be particularly useful when using a very low accuracy sequencing and/or amplification method or in any methods wherein a very high level of accuracy is desirable in determining identifier sequences or identifying groups of molecules amplified from a single original polynucleotide in a sample.

The term "nucleotide substitution" as used herein typically means the replacement of a nucleotide at a single position in a longer nucleotide sequence with a different nucleotide at the same position. For example, the nucleotide block sequences 'AC' and 'GT' are considered to differ by two nucleotide substitutions because the nucleotide in the first position of the nucleotide block sequence has been replaced, and the nucleotide in the second position of the nucleotide block sequence has been replaced. Similarly, the nucleotide block sequences 'AC' and 'CA' are considered to differ by two nucleotide substitutions because the nucleotide in the first position of the nucleotide block sequence has been replaced, and the nucleotide in the second position of the nucleotide block sequence has been replaced.

A nucleotide block is typically two or three nucleotides in length, particularly for nucleotide block pools wherein the sequence of every nucleotide block differs from every other nucleotide block in the pool by at least two or at least three nucleotides, respectively. Longer sequences can also be used, for example nucleotide blocks of up to 4, 5, 6, 7, or 8 nucleotides. Pre-synthesised nucleotide blocks used to generate the identifier sequence, as described elsewhere herein, may in some cases include additional nucleotides or spacers that are not part of the identifier sequence. Once inserted into the polynucleotide, these additional nucleotides provide additional sequence elements in between the nucleotide blocks of the identifier sequence. They are not part of the identifier sequence as described herein. However, they may be used to determine the relative position of nucleotide blocks of the identifier sequence.

In some cases, all of the nucleotide blocks used in the identifier sequence have the same length. This may be particularly useful when the nucleotide blocks are added using degenerate polynucleotide synthesis, as described further elsewhere herein, where a number of the nucleotide blocks are added consecutively to each polynucleotide. Hence, using a pool of nucleotide blocks each with the same number of nucleotides ensures that the start and end position of each consecutive nucleotide block in each identifier sequence added to different polynucleotides can be identified. In other cases, different lengths of nucleotide blocks may be used to build up the identifier sequence, particularly when the length used at each position in each identifier sequences added to different polynucleotides is the same, pre-determined or otherwise known. In some cases, for example, the nucleotide blocks may be added using split-and-pool synthesis. In this case, the same length nucleotide block will typically be added to all of the polynucleotides at the same nucleotide block position, across different pools. However, different sized nucleotide blocks could be added in different rounds of the split and pool synthesis and/or at different nucleotide block positions.

An identifier sequence comprises at least 2, more typically at least 3, 4, 5, 6, 7 or 8 sequence units (where a sequence unit is either a single nucleotide or a nucleotide block as described herein) and up to 12, 13 or 14 sequence units or more, more typically 6 to 14, 7 to 13, 10 to 14 or 8 or 12 sequence units. 8 to 12 sequence units are most typical. The sequence units are added to, or present in, the relative polynucleotide at successive, consecutive or nonconsecutive unit or nucleotide block positions. The identifier sequence is defined by the sequence of the unit or nucleotide block at each position. In general, identifier sequences comprising more sequence units will provide a greater diversity of possible identifier sequences. Hence, the number of sequence units chosen will be influenced by the total diversity or number of different possible identifier sequences that are needed for a particular purpose.

The total diversity of possible identifier sequences is also dependent on the number of nucleotides or nucleotide block sequences that are permitted at each unit or nucleotide block position in the identifier sequence, i.e. the size of the (nucleotide block) sequence pool for each position. A pool of di-nucleotide block sequences using only the four canonical nucleotides A, G, T and C (or U) could comprise 2, 3 or 4 different sequences (to ensure that each sequence differs from every other sequence by at least two nucleotide substitutions). A similar pool of tri-nucleotide block sequences using only the four canonical nucleotides may comprise up to 12 different sequences. A greater diversity of nucleotide block sequences could be generated by using longer nucleotide blocks (i.e. 4 or more nucleotides in length) and/or by including non-canonical nucleotides and/or nucleotide analogues, as long as the nucleotides and/or nucleotide analogues use in the sequence can be adequately distinguished when correctly sequenced.

For example, 10 to 12 sequence units, with four possible options for each unit, provides about 1e+6 to 1.6e+7 possible unique identifier sequences. If an identifier sequence from this pool is added at each end of sample polynucleotides, then the polynucleotides have about 1e+12 to 2.8e+14 possible identifier sequence combinations. A typical bulk mRNAseq experiment might start with 1 $\mu$g of total RNA and contain about 4e+10 mRNA molecules. Hence, in this example, the number of mRNA molecules is greater than the number of identifier sequences in the pool, but smaller than the number of unique combinations of identifier sequences added at both ends. Moreover, the mRNA sample may be made up of many thousands of non-identical transcripts, which can further distinguish between indexed library sample polynucleotides amplified from different sample molecules flanked by an identifier sequence at each end (as described further elsewhere herein). A typical sequencing depth might be only 2e+7 to 1e+8 for a given sample. Hence, the chances of two identical sample RNA transcripts getting an identical pairing of flanking identifier sequences becomes, in this example, vanishingly small.

The identifier sequences may in some cases be synthesised using pre-synthesised nucleotide blocks, as described elsewhere herein. One advantage of this is that more diversity in the identifier sequence can be generated per round of synthesis and using just the same limited pool of nucleotides, for example just the canonical nucleotides A, G, T and C (or U), i.e. by including a larger pool of different nucleotide block sequences as described above.

Hence, the total diversity of possible identifier sequences is determined by (i) the number of sequence units included in the identifier sequence; and (ii) the number of different units or nucleotide block sequences included in the pool of sequences that can be used at each unit/nucleotide block position. If the same pool is used at every position, then the total possible diversity of sequences is equal to [the size of the pool] x [the number of sequence units or nucleotide blocks in the identifier sequence]. For some applications, it is desirable to design the identifier sequence such that the total possible diversity of sequences is the same as, or in excess of the number of polynucleotides, or the number of sub-arrays of polynucleotides that the identifier sequence is intended to distinguish. In some cases, the total diversity of possible identifier sequences, or the total diversity of unique pairings of identifier sequences added at each end of ample polynucleotides, is at least 10, or at least 20, 50, 100, 200 or 500 times in excess of the number of sample polynucleotides.

In some cases sequence units or nucleotide blocks may be used consecutively to form a single longer nucleotide block corresponding to the full identifier sequence, i.e. a series of consecutive sequence units or nucleotide blocks. The term "consecutive" is used to refer to sequential sequence units or nucleotides blocks in a polynucleotide which immediately follow the previous sequence unit or nucleotide block without intervening nucleotides. For example, an identifier sequence comprising 6 nucleotide blocks, wherein the nucleotide blocks are selected from di-adenosine, di-guanosine, di-cytidine, di-thymidine and di-uridine may have the sequence "AAGGCCTTAAGG".

In other cases, one or more spacers or other sequence elements may be included between the sequence units or nucleotide blocks that make up the identifier sequence. The position or identity of the sequence units or nucleotide blocks of the identifier sequence in the polynucleotide may be determined by any suitable means, for example by adding the sequence units or nucleotide blocks at pre-determined positions in the polynucleotide or using nucleotide analogues in or otherwise marking or tagging the sequence units or nucleotide blocks of the identifier sequence. The identifier sequence or the region of the polynucleotide containing all of the sequence units or nucleotide blocks of the identifier sequence, optionally with other intervening sequence elements, may in some cases be up to 20, 22, 24, 26, 28, 30, 25, 40, 45, 50, 70, 100 or 200 nucleotides in length. A typical UMI built up from a series of 8 to 12 consecutive nucleotide blocks would be 16 to 25 nucleotides in length if blocks that are two nucleotides in length are used, or 24 to 36 nucleotides in length if blocks that are two nucleotides in length are used.

In some cases, one or more or each of the nucleotide block in a pool consists of two or three or more of the same nucleotide. For example, the nucleotide block pool may in some cases comprise or consist of the nucleotide blocks AA (di-adenosine), TT (di-thymidine), GG (di-guanosine) and CC (di-cytidine) (or UU (di-uridine)) or/or the nucleotide blocks AAA (tri-adenosine, TTT (tri-thymidine), GGG (tri-guanosine) and CCC (tri-cytidine) (or UUU (tri-uridine)); or the nucleotide blocks AAA, TTT, CCX and GGY, wherein X is A, or otherwise T or G, and Y is C, or otherwise A or T; or any combination thereof. In other cases one or more or each nucleotide block sequence may comprise a duplicate or triplicate or other multiple of a nucleotide analogue.

The term "nucleotide" as used herein, particularly in relation to the nucleotide blocks of an identifier sequence, may refer to natural or canonical nucleotides (*i.e.,* "naturally occurring" or "natural" nucleotides), which include adenosine, guanosine, cytidine, thymidine and uridine, or to a non-canonical nucleotide or a nucleotide analogue. The polynucleotides or nucleotide blocks described herein may comprise any combination of natural nucleotides. Alternatively, any non-canonical nucleotides or nucleotide analogues may appear in one or more identifier sequence.

Generally, a nucleotide analogue contains a nucleic acid analogue, a sugar and a phosphate group, or variants thereof, and integrates into a polynucleotide chain in place of a natural nucleotide. Using nucleotide analogues in the identifier sequence may be particularly useful where the nucleotide analogues produce a more distinct signal when sequencing. The person skilled in the art is able to select appropriate nucleotide analogues or combinations of nucleotides/analogues to use in the identifier sequence. Examples of nucleotide analogues that may be included in the polynucleotides or nucleotide blocks described herein are as follows.

Peptide nucleotides, in which the phosphate linkage found in DNA and RNA is replaced by a peptide-like N-(2-aminoethyl)glycine. Peptide nucleotides undergo normal Watson-Crick base pairing and hybridize to complementary DNA/RNA with higher affinity and specificity and lower salt-dependency than normal DNA/RNA oligonucleotides and may have increased stability. Locked nucleotides (LNA), which comprises a 2'-O-4'-C-methylene bridge and are conformationally restricted. LNA form stable hybrid duplexes with DNA and RNA with increased stability and higher hybrid duplex melting temperatures. Propynyl dU (also known as pdU-CE Phosphoramidite, or 5'-Dimethoxytrityl-5-(1-Propynyl)-2'-deoxyUridine,3'-[(2-cyanoethyl)-(N,N-diisopropyl)]-phosphoramidite). An unlocked nucleotide (UNA), which is an analogue of a ribonucleotide in which the C2'-C3' bond has been cleaved. UNA form hybrid duplexes with DNA and RNA, but with decreased stability and lower hybrid duplex melting temperatures. LNA and UNA may therefore be used to finely adjust the thermodynamic properties of the polynucleotides in which they are incorporated. Triazole-linked DNA oligonucleotides, in which one or more of the natural phosphate backbone linkages are replaced with triazole linkages, particularly when click chemistry is used for synthesising the polynucleotide. A 2'-O-methoxy-ethyl base (2'-MOE), such as 2-Methoxyethoxy A, 2-Methoxyethoxy MeC, 2-Methoxyethoxy G and/or 2-Methoxyethoxy T. A 2'-O-Methyl RNA base. A 2'-fluoro base, such as fluoro C, fluoro U, fluoro A, and/or fluoro G. Other specific examples of nucleotide analogues that may be used include 2-Aminopurine, 5-Bromo dU, deoxyUridine, 2,6-Diaminopurine (2-Amino-dA), Dideoxy-C, deoxyInosine, Hydroxymethyl dC, Inverted dT, Iso-dG, Iso-dC, 5-Methyl dC, 5-Nitroindole, 5-hydroxybutynl-2'-deoxyuridine (Super T) and 8-aza-7-deazaguanosine (Super G). Super T 2,6-Diaminopurine (2-Amino-dA) and/or 5-Methyl dC. A biotinylated nucleotide. In some cases a polynucleotide, nucleotide block or identifier sequence described herein may comprise at least two, or at least 3, 4, 5, 6, 7, 8, 9, 10, 12, 14, 16, 18, 20, 25, 30, 35, 40, or up to 2%, 5%, 10%, 15%, 20%, 25%, 30%, 35%, 40% or more nucleotide analogues and/or biotinylated nucleotides, or any one type of nucleotide analogue as described herein.

*Methods of adding an identifier sequence to a polynucleotide*

Methods and products can be used to add identifier sequences (first and second identifier sequences) at both ends of polynucleotides in a mixed sample when generating a library of the polynucleotides. Libraries of polynucleotides from a mixed sample can have identifier sequences added at both ends. Such libraries are useful in particular for detecting chimeric artefact polynucleotides produced during amplification of the mixed sample of polynucleotides. The first and second identifier sequences that flank the sample polynucleotide sequences are from a mixed pool of first and second identifier sequences. The first and second identifier sequence pools may comprise the same sequences, partly overlapping sets of sequences, or different sequences. In some cases the first and second identifier sequence are added to the sample polynucleotides at the same time or in a single process step or sample reaction and optionally from the same pool of identifier sequences. In other cases the first and second identifier sequences are added sequentially or in separate process steps or sample reactions and optionally from a different pool of identifier sequences. For example, the first identifier sequences may be provided on a set/array of capture polynucleotides and the second identifier sequences may be provided on a set/array of template switch oligonucleotides. Hence, the first and second identifier sequences are added in different process steps. Nonetheless, the sequence pool of the first and second identifier sequences, whether provided in the same or separate processing steps, may be either the same or different.

In some cases it is beneficial to add identifier sequences of different lengths or having a different number of sequence units or sequence nucleotide blocks at each end of the sample polynucleotides. Hence all of the first identifier sequences of the pool of first identifier sequences have a different sequence length, or a different number of nucleotide blocks (or a different set of sequence lengths or number of sequence units or nucleotide blocks), from all of the second identifier sequences of the pool of second identifier sequences. Provided the lengths/number of sequence units available in each of the first and second pool are known, then identifier sequences from the first and second pools can be distinguished based on their length alone. This can be used to identify some amplification chimeric artefact library polynucleotides without needing to compare the identifier sequence sequences *per se.* For example, the pool of first identifier sequences may consist of identifier sequences that are all longer (or all have more sequence units or nucleotide blocks) than all of the pool of second identifier sequences. Any amplified library polynucleotides that have a "long" identifier sequence at each end can be identified as a chimeric artefact. Similarly amplified library polynucleotides that have a "short" identifier sequence at each end can be identified as a chimeric artefact. Library polynucleotides that have a "long" identifier sequence at one end and a "short" identifier sequence at the other end cannot be identified as either faithful amplification products or chimeric artefacts based on the length of the identifier sequences alone. In these cases further analysis of the identifier sequences themselves is typically needed.

The identifier sequences may be added either side of the sample polynucleotide sequences in various ways. An identifier sequence could be synthesised directly on the ends of sample polynucleotides using any suitable method known in the art. For example degenerative synthesis or split-and-pool synthesis may be used, as described elsewhere herein. In this case, the pool of first and second identifier sequences is determined by the pool of single nucleotides or nucleotide blocks used at each step of synthesis. Alternatively, the pool of identifier sequences may be provided on an array of pre-synthesised polynucleotides. These polynucleotides themselves can be synthesised using known methods as described above and elsewhere herein. The polynucleotides comprising the identifier sequences may be joined to the sample polynucleotides directly, e.g. using enzymatic ligation methods, such as using DNA or RNA ligase, chemical ligation methods, such as phosphoramidate ligation, and/or click chemistry ligation methods, such as the azide-alkyne cycloaddition reaction. Alternatively, the polynucleotides comprising the identifier sequences may have other features that can be used to join them to the sample polynucleotides. Such features are described in more detail elsewhere herein, including in the sections relating to capture polynucleotides, template switch oligonucleotides and annealed oligonucleotide strand pairs.

The sample polynucleotides may be RNA or DNA and from any suitable source. In some cases an identifier sequence is added to each of at least 10, or at least 12, 20, 24, 48, 100, 200, 500, 1000, 2000, 5000, $10^4$, $10^5$, $10^7$, $10^8$, $10^9$ or $10^{10}$ sample polynucleotides/analytes, for example between 200 and $10^{12}$, or between $10^2$, $10^3$, $10^4$ or $10^5$ and $10^{11}$ polynucleotides/analytes. In some cases, a different identifier sequence is added to at least 10, or at least 20, 50, 100, 200, 500, 1000, 2000, 5000, $10^4$, $10^5$, $10^6$ or $10^7$ different polynucleotides/analytes. In some cases, a different identifier sequence is added to each polynucleotide/analyte in the sample to which the identifier sequence is added. In other cases, the same identifier sequence may be added to more than one polynucleotide/analyte. For example, if both a BC sequence and a UMI sequence is to be added to each polynucleotide/analyte (e.g. at the same end), wherein the BC sequence identifies each of a number of sub-arrays of the polynucleotides/analytes, then the UMI sequence may be designed (i.e. to have sufficient total possible sequence diversity) such that essentially every polynucleotide/analyte of the same sub-array receives a different UMI sequence, but the same UMI sequence may be added to two or more polynucleotides/analytes that are in different sub-arrays. Such polynucleotides receiving the same UMI sequence can be distinguished based on their combined BC and UMI sequences.

Similarly, unique polynucleotides or the polynucleotides amplified from a unique polynucleotide or as the result of a unique capture event, can also be identified using an identifier sequence in combination with sequence of the sample polynucleotide and/or the PCR handle sequence, or a portion thereof, for example a portion that is adjacent to the identifier sequence in a library/indexed polynucleotide. In some cases up to 100, or up to 200 or 300 or 500 nucleotides of sequence of a sample polynucleotide may be used. In some cases, for example when short-read sequencing is used, a stretch of 30 to 300 nucleotides of sample polynucleotide sequence/base-pairs may typically be used. For example, 50 base pairs are typically used with DropSeq and 91 base pairs with 10X platforms. For long-read sequencing, about a 30 to 100 nucleotides/base pairs of sequence might typically be used. The sequence of the sample polynucleotide used for this purpose is typically the sequence at one end of the sample polynucleotide, adjacent to the added sequence containing the identifier sequence.

Hence, it is not necessary that every sample polynucleotide is indexed using a unique identifier sequence, or a unique pair of identifier sequences flanking the sample polynucleotide sequence. The skilled person is able to design suitable identifier sequences and strategies for adding the identifier sequences for their specific purpose. However, in some cases at least 1%, or at least 2%, 5%, 10%, 20%, 30%, 40%, 50%, 60%, 70%, 80% of the identifier sequences (or first or second identifier sequences) that are added to or included in the indexed sample polynucleotides have a unique sequence that is not shared with any of the other included (first or second) identifier sequences. In some cases the maximum number of repeats of any one identifier sequence added to the sample polynucleotides is less than 10%, or less than 7%, 5%, 2%, 1%, 0.5%, 0.2%. 0.1%, 0.05%, 0.02%. 0.01% of the total number of indexed polynucleotides.

PCR handle sequences are also added to the sample polynucleotides and used for amplification. The PCR handle sequences are included upstream of the identifier sequences such that the first and second identifier sequences that flank the sample polynucleotide sequences are amplified together with the sample polynucleotide sequence from the PCR handle sequences. It is typically convenient to add the PCR handle sequences together with the identifier sequence added at the same end of the sample polynucleotide, in the same process step or sample reaction and/or from the same source polynucleotide, e.g. capture polynucleotides, TSOs or annealed oligonucleotide pairs comprising both the PCR handle sequence and the identifier sequence, as described further elsewhere herein. In principal, however, the identifier sequence and the PCR handle sequence could be added consecutively, in separate process steps or sample reactions. The first identifier sequence and first PCR handle sequence could be added at one end of the sample polynucleotides and then the second identifier sequence and PCR handle sequence could be added at the other end. Alternatively, both the first and second identifier sequences could be added first, followed by the first and second PCR handle sequences.

*Polynucleotides*

Polynucleotides (or arrays thereof) may comprise an identifier sequence as described herein. The terms "polynucleotide", "oligonucleotide" or "oligo" may in some cases be used herein interchangeably, and refer to a string of nucleotide monomers in a chain typically linked by phosphodiester bonds. As used herein, a polynucleotide is a chain of nucleotides of any length, whilst an oligonucleotide typically comprises up to 100 nucleotides. In some cases the polynucleotides may be at least 40 and/or up to 100 or 120, 140, 160, 180, 200, 225, 250, 275, 300, 350, 400, 500, 1000 or 2000 nucleotides in length, for example between 40 and 1000, 500, 400, 300 or 200 nucleotides in length. The polynucleotides may be DNA (single stranded or double stranded DNA) or RNA. Hence the term "polynucleotides" as used herein may, where appropriate in the context of the invention, mean "RNA molecules". Likewise, "sample polynucleotides" may mean "sample RNA molecule" and "polynucleotides of mixed sequence from a sample" may mean "polynucleotides of mixed sequence from a sample". RNA molecules of mixed sequence in a sample may be converted to corresponding cDNA sequences of mixed sequence in a library corresponding to the RNA molecules in the original sample. The RNA molecules may be mRNA molecules and/or full length RNA molecules. The sample of mixed RNA molecules may be the full length RNA or full length mRNA molecules from single cells, or single cell nuclei. The polynucleotides may comprise a strand of RNA hybridised to a strand of DNA. The polynucleotides may comprise both DNA and RNA nucleotides, such as a DNA-RNA hybrid molecule. For example, template-switch oligonucleotides typically comprise a single stranded DNA molecule with three 3' RNA nucleotides. The polynucleotides may comprise non-canonical nucleotides or nucleotide analogues. Non-canonical nucleotides and nucleotide analogues are discussed herein. The polynucleotides may comprise DNA nucleotides and non-canonical nucleotides and/or nucleotide analogues. For example, a template-switch oligonucleotide may comprise DNA nucleotides and three 3' LNA nucleotides. A template-switch oligonucleotide may comprise solely of RNA nucleotides. A template-switch oligonucleotide may comprise DNA nucleotides, non-canonical nucleotides and/or nucleotide analogues, and RNA nucleotides.

Polynucleotides have a chemical orientation defined by the position of the linking carbon in the five-carbon sugar of each consecutive nucleotide in the chain. Polynucleotides may be manufactured by the addition of nucleotides at either the 5' end (manufacture in a 5' direction) or the 3' end (manufacture in a 3' direction) to elongate the chain. Likewise, sequence elements along the length of a polynucleotide have a sequential order defined by the directionality of the chain of nucleotides that is either 5' to 3' or 3' to 5'.

Unless otherwise indicated, the term "sample polynucleotide" or "sample DNA fragment" or "sample polynucleotide sequence" or similar used herein means the polynucleotide/RNA molecule/DNA fragment in the initial sample or a sequence corresponding thereto, for example a sequence in an amplified library polynucleotide that corresponds to and has been amplified from an initial polynucleotide/RNA molecule/DNA fragment in the initial sample. The library sequence may have additional sequence elements, such as identifier sequence(s) or PCR handle sequence(s), but these fall outside of the "sample polynucleotide sequence". Unless otherwise indicated, the term "library polynucleotide", "library DNA fragment", "library polynucleotide sequence" and similar as used herein refers to a polynucleotide/DNA that has been amplified from a polynucleotide/DNA fragment in the initial sample. Typically a library polynucleotide/DNA has been "indexed" in the sense that it has had identifier sequences added to the initial sample sequence at both ends. Unless otherwise indicated, the terms "indexed polynucleotide", "indexed DNA fragment", "indexed polynucleotide sequence" and similar as used herein refers to a sample polynucleotide/DNA/RNA/sequence or amplified/library polynucleotide/DNA/RNA/sequence with additional identifier sequences added at each end.

In some cases, an array, or isolated set, of polynucleotides is described. In some cases each polynucleotide of the array comprises at least one identifier sequence as described herein. In some cases, every polynucleotide of the array, or a sub-array thereof, has an identifier sequence, such as a UMI sequence that, alone or in combination with other sequences such as a barcode sequence, uniquely identifies each polynucleotide in the (sub-)array. As described above, it is not always necessary that each UMI sequence in the array is "unique" in the sense that it is different from every other UMI in the array of polynucleotides, as long as the UMIs permit essentially each polynucleotide in the array, or each sample polynucleotide to which a UMI is added, to be uniquely identified. In some cases, a (sub-)array of polynucleotides may all comprise the same identifier sequence, i.e. a barcode sequence, which identifies each polynucleotide as belonging to, or having been copied/amplified from, that (sub-)array. The polynucleotide array may comprise a plurality of sub-arrays, wherein the barcode sequence of each sub-array is different from the barcode sequence of each other sub-array.

In some cases the array may comprise at least 10, or at least 12, 20, 24, 48, 100, 200, 500, 1000, 2000, 5000, $10^4$, $10^5$, $10^6$, $10^7$, $10^8$, $10^9$ or $10^{10}$ polynucleotides, for example between 100 and $10^{12}$, or between 50, 200, $10^2$, $10^3$, $10^4$ or $10^5$ and $10^{11}$, or between $10^6$, $10^7$, $10^8$, or $10^9$ and $10^{10}$ polynucleotides. In some cases, the array of polynucleotides is divided into at least 10, or at least 12, 20, 24, 48, 100, 200, 500, 1000, 2000, 5000, $10^4$, $10^5$, $10^6$ or $10^7$ different sub-arrays, as described herein. A typical sub-array may comprise at least 10, or at least 12, 20, 24, 48, 100, 200, 300, 400, 500, 700,

1000, 2000, 5000, $10^4$, $10^5$, $10^6$, $10^7$, $10^8$, $10^9$ or more polynucleotides, for example between 100 and $10^{12}$, or between 1000 and $10^{11}$, or between $10^4$ and $10^{10}$ polynucleotides per sub-array.

The polynucleotides may be single-stranded or double stranded or may have both single stranded and double-stranded portions, for example as described further elsewhere herein.

*Capture polynucleotides*

An array or set of capture polynucleotides may in some cases provide a pool of identifier sequences for use in the methods described herein. A capture polynucleotide comprises a polynucleotide/analyte capture region and an identifier sequence, such as one of a mixed pool of first identifier sequences. A capture polynucleotide typically further comprises a polymerase chain reaction (PCR) handle sequence.

Examples of polynucleotide/analyte capture regions include a polythymidine sequence; a sequence of at least 10 (or 12, or 15, or 18 or 20) nucleotides for hybridising to a target polynucleotide analyte; a biotinylated nucleotide sequence; or an ATAC-med sequence. In some cases it can be useful to include a further (known) sequence in between an identifier sequence and a polynucleotide/analyte capture region (such as a polythymidine) to ensure that the identifier sequence and the polynucleotide/analyte capture region can be clearly distinguished. For example, in some cases the additional sequence may comprise or consist of the sequence "ACGCACGC". The sequence elements of the capture polynucleotides (polynucleotide/analyte capture regions, PCR handles, linkers, identifier sequences and so on) are generally arranged such that copies of the polynucleotide sequence that tag any captured analytes (e.g. at the 3 and/or 5' ends of the capture polynucleotides) include the relevant identifier sequences (e.g. UMI and/or BC sequences). The identifier sequences typically allow the tagged analytes to be identified as having been captured by a particular capture polynucleotide or as originating from a particular sample or to be digitally counted, as described further elsewhere herein. Identifier sequences included at each end of a sample polynucleotide may be used to detect amplification chimeric artefacts.

In some cases, the capture polynucleotides each comprise in order a PCR handle sequence, one of a mixed pool of identifier sequences and a polynucleotide/analyte capture region.

In some cases, the capture polynucleotides each comprise in a 5' to 3' direction a PCR handle sequence, one of a mixed pool of identifier sequences and a polynucleotide/analyte capture region.

In some cases, the capture polynucleotides each comprise in a 3' to 5' direction a PCR handle sequence, one of a mixed pool of identifier sequences and a polynucleotide/analyte capture region.

In some cases, the capture polynucleotides may comprise the following sequence elements in a 5' to 3' direction: (a) a PCR handle sequence; (b) at least one identifier sequence; and (c) a polynucleotide/analyte capture region. This design is typical for polynucleotides synthesized in a 5' to 3' direction on a solid support such as a micro-bead.

In some cases, the capture polynucleotides comprise the following sequence elements in a 3' to 5' direction: (a) optionally a linker that is cleavable to provide a free 3' hydroxyl group on the polynucleotide after cleavage; (b) a 3' end polynucleotide/analyte capture region; (c) at least one identifier sequence, optionally including a BC sequence and/or a UMI sequence, in either order or orientation; and (d) a PCR handle sequence. In some cases, the capture polynucleotides comprise the following sequence elements defined in a 3' to 5' direction: (a) optionally a linker that is cleavable to provide a free 3' hydroxyl group on the polynucleotide after cleavage; (b) a 3' end polynucleotide/analyte capture region; (c) a first PCR handle sequence, (d) at least one identifier sequence, optionally a BC sequence and/or a UMI sequence, in either order or orientation; (d) optionally a second PCR handle sequence; and (e) a 5' end polynucleotide/analyte capture region. In other cases, the polynucleotides comprise in a 3' to 5' direction: (a) 3' hydroxyl group; (b) a 3' end polynucleotide/analyte capture region; (c) optionally a first PCR handle sequence; (d) at least one identifier sequence, optionally a BC sequence and/or a UMI sequence, in either order or orientation; (e) optionally a (second) PCR handle sequence; and (f) optionally a 5' end polynucleotide/analyte capture region. This type of dual analyte capture bead, synthesized in the 3' to 5' direction, is described in PCT Publication No. WO 2021/229230. One advantage of using a polynucleotide synthesized in a 3' to 5' direction, particularly in the context of the present invention, is that pre-synthesised 3' to 5' nucleotide blocks, particularly trimer nucleotide blocks or longer, are less complex and costly to produce than 5' to 3' pre-synthesised nucleotide blocks.

In some special cases, some of the elements may overlap. In particular, in some cases, the 3' end polynucleotide/analyte capture region may partially or fully overlap with the first PCR handle sequence and/or the 5' end polynucleotide /analyte capture region may partially or fully overlap with the second PCR handle sequence.

In some cases, the polynucleotides may comprise non-nucleotide linking elements (i.e. spacers), for example phosphoramidite spacers, such as 17-O-(4,4'-Dimethoxytrityl)-hexaethyleneglycol, 1-[(2-cyanoethyl)- (N,N-diisopropyl)]-phosphoramidite (HEG). For example, a spacer may be included 3' to the 3' analyte capture region, or between the 3' analyte capture region and the bead. The polynucleotides may also comprise further sequence elements in addition to those defined herein. Typically, however, the 3' analyte capture region is immediately downstream of the 3' end hydroxyl group produced on cleavage of the linker. Likewise, the 5' analyte capture region is typically at the 5' terminus of the polynucleotide.

The polynucleotides of the present invention may in some cases comprise any of the further features described in PCT Publication No. WO 2021/229230.

In some cases, the pool of identifier sequences used in the methods may be provided on a set of capture polynucleotides, as described herein.

In some cases, the methods may comprise capturing the polynucleotides in a sample on a set of capture polynucleotides, wherein the capture polynucleotides each comprise a sample polynucleotide capture region, an identifier sequence (e.g. one of a mixed pool of first identifier sequences) and a PCR handle sequence.

For example, a method for detecting chimeric artefact polynucleotides produced during amplification of a mixed sample of polynucleotides, may comprise capturing polynucleotides in a sample on a set of capture polynucleotides, wherein the capture polynucleotides each comprise a sample polynucleotide capture region, one of a mixed pool of first identifier sequences, and a PCR handle sequence; adding a second identifier sequence from a mixed pool of second identifier sequences to the end of polynucleotides in the sample not bound by the capture polynucleotide); amplifying the polynucleotides to generate a library of amplified sample polynucleotides flanked by first and second identifier sequences; sequencing the library; identifying pairings of first and second identifier sequences in library polynucleotides; counting library polynucleotides having identified pairings of identifier sequences; identifying first and second identifier sequence pairings that are under-represented in the library; and identifying an amplified sample polynucleotide flanked by an underrepresented pairing of first and second identifier sequences as a chimeric artefact polynucleotide.

In other cases, or in addition, a method for detecting chimeric artefact polynucleotides produced during amplification of a mixed sample of polynucleotides may comprise: capturing polynucleotides in a sample on a set of capture polynucleotides, wherein the capture polynucleotides each comprise a sample polynucleotide capture region, one of a mixed pool of first identifier sequences, and a PCR handle sequence; adding a second identifier sequence from a mixed pool of second identifier sequences to the end of polynucleotides in the sample not bound by the capture polynucleotide; amplifying the polynucleotides to generate a library of amplified sample polynucleotides flanked by first and second identifier sequences; sequencing the library; identifying pairings of first and second identifier sequences in library polynucleotides; identifying pairings as mismatch pairings when they comprise (a) a first identifier sequence in common with another pairing in the library having a different second identifier sequence to the mismatch pairing; and (b) a second identifier sequence in common with another pairing in the library having a different first identifier sequence to the mismatch pairing; and identifying an amplified sample polynucleotide flanked by a mismatch pairing of first and second identifier sequences identified in the previous step as a chimeric artefact polynucleotide.

In some cases, a method of producing a library of polynucleotides of mixed sequence from a sample may comprise: capturing polynucleotides of the sample on a set of capture polynucleotides, wherein the capture polynucleotides each comprise a sample polynucleotide capture region, an identifier sequences and a PCR handle sequence; performing reverse transcription of captured sample polynucleotides using a template switch reverse polymerase and primed using the capture polynucleotides to generate cDNA polynucleotides having 3' end non-templated nucleotides; annealing a set of template switch oligonucleotides (TSOs) to the 3' end non-templated nucleotides, wherein each TSO comprises a PCR handle sequence and one of a mixed pool of second identifier sequences; and amplifying the polynucleotides using the capture polynucleotide PCR handle sequences and TSO PCR handle sequences to generate a library of amplified sample polynucleotides flanked by first and second identifier sequences.

In some cases, a method of producing a library of polynucleotides of mixed sequence from a sample may comprise: capturing polynucleotides of the sample on a set of capture polynucleotides, wherein the capture polynucleotides each comprise a sample polynucleotide capture region, one of a mixed pool of first identifier sequences and a PCR handle sequence; performing reverse transcription of captured sample polynucleotides using a template switch reverse polymerase and primed using the capture polynucleotides to generate cDNA polynucleotides having 3' end non-templated nucleotides; annealing a set of template switch oligonucleotides (TSOs) to the 3' end non-templated nucleotides, wherein each TSO comprises a PCR handle sequence and one of a mixed pool of second identifier sequences; and amplifying the polynucleotides using the capture polynucleotide PCR handle sequences and TSO PCR handle sequences to generate a library of amplified sample polynucleotides flanked by first and second identifier sequences.

The amplification step of the methods described herein may comprise amplifying the cDNA using one or more primers comprising a purification tag. For example, the amplification step may comprise amplifying the cDNA using a tagged primer against the capture polynucleotide PCR handle sequence and a non-tagged primer against the TSO PCR handle sequence, a non-tagged primer against the capture polynucleotide PCR handle sequence and a tagged primer against the TSO PCR handle sequence, or a tagged primer against the capture polynucleotide PCR handle sequence and a tagged primer against the TSO PCR handle sequence. This step results in a library of amplified cDNA flanked by first and second identifier sequences and tagged on one or both sides with the purification tag. The purification tag may be used to separate tagged polynucleotides from non-tagged polynucleotides. This allows artefacts introduced during the reverse transcription step to be removed, as these artefacts will not be amplified using the tagged primers. The method may therefore comprise separating tagged polynucleotides from non-tagged polynucleotides. Any suitable method of separating tagged polynucleotides from non-tagged polynucleotides may be used. The method may comprise separating tagged polynucleotides from non-tagged polynucleotides by pull down of the tagged polynucleotides, for example by binding tagged polynucleotides indirectly or directly to a solid support (e.g. a bead, a paramagnetic particle). The tagged polynucleotides may be bound by an antibody specific for the tag. The method may comprise a wash step.

The purification tag typically retains its ability to separate tagged polynucleotides from non-tagged polynucleotides following one or more PCR cycles. The purification tag may be biotin. The amplification step may comprise using biotinylated primers, such as biotinylated primers against the capture polynucleotide PCR handle sequences and/or biotinylated primers against the TSO PCR handle sequences. This step results in a library of amplified cDNA flanked by first and second identifier sequences and tagged with a biotin molecule. The biotin tag may be used in a "clean-up" step to separate biotin-tagged polynucleotide molecules from polynucleotide molecules that do not have a biotin tag. The method may therefore comprise separating biotin tagged polynucleotides from non-biotin-tagged polynucleotides by streptavidin pull down. The streptavidin pull down may be performed using streptavidin beads, such as paramagnetic beads. The biotin tagged polynucleotides may be released from the streptavidin by photocleavage. Variants of the biotin/streptavidin system may be used. For example, the tagged primers may be primers comprising desthiobiotin. The tagged primers may be primers comprising iminobiotin. Desthiobiotin and iminobiotin have a lower binding affinity to streptavidin than biotin, enabling the desthiobiotion or iminobiotin to be displaced on streptavidin by biotin. Desthiobiotin tagged polynucleotides and iminobiotin tagged polynucleotides may be released from the streptavidin using excess free biotin. Variants of streptavidin may be used, for example avidin or neutravidin.

Alternative methods of tagging polynucleotides are known in the art. For example, the tag may be digoxigenin. Digoxigenin is a small hapten that can be conjugated to the ends of polynucleotides. The amplification step may comprise using digoxigenin-tagged primers, such as digoxigenin-tagged primers against the capture polynucleotide PCR handle sequences and/or digoxigenin-tagged primers against the TSO PCR handle sequences. This step results in a library of amplified cDNA flanked by first and second identifier sequences and tagged with a digoxigenin molecule. The digoxigenin tag may be used in a "clean-up" step to separate digoxigenin-tagged polynucleotide molecules from polynucleotide molecules that do not have a digoxigenin tag. The method may comprise separating digoxigenin tagged polynucleotides from non-digoxigenin-tagged polynucleotides using anti-digoxigenin antibodies, which may in turn be directly or indirectly attached to beads (similar to the biotin/streptavidin approach). The tag may be a fluorophore (e.g. FAM or FITC). The fluorophore may be incorporated into oligonucleotides and tagged polynucleotides may be separated from non-tagged polynucleotides using antibodies specific for the fluorophore, which may in turn be directly or indirectly attached to beads. The tag may be a polynucleotide that comprises a specific sequence that is complementary to a specific antisense capture oligonucleotide, which may in turn be directly or indirectly attached to beads.

The capture polynucleotides and/or the TSOs described herein may comprise a purification tag. The reverse transcription may generate cDNA polynucleotides having 3' end non-templated nucleotides and tagged on one or both sides with a purification tag. Following the reverse transcription step, and preferably prior to the amplification step, the methods described herein may comprise separating tagged polynucleotides from non-tagged polynucleotides. This allows artefacts introduced during the reverse transcription step to be removed. Any of the tags discussed above with respect to tagged primers may be used. The tagged polynucleotides may be separated from non-tagged polynucleotides by pull down of the tagged polynucleotides, for example by binding tagged polynucleotides indirectly or directly to a solid support (e.g. a bead, a paramagnetic particle). The tagged polynucleotides may be bound by an antibody specific for the tag. The method may comprise a wash step. The tag may be a biotin tag, such as a desthiobiotin tag. Following the reverse transcription step, and preferably prior to the amplification step, the methods described herein may comprise separating biotin tagged polynucleotides from non-biotin-tagged polynucleotides by streptavidin pull down. This allows artefacts introduced during the reverse transcription step to be removed. Other tags may be used to separate tagged polynucleotides from non-tagged polynucleotides.

One or more steps of separating tagged polynucleotides from non-tagged polynucleotides may be performed during or after the amplification step. For example, the amplification step may comprise a number of PCR cycles, followed by a separating step, followed by additional PCR cycles, followed by an additional separation step. The additional PCR cycles may comprise the tagged primers described herein and the additional clean-up step may comprise the separating step as described herein.

Libraries produced by such methods may be used in a method of detecting chimeric artefacts as described herein.

*Polynucleotide/analyte capture regions*

The polynucleotide/analyte capture region(s) may be any nucleotide sequence suitable for capturing polynucleotides/analyte in a sample. In some cases the analyte(s) may be biological analytes or may be selected from polynucleotides DNA and/or RNA, or from oligonucleotides, DNA, cDNA, RNA, mRNA, rRNA, tRNA, snRNA, siRNA and/or ribozymes, proteins, polypeptides and/or peptides, cell surface receptors or cells. In some cases the analytes may additionally be selected from amino acids, metal ions, inorganic salts, polymers, nucleotides, oligonucleotides, polynucleotides, dyes, bleaches, pharmaceuticals, diagnostic agents, recreational drugs, explosives and/or environmental pollutants. Such analytes may be captured, for example, by an aptamer.

Typically the polynucleotide/analyte capture region(s) sequence may be at least 10, or at least 15, 20, 25 or 30 nucleotides in length, such as from about 15 to about 50, from about 20 to about 40 or from about 25 to about 35 nucleotides. In some cases, the polynucleotide/analyte capture region(s) may comprise one or more nucleotide analogues, such as analogues described herein, that form double-stranded hybrids with higher stability than natural nucleotides. In this case, the polynucleotide/analyte capture region(s) could be shorter, such as at least 3, 4, 5, 6, 8 or 9, for example between 3 and 50, or 40 or 30 or 20 nucleotides in length, provided that the polynucleotide/analyte capture region(s) was capable of hybridizing to target polynucleotide/ analyte such that the polynucleotide/analyte sequence can be amplified as described herein. One or both polynucleotide/analyte capture regions may include nucleotide analogues as described herein.

In some cases, an polynucleotide/analyte capture region may be a DNA capture region. In some cases, a polynucleotide/analyte capture region may be an RNA or mRNA capture region. In some cases, a polynucleotide/analyte capture region may be a polypeptide capture region.

In one example, a polynucleotide/analyte capture region, particularly a 3' polynucleotide/analyte capture region, may be a polythymidine. Polythymidine may hybridise to and capture any polynucleotide in the sample that comprises a suitable polyadenosine, such as polyadenylated mRNA. Typically the polythymidine may be at least 10, or at least 15, 20, 25 or 30 thymidines in length, such as from about 15 to about 50, from about 20 to about 40 or from about 25 to about 35 thymidines. When the capture polynucleotide is attached to a bead, the polythymidine may be immediately to a 3' side of the cleavage site of a linker that provides a free 3'hydroxyl group after cleavage (for example as described in WO 2021/229230). When the capture polynucleotide is not attached to bead, the polythymidine may comprise the hydroxyl group at the free 3' end of the capture polynucleotide.

In other cases, an analyte capture region(s) may comprise or consist of an aptamer. Aptamers can be produced using SELEX (Stoltenburg, R. et al. SELEX: A (r)evolutionary Method to Generate High-Affinity Nucleic Acid Ligands. Biomol Eng 24, 381-403 (2007); Tuerk, C. & Gold, L. Systematic evolution of ligands by exponential enrichment: RNA ligands to bacteriophage T4 DNA polymerase. Science 249, 505-510 (1990); Bock, L.C. et al. Selection of single-stranded DNA molecules that bind and inhibit human thrombin. Nature 355, 564-566 (1992)) or NON-SELEX (Berezovski, M. et al. Non-SELEX Selection of Aptamers. J Am Chem Soc 128, 1410-1411 (2006)). Typically, an aptamer may be at least 15 nucleotides in length, such as from about 15 to about 50, from about 20 to about 40 or from about 25 to about 30 or nucleotides in length. An aptamer may bind to analyte such as small molecules, proteins, nucleic acids or cells. Aptamers may be designed or selected to bind to pre-determined target analyte(s). In one example, the aptamer may bind to a Coronaviridae protein or SARS-CoV-2 protein, such as any of the SARS-CoV-2 structural protein sequences provided herein.

In some cases, a polynucleotide/analyte capture region(s) may comprise or consist of a biotinylated nucleotide sequence. Nucleotides or polynucleotides may be biotinylated using methods known in the art. Typically the biotinylated sequence may be at least 10, or at least 15, 20, 25 or 30 nucleotides in length, such as from about 15 to about 50, from about 20 to about 40 or from about 25 to about 35 nucleotides. A biotinylated capture region may be used to capture any suitable target polynucleotide/analyte comprising streptavidin or avidin.

In some cases, the polynucleotide/analyte capture region(s) may comprise or consist of a nucleotide sequence designed to hybridise to a complementary sequence in a target polynucleotide/analyte. In some cases, the polynucleotide/analyte capture region is for capturing/hybridising to transposed DNA. In this case a polynucleotide/analyte capture region may comprise or consist of a sequence that is complementary to transposed DNA in a sample, for example to a transposed MEDS DNA sequence. In some cases, the sequence may be gene or transcript-specific, such as a polynucleotide sequence that is complementary to, or at least 80%, 85%, 90%, 95%, 98% or 99% complementary to, a viral sequence, a bacterial sequence or a sequence associated with a disease or disorder, such as a sequence from a cancer-associated antigen or a neoantigen. In some cases, the polynucleotide capture region(s) may hybridise to a nucleotide sequence that encodes a part of a pathogen, a bacteria, and/or a virus, such as Coronaviridae protein or SARS-CoV-2 protein, as described in described in PCT Publication No. WO 2021/229230.

In other cases, the polynucleotide sequence may be designed to capture a polynucleotide tag added to analyte of interest.

In some cases, a 5' polynucleotide/analyte capture region may be absent or may comprise any of the characteristics described herein for the 3' polynucleotide/analyte capture region. Typically, however, a 5' polynucleotide/analyte capture region does not consist of a polythymidine sequence because mRNA hybridised to polythymidine at the 5' analyte capture region cannot be readily/directly converted to cDNA by reverse transcription from the 5' end.

*Template switch oligonucleotides*

An array or set of TSO's may in some cases provide a pool of identifier sequences for use in the methods described herein. During reverse transcription of a target/sample polynucleotide (typically an mRNA sequence) using a template switch reverse transcriptase, the reverse transcriptase will add a number of non-templated nucleotides (typically three cytosines) to the 3' end of the newly synthesised cDNA. A TSO anneals to the non-templated nucleotides and the reverse transcriptase switches templates from the target polynucleotide to the TSO to thereby generating a sequence, complementary to the sequence of the TSO, at the 3' end of the newly synthesised cDNA.

Typically, the TSOs each comprise (in a 5' to 3' direction) (a) a PCR handle sequence, (b) an identifier sequence, such as one of a mixed pool of second identifier sequences, and (c) a 3' end template switching sequence which can hybridise to 3' non-templated nucleotides added to a cDNA strand by a reverse transcriptase.

The 3' end template switching sequence is typically an RNA sequence or a locked nucleic acid (LNA) sequence. In some cases, the 3' end template switching sequence is three riboguanosines (rGrGrG).

The TSO is typically a DNA-RNA hybrid molecule. The TSO is typically a single-stranded DNA molecule with three 3' RNA bases. The TSO may be an RNA molecule, e.g. comprising a single-stranded RNA molecule with three 3' RNA bases. The TSO may be a hybrid of different types of nucleotide molecules, e.g. a hybrid of DNA nucleotides, RNA nucleotides, non-canonical nucleotides and/or nucleotide analogues. The TSO may be a single-stranded DNA molecule with three 3' LNA bases. The TSO may be a single-stranded RNA molecule with three 3' LNA bases. The TSO may comprise DNA nucleotides, RNA nucleotides, non-canonical nucleotides and/or nucleotide analogues anywhere in its sequence, such as in the identifier sequence, as described herein. For example, the TSO may be a single-stranded DNA molecule with three 3' RNA bases and non-canonical nucleotides and/or nucleotide analogues in the identifier sequence.

In some cases, the TSO is up to 100 nucleotides in length. For example, the TSO may be at least 20, 25, 30, 35, 40, 45 or 50 nucleotides in length. The TSO may be up to 30, 35, 40, 45, 50, 55, 60, 65, 70, 75, 80, 85, 90 or 95 nucleotides in length. The TSO may be from 20-200, 20-100, 25-80, 30-70, 35-60 or 40-60 nucleotides in length. A typical TSO comprising an identifier sequence made up of blocks that are three nucleotides in length may is about 60 to 95 nucleotides in length.

In some cases, the identifier sequence of the TSO may be at least 8, 9, 10, 15, 20, 25 or 30 nucleotides in length. The identifier sequence of the TSO may be up to 10, 15, 20, 25, 30, 35, 40 or 45 nucleotides in length. The identifier sequence of the TSO may be from 8 to 50, 10-40 or 12 to 36 nucleotides in length, such as 30 or 36 nucleotides in length.

In some cases, the identifier sequence of the TSO may be made up of nucleotide blocks as described herein. Typically, the nucleotide block is a nucleotide dimer or a nucleotide trimer. The identifier sequence of the TSO may comprise at least 2, more typically at least 3, 4, 5, 6, 7 or 8 sequence units (where a sequence unit is either a single nucleotide or a nucleotide block as described herein) and up to 12, 13 or 14 sequence units or more, more typically 6 to 14, 7 to 13, 10 to 14, 13 to 14, 7 to 9, or 8 or 10 or 12 sequence units.

In some cases it can be useful to include a further (known) sequence in between an identifier sequence and the PCR handle or the template switching sequence, to ensure that the identifier sequence and the polynucleotide capture region can be clearly distinguished. For example, in some cases the additional sequence may comprise or consist of the sequence "ACGCACGC".

In some cases, the TSO may comprise the sequence

AAGCAGTGGTATCAACGCAGAGT, or AAGCAGTGGTATCAACGCAGAGTGAATrGrGrG (where the last 3 bases are RNA bases), or TACACGACGCTCTTCCGATCTrGrGrG. This sequence may overlap partially or fully with the PCR handle of the TSO.

In some cases, the methods comprise capturing polynucleotides of the sample on a set of capture polynucleotides, wherein the capture polynucleotides each comprise a sample polynucleotide capture region, one of a mixed pool of first identifier sequences and a PCR handle sequence; performing reverse transcription of captured sample polynucleotides using a template switch reverse polymerase and primed using the capture polynucleotides to generate cDNA polynucleotides having 3' end non-templated nucleotides; annealing a set of template switch oligonucleotides (TSOs) to the 3' end non-templated nucleotides, wherein each TSO comprises a PCR handle sequence and one of a mixed pool of second identifier sequences; and amplifying the polynucleotides using the capture polynucleotide PCR handle sequences and TSO PCR handle sequences to generate a library of amplified sample polynucleotides flanked by first and second identifier sequences.

The methods may further comprise sequencing the library; identifying pairings of first and second identifier sequences in library polynucleotides; counting library polynucleotides having identified pairings of identifier sequences; identifying first and second identifier sequence pairings that are under-represented in the library; and identifying an amplified sample polynucleotide flanked by an underrepresented pairing of first and second identifier sequences as a chimeric artefact polynucleotide.

The methods may further comprise sequencing the library; identifying pairings of first and second identifier sequences in library polynucleotides; identifying pairings as mismatch pairings when they comprise (a) a first identifier sequence in common with another pairing in the library having a different second identifier sequence to the mismatch pairing; and (b) a second identifier sequence in common with another pairing in the library having a different first identifier sequence to the mismatch pairing; and identifying an amplified sample polynucleotide flanked by a mismatch pairing of first and second identifier sequences identified in the previous step as a chimeric artefact polynucleotide.

*Annealed oligonucleotide pairs*

An array or set of annealed oligonucleotide strand pairs, typically DNA (also referred to herein as "adapters"), may provide the pool(s) of first and/or second identifier sequences described herein. In particular, an identifier sequence may be added at one end of a sample DNA fragment by ligating an annealed oligonucleotide strand pair comprising an identifier sequence to the sample DNA fragment. Annealed oligonucleotide strand pairs as described herein may be added at both ends of sample DNA fragments to provide the first and second identifier sequences flanking a sample DNA sequence.

The annealed oligonucleotide strand pairs are double stranded at least at one end or may have a single nucleotide overhang. Ligating double-stranded DNA to another double-stranded DNA or portion of DNA is more efficient than ligating single-stranded DNA to a double-stranded DNA. Ligation is even more efficient if both DNA molecules have complementary single nucleotide overhangs, typically an adenosine (A) on one molecule and a complementary thymidine (T) on the other molecule (at the ends that are to be joined/ligated). Hence, the annealed oligonucleotide strand pairs typically comprise a double stranded region, referred to here as an adapter region or adapter sequence, which may comprise a single nucleotide overhang. The adapter region is used to ligate the annealed oligonucleotide strand pair to a sample DNA fragment. The single nucleotide overhang of the adapter region is typically a T or an A, most typically a T. The adapter region/end may be ligated to a sample DNA fragment using a DNA ligase.

The sample polynucleotides may be DNA fragments, and the first identifier sequences and PCR handle sequences and/or second identifier sequences and PCR handle sequences are provided on an array of annealed oligonucleotide strand pairs as described herein. A first oligonucleotide strand in a pair may comprise or consist of (in a 5' to 3' direction): (a) a PCR handle sequence; (b) one of the mixed pool of identifier sequences; and (c) an adapter sequence. The annealed oligonucleotide strand pairs of the array, each comprising one of the mixed pool of identifier sequences (i.e. the first pool of identifier sequences or the second pool of identifier sequences), together provide the pool of identifier sequences. The other, second oligonucleotide strand of a pair comprises or consists of a sequence that is complementary to and annealed to the adapter sequence to form the double-stranded end, or a double-stranded end with a single nucleotide 3' strand overhang as described above. In methods that use an array of annealed oligonucleotide strand pairs as described herein to provide the first and/or second pool of identifier sequences, the adapter end of the oligonucleotides are ligated to one or both ends of sample DNA fragments. Primers to the PCR handle sequences are used to amplify the DNA fragments together with the identifier sequences provided by the oligonucleotides.

The oligonucleotides may be produced by synthesising each strand separately and then annealing the strands together to produce the array of annealed oligonucleotide strand pairs. The strand comprising the PCR handle sequence and the identifier sequence may be produced using methods described elsewhere herein. For example, a single stranded DNA strand comprising (or consisting of) the PCR handle sequence may be synthesised, then the mixed pool of identifier sequences may be added in a 5' to 3' direction, for example using degenerative synthesis and/or pre-synthesised nucleotide blocks. Then a single stranded adapter region may be added to provide a mixed pool of identifier sequence strands. This pool of stands may then be annealed to strands comprising the reverse complementary sequence of the adapter sequence on the first strand, or the reverse complementary sequence but with one less nucleotide at the 5' end so that the PCR handle/identifier sequence strand has a single nucleotide 3' overhang. In some cases the second strand may also comprise a region that is reverse complementary to the PCR handle sequence and anneals thereto. A DNA or non-DNA linker, for example a phosphoramidite spacer, such as 17-O-(4,4'-Dimethoxytrityl)-hexaethyleneglycol, 1-[(2-cyanoethyl)- (N,N-diisopropyl)]-phosphoramidite (HEG), may be included in the second strand between the reverse complement PCR handle and adapter sequences in the arrays. In general, the linker is not reverse complementary to and does not anneal to the identifier sequence in the opposite strand, though there may be some regions of complementary or hybridization dependent sequence of the linker and the sequence of each identifier sequence. Alternatively, the double stranded adapter region may be extended to convert the single stranded region comprising the PCR handle sequence and identifier sequence to a fully double-stranded sequence.

Hence, in some cases, the annealed oligonucleotide strand pairs are reverse complementary (at least to the extent necessary to remain annealed at room temperature) and annealed across their full length. In other cases, the oligonucleotides are single stranded across the PCR handle and identifier sequences (a 5' overhang), but double stranded in the adapter sequence region used for ligation to a sample DNA fragment. In other cases, the two strands have reverse complementary sequences across the PCR handle and adapter sequences (at least to the extent necessary to remain annealed at room temperature), but have different, non-reverse complementary sequence across the region of the identifier sequence(s). This may be advantageous compared to having a single stranded PCR handle and identifier sequence to avoid the introduction of artefacts or bias or a loss of efficiency if the single strand self-anneals or forms secondary structures (particularly if the identifier sequences use nucleotide blocks that are reverse complementary to other nucleotide blocks that could be included in the sequence, e.g. if the nucleotide blocks have the sequences AAA, TTT, GGG and CCC) or binds to sample polynucleotides. Hence the inclusion of a region that is reverse complementary to the PCR handle sequence can constrain the identifier sequence and prevent un-wanted binding. In the second/non-PCR handle (i.e. reverse complementary) strand, an identifier sequence is not needed between the regions that anneal to the PCR handle and adapter sequences (in the first/identifier sequence strand). The only requirement on this section of the second strand is that it does not interfere with annealing of the other two regions to the first strand. Hence a DNA or non-DNA linker, as described above, may be used. In some cases the linker may be about the same length as the identifier sequence or the region of the first strand containing the identifier sequence that does not anneal to the second strand. However, a shorter linker could also be used, for example a stretch of at least 4, or at least 5 or 6 nucleotides.

In some cases, the PCR handle/identifier/first strand comprises thymines (T) (and optionally no uracils) and the other/second/reverse complementary strand comprises uracils (U) (and optionally no thymines). In this case, after the oligonucleotides are ligated to sample DNA fragments, and before amplification using the PCR handle sequences, a USER enzyme may be used to selectively remove/digest the uracil containing sequences. This includes at least the section between the reverse complement of the adapter and PCR handle sequences, or the section of the strand opposing the identifier sequence in the first strand. Hence, digesting the oligonucleotide sequences comprising uracils produces a single stranded region comprising the identifier sequence. If the whole section complementary to the PCR handle sequence is also removed/digested, then a 5' end overhang is produced, but this is not essential. The remaining double stranded region, which comprises the sample DNA sequence and optionally any portion of the adapter region that was not digested, can be extended (using polymerase) to provide a complementary double strand across the identifier sequence, and re-synthesise any digested (portion of) the reverse complementary strand of the PCR handle region. For example, this can be done at the beginning of PCR amplification of the indexed DNA fragments by replacing the standard hot start with a short extension step (e.g. about 5 mins at 72 C). Any standard non-hot start DNA polymerase could be used, for example a proof reading DNA polymerase, such as Kappa HiFi DNA polymerase, or Klenow fragment (DNA polymerase I).

A USER enzyme includes a mixture of Uracil DNA glycosylase (UDG) and the DNA glycosylase-lyase Endonuclease VIII that remove U bases and break the phosphodiester backbone so that the base-free deoxyribose is released. Short regions of annealed DNA strand in between digested U bases in the second strand melt away leaving the opposing sequence (which typically does not contain any uracils) as single strand. Typically stretches of less than about 10 bases between digested Us will melt away. Hence, including at least one U every 10 bases will result in the removal/digestion of the uracil containing strand in the region between the most 5' and most 3' uracils in the second strand, or between the most 5' uracil and the 3' end of the second strand if a uracil is included within about the last 10 bases at the 3' end of the strand. Hence, these oligonucleotides typically comprise at least one U in the reverse complementary/second strand either side of the identifier sequence in the first/opposite strand, for example, at least one U in the region that is reverse complementary to the adapter sequence and at least once U in the region that is reverse complementary to the PCR handle sequence. The oligonucleotides may further comprise at least one U every 10 bases in between these uracils either side of the identifier sequence region. This will be sufficient to ensure that the reverse complement of the identifier sequence is synthesised by the polymerase after the digestion step and across the full length of the identifier sequence. However, if the linker between the two reverse complementary regions of the oligonucleotides (i.e. the region containing the reverse complement of the PCR handle sequence and the region containing the reverse complement of the adapter sequence), then it may not be necessary to include further uracils in this region, or indeed the linker may be any suitable non-DNA linker, as described elsewhere herein. Hence, the second/reverse complementary strand may in some cases include some thymines, as long as there are sufficient uracils to digest/melt away any sequence in the second strand that opposes the identifier sequence on the first strand. It would usually be sufficient to simply synthesise the second strands to be reverse complementary across the PCR handle sequence and adapter regions of the first strand, but replacing all of the thymines (reverse complement of adenines in the first strand) with uracils.

The annealed oligonucleotide pairs or single stranded oligonucleotides may consist only of features (a) to (c) set out above. Other spacer elements, or short known additional sequences (for example, up to 15, or up to 12, 10, 8 or 6 nucleotides in length), or additional sequence elements, may be included, as described elsewhere herein. In some cases the annealed oligonucleotide strand pairs (or the first strand comprising the PCR handle sequence and the identifier sequence) may be up to 200, or up to 150, or 120, or 100 nucleotides in length, for example 25 to 200, or 30 to 150, or 40 to 120 nucleotides in length. For example, a typical PCR handle sequence could be 18 to 25 or 26 nucleotides in length, but could be shorter, in particular if nucleotide analogues such as LNA are used. Likewise, the adapter also needs to be long enough for the two strands to remain annealed at room temperature (20 to 25 C), so is typically around 18 to 25 nucleotides in length, but could be shorter if nucleotide analogues are used. These sequences could also be longer, such as p to 50 or up to 100 nucleotides in length. Identifier sequence lengths are described elsewhere herein.

Methods of producing a (amplified) library of DNA fragments of mixed sequence from a sample are described herein. The library of DNA fragments is an indexed library of amplified DNA fragments, wherein the sample DNA fragment sequence is flanked at both ends by an identifier sequence, or with a first identifier sequence at one end/ flank, and a second identifier sequence at the other end/flank. The identifier sequences are provided by an array of annealed oligonucleotide strand pairs as described above, or by a first and second array providing the first and second identifier sequences respectively. An annealed oligonucleotide strand pair from an array is ligated at each end of DNA fragments of the sample, using standard methods as known in the art, for example using a T4 ligase.

A variety of commercial solutions exist to end repair and A-tail sample DNA and improve the efficiency of DNA ligation, as described above. Typically this is achieved with a combination of T4 DNA polymerase, polynucleotide kinase (PNK) and Taq DNA polymerase. At mesophilic temperatures, the T4 DNA polymerase fills in 5' overhangs (via 5'->3' polymerase activity) and removes 3' overhangs (via 3'->5' exonuclease activity), resulting in blunt ends, and the PNK phosphorylates 5' ends. The temperature is then raised, destroying the T4 DNA polymerase and PNK and the thermophilic Taq polymerase adds 3' single base adenosine overhangs.

End-repaired and A-tailed DNA is combined with the adapter regions of the oligonucleotides, as described above. Complementarity between the 3' T overhang of the adapter and the 3' A overhang on the sample DNA stabilise their interaction and the ligase (e.g. T4 ligase) covalently joins the two molecules. Since this happens at both ends of the sample DNA, this results in sample DNA fragments that are flanked at both ends by adapters comprising PCR handle sequences and identifier sequences, ready for PCR amplification.

If adapters incorporating uracil have been used, these can be digested with USER enzyme (a mixture of Uracil DNA glycosylase (UDG) and the DNA glycosylase-lyase Endonuclease VIII) as described above.

In other cases, the annealed oligonucleotide strand pairs comprise a single stranded region including or consisting of a PCR handle sequence and an identifier sequence, as described above.

After clean up (typically with paramagnetic SPRI beads), samples are PCR amplified using a primer against the PCR handle of the adapters and a non-hotstart DNA polymerase. Before the first cycle of the PCR begins, the 5' overhangs of the adapters are filled in by extension (typically at 72 C) e.g. for about 5 minutes. This ensures the adapters are complimentary across their full length, including the identifier sequence, prior to PCR amplification.

The sample DNA fragment ligated to annealed oligonucleotide strand pairs at both ends are then amplified using the oligonucleotide handle sequences at each end to generate a library of amplified sample DNA fragments flanked by a first identifier sequence from a mixed pool of first identifier sequences (on one side of the sample DNA fragment sequence) and a second identifier sequence from a mixed pool of first identifier sequences (on the other side of the sample DNA fragment sequence).

An indexed library can be produced by a method described above. The library is an indexed library of amplified DNA fragments from a sample of mixed sequence DNA fragments, wherein the library DNA fragments are flanked by first and second identifier sequences.

A library as described above may be produced by directly ligating a single stranded oligonucleotide from an array of single-stranded oligonucleotides as described above, to either end of sample DNA fragments, for example using T4 ligase. The single stranded oligonucleotides ligated to the sample DNA fragments may then be extended to convert the single stranded regions to double strand, as described above.

*PCR handle sequences*

A PCR handle sequence hybridizes to PCR oligonucleotide primers during a PCR reaction. Typically a PCR handle sequence may be at least 15, 16, 17, 18, 19 or 20 nucleotides in length and/or up to and 21, 22, 23, 24, 25, 30 or 35 nucleotides in length, for example about 15 to 30, or 18 to 25 nucleotides. In some cases, the PCR handle sequence(s) may comprise one or more nucleotide analogues, such as analogues described herein, that form double-stranded hybrids with higher stability than natural nucleotides. In this case, the PCR handle sequence(s) could be shorter, such as at least 3, 4, 5, 6, 8, 9, 10, 11, 12, 13 or 14 nucleotides in length, provided that the PCR handle sequence(s) was capable of hybridizing to PCR oligonucleotide as described herein. One or both PCR handle sequence(s) may include nucleotide analogues as described herein.

In some cases the PCR handle sequences added at each end of the sample polynucleotides may be the same. In other cases, difference or complementary sequences may be used. In some cases one or more of the PCR handle sequences may comprise or consist of one of the following sequences: 5'- GTGGTATCAACGCAGAGTAC-3'; 5'-GTC CGAGCGTAGGTTATCCG-3' The PCR handles may be compatible with standard Illumina sequencing to eliminate the need for custom read 1 sequencing primers to read BC/UMI.

*Micro-particles*

Micro-particles may comprise a micro-bead and an (sub-)array of capture polynucleotides as described herein. Microbeads are typically less than 500 μm, or less than 400 μm, 300 μm or 200 μm in diameter, for example, between 10 and 500 μm , 20 and 400 μm, 40 and 300 μm , or 50 and 200 μm. Typically a micro-bead is approximately spherical or sphere-like. Micro-beads with surface-attached capture polynucleotides are well-known in the art and may be made from, for example, a biocompatible polymer such as polystyrene, polyacrylamide or hydroxylated methacrylic polymer, or from controlled pore glass. The micro-particles described herein may comprise a micro-bead as described herein with an array of capture polynucleotides as described herein, wherein each polynucleotide in the array is attached to the bead at the 3' or the 5' end of the polynucleotide. The opposite end (5' or 3') is typically free in solution.

Dissolvable beads and hydrogel beads have also been described and are encompassed in the present disclosure. Dissolvable beads may, for example, be made from crosslinked acrylamide with disulfide bridges that are cleaved with dithiothreitol. An array of capture polynucleotides may be embedded in the bead matrix and released when the bead is dissolved. A micro-particle comprising a micro-bead and an array of capture polynucleotides bound to the micro-bead, as described herein, may be a typical micro-bead with surface bound capture polynucleotide or a dissolvable bead with embedded polynucleotide.

Capture polynucleotides may be synthesised on the bead using methods known in the art or described herein. For example, the phosphoramidite method may be used, in which one nucleotide or pre-synthesised nucleotide block is added per synthesis cycle. The identifier sequence(s) may be generated as described elsewhere herein. Other and/or longer sequence elements may in some cases be added using enzymatic ligation methods, such as using DNA ligase, chemical ligation methods, such as phosphoramidate ligation, and/or click chemistry ligation methods, such as the azide-alkyne cycloaddition reaction. Suitable methods are known in the art.

The number of micro-particles may be selected for a specific purpose or experiment, such as to capture sample analytes for the generation one or more libraries. Typically a plurality of micro-particles comprises at least 1000, or at least 10,000, or 50,000, or 100,000, or 150,000, for example between 1000, 10,000, or 50,000, or 100,000, or 150,000 and $10^7$, or between 10,000 and $10^6$, or between 50,000 and 600,000, or between 50,000 and 400,000, or between 10,000 or 100,000 and 300,000 micro-particles. Typically the set of capture polynucleotides associated with each micro-particle have a different shared barcode sequence from the capture polynucleotides associated with essentially each of the other micro-particle. Hence, the analytes that are captured by the set of capture polynucleotides associated with each micro-particle can be distinguished. The potential diversity of barcode sequences is dependent on the number of nucleotide blocks that make up the barcode sequence and the size of the nucleotide block pools, as described above. The barcode sequence will typically be long enough that the potential sequence diversity is well in excess of the plurality of micro-particles, typically at least 50x or 100x in excess, or least 10, 20, 50, 100, 200 or 500 times in excess.

*Solid support-based arrays*

A surface comprising a plurality of wells or discrete pre-determined positions is described herein, wherein each well or discrete pre-determined position is associated with a (sub-)array of capture polynucleotides as described herein. Typical examples include solid supports such as well plates (for example, one or more 6, 12, 24, 48, 96, 384, 1536 or 3456 well plates) microplates, or slides, as are known in the art.

The capture polynucleotides may be attached to the surface using chemistry known in the art. The polynucleotide may be attached to the solid support through a linker of non-specific bases. The surface may be made from any suitable materials, for example, a biocompatible polymer such as polystyrene, polyacrylamide or hydroxylated methacrylic polymer, or from controlled pore glass.

In some cases, the capture polynucleotides may be synthesised on the solid support in a similar way to synthesis on a micro-bead, as described above. In other cases, the polynucleotides may be pre-synthesised and then divided by aliquoting different sub-sets (sub-arrays) of the capture polynucleotides and/or micro-particles, as described herein, into the wells or onto the pre-defined discrete spatial positions.

Solid-supports or surfaces comprising an array or plurality of sub-arrays of polynucleotides as described herein are particularly useful for capturing and/or analysing sample analytes in a spatial manner, for example the analytes of a tissue or other two- or three-dimensional sample laid over other otherwise contacted with the surface/support in a manner that captures the spatial arrangement of the analytes as captured.

*Kits*

In some case the invention relates to a kit. The kit may be for generating one or more libraries from one or more groups of analytes or from polynucleotides of mixed sequence from a sample. The kit may comprise any combination of the arrays of polynucleotides described herein, each array comprising or providing a pool (first and/or second) of identifier sequences (as described herein). Particularly envisaged are kits comprising an array of polynucleotides (capture polynucleotides, template switch oligonucleotides, annealed oligonucleotide strand pairs and single stranded oligonucleotides for producing annealed oligonucleotide strand pairs) as described herein, wherein each polynucleotide in the array comprises an identifier sequences that comprises or consists of a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two (or more typically at least three) nucleotide substitutions. The kit may comprise an array of capture polynucleotides, a micro-particle, a plurality of micro-particles, or a surface/solid support as described herein. The kit may further comprise an array of template switch oligonucleotides as described herein. A kit as described herein may be used for generating one or more libraries from one or more groups of analytes or from polynucleotides of mixed sequence from a sample. The kit may also comprise buffers, enzymes (for example a polymerase (such as those described herein) and/or a reverse transcriptase or template switch reverse transcriptase (such as Moloney Murine Leukemia Virus (MMLV) reverse transcriptase) and optionally suitable accompanying buffers) and other components used for generating a library as described herein and/or instructions for use of the kit in a method of generating the library.

*Analyte Capture, Libraries and Library Generation*

In some cases, the sample polynucleotides/analytes/RNA/mRNA/full length mRNA may be from a single cell, such as a bacterial cell, a mammalian cell or a human cell, or single cell nucleus, single cell vesicle, such as an exosome or mini-vesicle, or other compartment enclosed by a lipid membrane. In some cases, the method may comprise isolating the single cell, nucleus, vesicle or other compartment, or a lysate thereof, and contacting the isolated cell, nucleus, vesicle, compartment or lysate with a single micro-particle or array of polynucleotides as described herein.

In some cases, the polynucleotides/analytes may be from a sample comprising a plurality of cells (such as bacterial, prokaryotic or eukaryotic cells), cell nuclei, vesicles or other compartment enclosed by a lipid membrane, or a two- or three-dimensional sample such as a tissue sample. Analytes from different cells, cell nuclei, vesicles or other compartments or from different spatial positions in the sample may be captured by separate arrays of polynucleotides as described herein.

The method may in some cases include preparing a single cell or single cell nuclei or vesicle suspension from the sample and/or isolating single cells, nuclei, vesicles or lysates thereof in separate compartments. A single cell, nuclei, vesicle or cell/nuclei/vesicle lysate of the sample and a single micro-particle may be isolated in each of a plurality of separate compartments. In some cases, there may be at least 500, or at least 5,000, or 25,000, or 50,000, or 100,000, for example between 500 and 500,000, or between 2,000 and 200,000, or between 20,000 and 100,000 separate compartments, each comprising a single cell, nuclei or cell/nuclei lysate and a single micro-particle or (sub-)array of polynucleotides as described herein. The method may in in other cases include preparing a two- or three-dimensional sample for contact with a surface/solid support as described herein.

Micro-particles or (sub-)arrays of polynucleotides may be contacted with sample or analyte in a compartment, typically a fluidic compartment. For example, the compartment may be a well, such as a well in a multi-well plate or microplate or slide, a discrete site/position on a microfluidic chip, or a (micro-)droplet, which may be formed in an oil emulsion. Such compartments may in some cases be made using a microfluidics device as known in the art. In some cases, the sample, a micro-particle, an array of polynucleotides, a cell, cell nuclei or cell/nuclei lysate may be encapsulated or co-encapsulated in a fluidic compartment. In some cases, the cell and/or cell nucleus membrane(s) may be lysed, before or after contact with the micro-particle or array of polynucleotides.

In some cases, a linker of the polynucleotides may be cleaved or a micro-particle hydrogel may be dissolved to separate the polynucleotides from (the bead of) a micro-particle or from a surface/solid support before or after contact with the cell, nucleus or lysate, for example by exposing the micro-particle to UV light or heat, or contacting the micro-particle with appropriate chemicals or enzyme(s), such as the enzyme(s) described herein. In some cases, it may be convenient to include the micro-particle or array or polynucleotides in cell/membrane lysis buffer, and/or the cell, nucleus or lysate in a buffer comprising agents needed for cleavage of the linker, such that mixing the two buffers exposes cell/nucleus to lysis buffer and/or micro-particle to the chemical milieu for linker cleavage, as well as bringing the array of polynucleotides into contact with analyte from the cell or nuclei. For example, a microfluidics device may be used to join two aqueous flows into discrete microfluidic droplets. One flow may comprise a single cell or single cell nuclei suspension in cell buffer and optionally chemicals or enzymes needed to cleave the polynucleotide linker. The other flow may comprise a suspension of micro-particles as described herein, optionally in cell/membrane lysis buffer. Some of the droplets that are formed comprise both a cell or nuclei and a micro-particle, resulting in contact between the analytes and the array of polynucleotides.

Other components needed for downstream reactions and processes may be included in the cell/nucleus buffer and micro-particle/lysis buffer or be added to or used to wash the plate or other surface/solid support. In some cases, the cell/nucleus buffer may comprise template switch oligonucleotides. In some cases, the micro-particle/lysis buffer may comprise reverse transcriptase, in particular when the 3' analyte capture region of the polynucleotides is an RNA capture region, such as a polythymidine.

RNA in the sample may bind to the 3' RNA capture regions of the polynucleotides and reverse transcription using the bound RNA as template provides an RNA/cDNA hybrid at the 3' end of the polynucleotide. Template switch oligonucleotides may be added at the end of the RNA/cDNA hybrid. The template switch oligonucleotides and the PCR handle sequence 5' to the identifier sequence(s) (e.g. BC and/or UMI) provide a pair of PCR handle sequences for PCR amplification of the cDNA/RNA hybrid. PCR amplification may use a pair of oligonucleotide primers that hybridise to the template switch oligonucleotide sequence and the PCR handle sequence 5' to the identifier sequence(s).

DNA in the sample may bind to a 5' and/or 3' DNA capture region of the polynucleotides. DNA captured at the 5' end may be amplified using oligonucleotide primers that hybridise to a PCR handle sequence on the captured DNA and the complement of the PCR handle sequence 3' to the identifier sequence(s) (e.g. BC and/or UMI). DNA captured at the 3' end may be amplified using oligonucleotide primers that hybridise to a PCR handle sequence on the captured DNA and the PCR handle sequence 5' to the identifier sequence(s).

After amplification, the PCR products may be sequenced using methods known in the art and/or disclosed herein. Barcoding means that different compartments containing different polynucleotide arrays bound to analyte, or reaction products thereof, may be merged after capture. For example, the different compartments, such as droplets, may be merged after reverse transcription and/or before PCR. Downstream processes may then be carried out in bulk.

PCR product sequences having different barcodes can be digitally assigned to different samples or sample parts or sub-elements (for example cells or cell nuclei), as described herein. Analytes can be digitally counted by using the UMI sequences to identify duplicates derived from the same capture event.

The products and methods described herein may be used in a method of generating one or more libraries, and libraries may be produced by such methods. The methods described herein can be used to generate libraries corresponding to the sample polynucleotides, for example the analytes that bind to the 3' and/or 5' analyte capture regions of capture polynucleotides described herein. The libraries may be sequenced using methods known in the art and/or disclosed herein. The determined sequence of the identifier sequences added to each end of a sample polynucleotide sequence may be used to detect amplification chimeric artefacts as described herein.

The library or libraries may be made from the analytes of any suitable sample. Specific examples include a sample of cells, cell nuclei or cellular vesicles, a single cell, a single cell nucleus, a single vesicle, a tissue sample or tissue section, or a biological fluid sample, optionally blood, a blood fraction, serum, plasma, saliva or urine sample.

*Nucleotide Block Identifier Sequence Handling and Analysis*

The methods may use either traditional single nucleotide unit identifier sequences or identifier sequences that comprise a series of discrete nucleotide blocks as described herein. Using nucleotide block-based identifier sequences provide a number of advantages as described herein and discussed further in the Examples. In particular, they allow for improved detection and correction of sequencing and amplification errors, which is especially important when used for long-read or full-length single molecule sequencing, or other sequencing methods having relatively high base-calling error rates. The methods described herein may comprise obtaining sequencing data for each indexed polynucleotide, that is each sample polynucleotide having an identifier sequence added at both ends as described herein. Any suitable sequencing method known in the art and/or disclosed herein may be used, including long- and short-read sequencing methods. When a nucleotide block-type identifier sequence has been used, the percentage of the identifier sequences of the polynucleotides that are correctly sequenced as consisting only of one of the pre-defined nucleotide block sequences at each nucleotide block position may be estimated or determined. This can be done by comparing the obtained sequence in each polynucleotide at each nucleotide block position with the pre-defined pool of nucleotide block sequences at each respective position and determining the percentage of polynucleotides that are a complete match. This percentage is indicative of and may be used to determine the accuracy of the method of amplification and/or sequencing, and/or of the obtained polynucleotide sequences.

A nucleotide block-type identifier sequence may also be used to identify identifier sequences that have been sequenced or amplified incorrectly. A nucleotide block that has been sequenced or amplified incorrectly will have a nucleotide block sequence at the pre-determined position which does not match a nucleotide block sequence from the pre-defined pool of nucleotide block sequences relating to the nucleotide block position.

Sequenced polynucleotides having a "complete match" across the identifier sequence may be allocated into groups, wherein each group has the same identifier sequence, or in some cases the reverse complement of the identifier sequence. This process may be referred to as "deduplication", i.e. the elimination of reads that share the same complete UMI sequence across its entire identifier sequence, or collapsed identifier sequence. For example, where homodimer, homotrimer etc. nucleotide blocks are used, then the sequence may be collapsed or "demultimerised" to a single nucleotide unit sequence, e.g. AAA GGG CCC would be "demultimerised" to A G C. Sequenced polynucleotides allocated to the same group/having the same complete match identifier sequence are assumed to have all been sequenced from and/or amplified from polynucleotides of the array having the same identifier sequence. Where errors are detected in a sequence nucleotide block, i.e. based on non-complementarity with the known nucleotide block sequences in the pool (or the pool for the relevant position in the sequence), then the nucleotide block sequence can be corrected by assuming that the correct nucleotide block sequence is the one in the known pool that requires the fewest single nucleotide substitutions starting from the determined sequence containing the error, i.e. using a majority vote approach. For example, if the pool of nucleotide block sequences comprises only trimers, AAA, CCC, TTT and GGG, and an identifier sequence is sequences as comprising the sequence "AAA.**G**TT.GGG.CCC", then the second nucleotide block sequence "**G**TT" can be corrected to "TTT", because it requires few substitutions to correct than the next closest option which is "GGG". Similarly indels can be detected where they disrupt the expected pattern of known nucleotide block sequences in the pool. For example, a sequence of "AAA.**G.T**TT.GGG.CCC" can be corrected to "AAA.**T**TT.GGG.CCC".

Once the percentage of "complete match" sequenced identifier sequences has been determined, this value may further be used to improve allocation of the remaining sequenced polynucleotide (i.e. those known to comprise one or more amplification and/or sequencing error) to the groups described above. The percentage of "complete match" identifier sequences can be used to determine a cut-off for discarding from further analysis polynucleotide sequences comprising more than the determined cut-off number or percentage of nucleotide blocks in the sequenced identifier sequence that have not been correctly sequenced as having one of the pre-selected sequences. For example, in some cases a read may be removed from the analysis if it is deemed to have more than about 20% of errors in the identifier sequence, or more than about 1%, or 2%, or 5% or 10% or 12% or 15%, or 25% or 30% or 35% or 40% or 45% or 50% of errors. Knowing the percentage of "complete match" identifier sequences/the overall accuracy of the amplification/sequencing allows sequences to be allocated with greater confidence to the correct groups and allows more of the sequenced polynucleotides to be allocated to a group. This improved the efficiency of polynucleotide/analyte library generation, validation and analysis, allowing more data to be extracted from the same method of analyte capture and library generation. The remaining polynucleotide sequence that have not been discarded according to the determined cut-off may be further collapsed into the groups described above according to the best match of their sequenced identifier sequence, as described above. Sequenced polynucleotides allocated to the same group by this method are assumed to have all be sequenced from and/or amplified from the same sample polynucleotides having the same identifier sequence, despite errors in the sequence known to have been introduced by the method of amplification and/or sequencing. In other words, variation in the sequence of the polynucleotides that are grouped according to this method is assumed to be the result of replication, amplification and/or sequencing errors.

The percentage of "complete match" sequences may also be used in some cases to determine a further (second) cut-off for assigning/allocating any polynucleotide sequences not comprising a "complete match" (and optionally not discarded according to the first cut-off described above) and having more than the determined second cut-off number of nucleotide blocks in the sequenced identifier sequence that are not correctly sequenced as having one of the pre-selected nucleotide blocks sequences into different groups instead of the same group. This is particularly useful when the total number of groups and/or initial polynucleotides in the library of polynucleotides is unknown, which is often the case for UMI sequences resulting from unique analyte capture events, but may be the result of a number of different factors. The number of polynucleotides on a single micro-particle, single cell or well of a plate, or at a single pre-determined position on a surface is difficult to determine, although it may be estimated. Moreover, the number of polynucleotides that capture an analyte may only be a small proportion of the total number of polynucleotides sample.

The sequenced polynucleotides, or the remaining sequenced polynucleotides, can then be put into groups based on sequence identity across the identifier sequences and using the first and/or second cut-off. Sequenced polynucleotides allocated to the same group by this method are assumed to have all been sequenced from and/or amplified from polynucleotides of the array having the same identifier sequence, despite errors in the sequence known to have been introduced by the method of amplification and/or sequencing.

## Examples

*Example 1 - Establishing a simplified workflow for introducing dual homotrimeric-UMIs into RNA for long-read amplicon sequencing.*

An improved molecule counting approach designed specifically to improve long-read transcriptome sequencing was applied. During reverse transcription, each RNA molecule is tagged with a homotrimeric UMI and primer sequence at both the 3' and the 5' terminal ends. The cDNA is then amplified using at least 25 cycles of PCR to generate enough input material for long-read ONT or PacBio library preparation and sequencing (Fig. 1a-d). After sequencing, UMIs are then detected based on pattern recognition of the primer sequences, as well as the 30bp UMI length (Fig. 1e). Sequence errors across the full UMI sequence may be detected based upon discordant trimer complementarity and then corrected and collapsed into single nucleotide UMI sequence by selecting the most common nucleotide within the trimer (Fig. 1f). Simulations show that implementing our homotrimeric correction approach improves the efficiency of error detection, allowing complete recovery of all simulated UMIs (Fig 1h). To improve error correction further, mclUMI was developed, an approach which implements the Markov Cluster Algorithm to deduplicate UMIs while eliminating errors. Simulated data show that mclUMI outperforms UMI-tools (Smith, T. et al. UMI-tools: modeling sequencing errors in Unique Molecular Identifiers to improve quantification accuracy. Genome Res 27, 491-499 (2017)) for longer UMI lengths and provides a modest improvement in deduplicating UMI sequences when sequencing error rates are comparable to Illumina sequencing. (Fig. 1i). However, unlike other alternative UMI deduplication approaches, mclUMI uses both terminal UMIs for removing chimeric artefacts (Smith, T. et al. I. UMI-tools: modeling sequencing errors in Unique Molecular Identifiers to improve quantification accuracy. Genome Res 27, 491-499 (2017); Vander Heiden, J.A. et al. pRESTO: a toolkit for processing high-throughput sequencing raw reads of lymphocyte receptor repertoires. Bioinformatics 30, 1930-1932 (2014)). Simulations show that mclUMI removes the presence of most artefactual chimeric reads in simulated data (Fig 1j).

*Example 2 - Benchmarking transcriptome sequencing using homodimer UMI sequences*

To assess the effectiveness of the homotrimeric UMI error correction approach, a constant 30 mer homotrimeric Common Molecular Identifier (CMI) oligonucleotide sequence was synthesised and attached to equimolar concentrations of mouse and human (50:50) RNA at the 3' terminal end. The resulting library was then split and sequenced using Illumina, nanopore or PacBio approaches. The Q score for both Illumina and PacBio sequencing was significantly higher for than for ONT sequencing (Fig. 2a). The accuracy of sequencing was measured by calculating the hamming distance between the observed sequence versus the expected oligonucleotide sequence. 68.08% of CMIs sequenced using the Illumina platform were error free, and the homotrimer correction approach increased the CMI recovery rate to 98.04%. The same library was sequenced using PacBio SMRT2 sequencing and show that 73.36% of HiFi CMIs were error free, with the homotrimer correction approach able to recover 99.64% of total reads (Fig. 2b). The same library was sequenced using Oxford Nanopore Technologies sequencing using LSK-109 and LSK-112 (Q20+) chemistries. Libraries prepared using the LSK-109 chemistry and sequenced using R9.4.1 or R10.3 flow cells led to 18.36% and 33.14% error free UMI reads, respectively. The majority of these CMI sequences were able to be error corrected, with 5-15% of errors remaining. However, libraries prepared using LSK-112 chemistry led to substantial increase in error free CMIs, with 37.28% error free following sequencing using R9.4.1 flow cells and 60.98% error free using R10.4. The majority of the CMI sequences were able to be error corrected, with only 3-7% of errors remaining. Despite the improvement in CMI recovery using the R10.4 flow cells, significantly fewer reads sequenced per flow cell were observed. Overall, the basecalling accuracies were calculated to be 98.7% for Illumina, 98.9% for PacBio, 96.5% for ONT LSK112-R9.4.1 and 98.4% for ONT LSK112-R10.4.

Trimers afford the ability to measure the frequency of errors within each molecular identifier, allowing thresholds to be set upon which to remove reads that have an excess of errors. The simulations (Fig. 1i) suggested that removing reads where there was the presence of more than 10% errors within each UMI sequence could improve error correction further. Several filtering thresholds were applied (Fig. 2c) that show that filtration of any CMI that contained at least one homotrimer error resulted in 100% of the CMIs being correctly identified. However, a significant fall in the proportion of CMIs recovered following Illumina sequencing was observed but not with PacBio or Nanopore sequencing when a filtering error threshold of 50% was applied. When the frequency of errors before correction was observed, increased frequency of homotrimer errors greater than 5 in the Illumina sequence data but not in the PacBio or ONT sequenced CMIs were seen (Fig. 2d).

Despite the simulations showing that further accuracy could be achieved by applying mclUMI or UMI-tools (Smith, T. et al. UMI-tools: modeling sequencing errors in Unique Molecular Identifiers to improve quantification accuracy. Genome Res 27, 491-499 (2017)) deduplication correction (Fig. 1i), experimentally UMI-tools and mclUMI performed less well against the homotrimer deduplication approach (Fig. 2e). As fusion transcripts can be falsely generated because of PCR artefacts (Cocquet, J. et al. Reverse transcriptase template switching and false alternative transcripts. Genomics 88, 127-131 (2006)), for long-read sequencing data the presence of mouse-human chimeric reads was used as an indication of false positive fusion events. Since mouse-human fusions would never occur naturally, the mcUMI chimeric artefact removal was evaluated using this data. ~2% of chimeric human-mouse reads are detected in the sequencing library, with the majority of chimeric reads being removed follow paired UMI filtering (Fig. 2f). Collectively, homotrimer sequences are demonstrated to allow for the measurement of sequencing errors in UMI based counting and pairs of homotrimer UMIs can be used to remove PCR artefacts that can be problematic when performing translocation analysis.

The homotrimer method has advantages over the current computational approaches for correcting errors within UMI sequences because errors can be precisely pinpointed and then either the error may be corrected based on trimer complementarity or the read be removed from the analysis if it is deemed to have more than 20% of errors within the sequence. The UMI correction approach leads to substantially improved accuracy during differential expression analysis when compared to demultiplexing UMIs using computational approaches.

*Example 3 - The inclusion of UMIs for Long-read sequencing are necessary for accurate absolute quantification*

The application of long-read sequencing of mRNA is an important emerging method for studying isoform expression, variant calling and translocation analysis. Nanopore sequencing was applied to homotrimer UMI tagged cDNA generated from treating Ewing's RM82 sarcoma cells with a splicing kinase CLK1 inhibitor and DMSO control. The correlation between the gene and transcript expression was assessed when our homotrimer UMI correction method was performed versus read counting. Good correlation was found between homotrimer UMI correction and read counting for both genes and transcripts (Fig. 3, left two panels). 192 differentially expressed genes were identified between DMSO and CLK1 following read counting, while 177 genes were differentially regulated when UMIs were corrected using the homotrimer method. Differential transcript expression analysis identified 563 transcripts as being differentially expressed following a read counting approach, while 501 transcripts were differentially expressed when homotrimer UMI correction was applied. Illumina sequencing data identified 84 genes that were differentially expressed genes for read counting approach and 80 genes differentially expressed when comparing to homotrimer UMI correction.

Next, each trimer across the full 30bp UMI was collapsed into a monomer by selecting the first base of the timer to generate a 10 base UMI. Both UMI-tools and mclUMI were then applied to demultiplex the counts and this was compared to the homotrimer UMI correction approach. A good correlation was shown between genes (Fig. 3, third panel from left), but a less well-correlated transcript expression (Fig. 3, further panel from left). 192 genes differentially expressed between DMSO and CLK1 inhibitor treatment were identified following UMI-tools following mclUMI tools demultiplexing. Differential transcript expression analysis identified 626 differentially regulated transcripts following UMI-tools demultiplexing following mclUMI demultiplexing. Illumina sequencing data identified only 73 differentially expressed genes following UMI tools demultiplexing. Overall, homotrimer correction was shown to reduce the number of potential false positive results in bulk RNA sequencing and demonstrates that the inclusion of homotrimer UMIs is critical for performing accurate differential transcript analysis.

*Discussion*

Here is described a highly accurate UMI-based error correction approach for both short and long-read RNA sequencing that does not require the explicit need for complex computational demultiplexing. The method described has advantages over the current computational approaches for correcting errors within UMI sequences because errors can be precisely pinpointed. Then the error can either be corrected, based on the complementarity to the known identifier sequence nucleotide block sequences in the pool (trimer nucleotide blocks in these Examples) or the read can be removed from the analysis if it is deemed to have too high a proportion of errors within the sequence. This UMI correction approach leads to substantially improved accuracy during differential expression analysis when compared to demultiplexing UMIs using computational approaches.

Typically, UMI correction is performed using either network based, clustering based or UMI and read edit distance approaches. These methods were originally developed for high accuracy short-read Illumina sequencing data and have a low tolerance to high sequencing error rates, such as those observed in long-read sequencing. However, it is difficult to ascertain the real-world accuracies of these methods because of a lack of ground truth data, with all methods relying on simulated data as a validation of their accuracy. To experimentally validate the homotrimer UMI correction approach, the concept of the common molecular identifier (CMI) was introduced, a known nucleotide sequence that is added as a substitute for a UMI. This approach was used to experimentally confirm that synthesising homotrimer UMIs can significantly overcome the error rate of Illumina, ONT and PacBio sequencing and recover up to 99.6% of CMI tagged reads or remove 100% of the error containing UMI tagged reads. Surprisingly, despite the high basecalling accuracy of Illumina, many CMIs containing greater than 5 homotrimer errors were observed. This lower basecalling accuracy of Illumina may be explained, in part, by the first 10 bases being used to calibrate the clustering assignment for NextSeq 500 instruments. However, the Illumina sequencer also has several known biases which include phasing (lagging due to a failure to incorporate a base) and prephasing (two base incorporations during a cycle), signal decay and crosstalk. Measured basecalling accuracy of PacBio was higher than Illumina, while ONT was lower but increased substantially using the new LSK-112 (known also as Q20+ chemistry) and R10.4 flow cells.

Despite improvements in the ability to detect and then correct errors within homotrimer UMIs, chimeric artefacts are generated during PCR amplification. These errors especially complicate the analysis of oncogenic translocations. The presence of two UMIs attached at each end of the cDNA can be used to perform paired UMI artefact removal, such as by using mclUMI. mclUMI utilises a Markov Cluster Algorithm for UMI deduplication and was developed specifically for longer UMI lengths, which are typically used for homotrimer UMI synthesis. Furthermore, the method is also able to remove PCR chimeric artefacts in long-read sequencing data where paired UMIs are included at both ends of the cDNA. The method showed similar deduplication accuracies when evaluated against the known software UMI-tools. When both mclUMI and UMI-tools were compared against homotrimer UMI correction a good correlation with gene level analysis was observed, but this was less well correlated at the transcript level. This suggests that graph network approaches to correct and demultiplex UMIs on their own are less reliable than demultiplexing using homotrimer UMIs. Despite this, the chimeric PCR removal method was surprisingly able to remove most PCR artefacts.

The choice of sequencing platform for RNA sequencing depends on several considerations and technological limitations. Short-read Illumina sequencing, while cost effective for gene level analyses, the short nature of the read length makes transcript resolution complex. Long-read sequencing platforms are more suited to transcript and translocation analyses at both the bulk and single-cell level. While the PacBio systems has superior error correction, it can only generate a maximum of 8 million reads and has a higher per base sequencing costs. This is contrasted with ONT sequencing, which can theoretically return up to 250 million reads using the PromethION™ device, albeit with a lower basecalling accuracy.

For sensitive applications where UMI counting is considered critical then PacBio sequencing seems best suited, but for the most applications where a high read depth is required then ONT sequencing seems to be more suitable.

*Example 5*

The inclusion of Unique Molecular Identifiers (UMI) in sequencing experiments creates a distinct identity for each input molecule, making it possible to accurately correct sampling and PCR amplification bias. The use of UMI sequences pre-dates sequencing, however including UMIs prior to library construction can improve the accuracy across almost all next-generation and third generation sequencing methods, including bulk RNA, single-cell RNA and genomic DNA approaches. However, the accuracy of molecular quantification can be impacted by the varying sequencing quality of different platforms. Moreover, different sequencing platforms require distinct PCR cycling conditions to generate adequate input material for sequencing, which can introduce UMI errors and lead to inaccurate molecule counts (Fig. 4). Unlike sample barcodes for multiplexing or cell barcodes in single-cell sequencing, which can be whitelisted due to a limited pool of barcodes, UMIs cannot be corrected using this approach as their synthesis is random. Therefore, UMIs are typically corrected using computational approaches, concatemeric consensus sequencing, or by bespoke UMI designs to aid error correction. Several computational approaches that leverage either hamming distances, graph networks, thresholding on UMI frequency have previously been used to overcome PCR or sequencing errors within UMIs. However, none of these solutions have been experimentally validated and simulations suggest that UMIs error persist following computational demultiplexing (Fig. 5).

The inventors reasoned that utilising homotrimer nucleosides to synthesise UMIs would simplify error detection and correction by using a "majority vote" method (Fig. 6). To enable more accurate error identification and better tolerance to indels, a strategy was developed that involves labelling each RNA molecule with an oligonucleotide containing a homotrimeric UMI located at the 5' and/or the 3' end, followed by sequencing using Oxford Nanopore Technologies (ONT), PacBio or Illumina (Fig. 1a-d). UMIs are identified and errors are detected across the entire UMI sequence by comparing trimer complementarity. To correct these errors, a "majority vote" method was used where the most common nucleotide within the trimer is selected (Figs. 1f and 6b). These simulations demonstrate that using trimers alone significantly improves the accuracy compared to relying solely on computational demultiplexing methods (Fig. 7). To further improve the homotrimeric majority vote error removal, a combinatorial optimisation approach was utilised (Fig. 8). To accurately identify translocated reads and distinguish them from chimeric artefacts, dual UMIs attached to cDNA ends were used. This method aids in computationally removing chimeric artefacts (Figs. 1g and 9). By implementing the homotrimeric correction approach, the error detection was improved and simulated UMIs that match the ground truth were recovered (Figs 1h and 10).

While sequencing simulations can offer valuable insights, their real-world applicability may be limited by biases. To validate the homotrimer UMI error correction approach, experiments were conducted using a Common Molecular Identifier (CMI) attached to every captured RNA molecule (Fig. 11). Having the same molecule attached to every RNA guarantees that, in the absence of errors, each transcript is only counted once. However, if errors are introduced into the CMI, transcripts will be overcounted. This provides a means for assessing the accuracy of library preparation and sequencing, as well as the impact of errors on the transcript counts (Fig. 11b).

The CMI was attached to equimolar concentrations of mouse and human cDNA at the 3' end, PCR amplified and the sample was split for sequencing on Illumina, PacBio or ONT platforms. The hamming distance was calculated between the observed and expected CMI sequence to measure sequencing accuracy. The results show that 73.36%, 68.08%, and 89.95% of CMIs were accurately called using Illumina, PacBio, and the latest ONT chemistry, respectively (Figs. 14a, 12 and 13). Older ONT chemistry gave substantially lower accuracy (Fig. 15), but the use of super accuracy basecalling led to substantial improvements (Fig. 16). Using the homotrimeric error correction approach, 98.45%, 99.64% and 99.03% of CMIs for Illumina, PacBio and the latest ONT chemistry could be corrected, respectively (Fig. 14a). The inventors hypothesised that the lower accuracy of Illumina and PacBio when compared to ONT sequencing may be due to higher number of PCR amplification cycles during sequencing (e.g. bridge amplification and rolling circle amplification with Illumina and PacBio, respectively). To decompose the effect of sequencing and PCR errors, a CMI-tagged cDNA library was subjected to increasing cycles of PCR and then sequenced using ONT's minion platform. To minimise batch effects across the data and simultaneously evaluate sequencing accuracy independent of PCR amplification effects, trimer sample barcodes were attached during PCR amplification. A high degree of barcode accuracy was shown, and it was observed that homotrimer correction had minimal effect in improving this accuracy. Based on these results, it can be inferred that sequencing errors make a negligible contribution to the overall error rate (Fig. 14b). Nevertheless, a substantial increasing in the number of errors was observed within the CMIs with increasing PCR cycles. The homotrimer approach was able to correct the majority of errors observed within the CMIs (Fig 17). This suggests that PCR is a significant source of errors. Next, homotrimer error correction was benchmarked against UMI-tools and substantial improvements in error correction were found (Fig. 18a). Furthermore, most human-mouse chimeric artefacts could be removed from the library, experimentally validating the dual UMI tagging approach (Fig. 18b).

Having demonstrated the ability to accurately correct PCR errors using homotrimers, an experiment was conducted in which Ewing's RM82 sarcoma cells were treated with a splicing kinase CLK1 inhibitor. This was done to induce splicing perturbations and observe an exaggerated isoform switching effect, which as then sequenced using either ONT's promthION or Illumina (Figs. 3, and 19 to 21). When UMI-tools was compared to the homotrimer correction methodology, 7.8% and 11% of discordant differentially expressed genes and transcripts was compared, respectively for ONT (Fig. 3). 4.7% discordant differentially expressed genes were also observed following Illumina sequencing (Fig. 21c-d). It was shown that homotrimer error correction corrects PCR amplification errors that lead to increased UMI variability between samples on a per gene level (Fig. 19). In addition, the homotrimer correction approach led to an increased fold enrichment of genes associated with gene ontology terms related to DNA replication and splicing (Fig. 22), highlighting the improved accuracy of this method in identifying biologically relevant gene sets.

To understand how PCR errors contribute to single-cell sequencing errors, JJN3 human and 5TGM1 mouse cells were encapsulated using the 10X Chromium system and reverse transcription was performed followed by 10 PCR cycles. Subsequently, the PCR product was divided into two portions and additional PCR amplification was performed, resulting in a combined number of PCR cycles of 30 and 35. These libraries were then prepared and sequenced using the ONT's PromethION platform, which does not require PCR amplification as part of the sequencing process. After assigning cell barcodes (Fig. 23) and filtering, clustering and annotating the cells (Fig. 24a), it was observed that the library subjected to 35 cycles of PCR had significantly greater number of UMIs compared to the library that underwent 30 PCR cycles (Figs. 24b and 25). This suggests that PCR errors contribute to inaccurate counting of transcripts and an inflated UMI count. Next, differential gene expression was performed between 30 and 35 PCR cycles in both the mouse and human cells and 50 differentially expressed transcripts were identified, of which ENSMUST0000034966 (Fig. 26a) and ENST00000532223 (Fig. 26b) were the highly significant.

Next, JJN3 human and 5TGM1 mouse cells we encapsulated and reverse transcription and template switching that included a CMI were performed, followed by 10 PCR cycles. Subsequently, the PCR product was divided into four portions and additional PCR amplification was performed so that each library was subjected to 20, 25, 30 and 35 PCR cycles. The libraries were then sequenced using the ONT minion platform. The results indicate a decrease in the percentage of reads with accurate CMIs as the number of PCR cycles increases. Nevertheless, homotrimer correction led to 96-100% correction of CMI sequences (Figs. 27a and 28). This underscores the effectiveness of this approach in removing errors introduced by PCR. Subsequently, the libraries that underwent 30 and 35 PCR cycles were sequenced using ONTs PromethION platform (Fig. 29). The results show that the incorporating homotrimers within the barcode region resulted in a slight increase in the numbers of cells recovered (Fig. 27b). Monomer UMIs resulted in over 300 differentially regulated transcripts between the 30 and 35 cycle libraries. However, homotrimer correction did not reveal any significant differentially regulated transcripts (Fig. 30), demonstrating the robustness of homotrimer UMIs to remove errors.

The results of this study underscore the crucial role of precise UMI counting in both bulk and single-cell sequencing, and demonstrates that homotrimer UMIs are a reliable solution to enhance the accuracy of absolute read counting. PCR amplification errors are the main source of UMI inaccuracy, but homotrimer UMIs can improve error correction and achieve near perfect sequencing of molecule counts.

*Example 6 - Materials and Methods*

Cell lines **and reagents**

JJN3, 5TGM1 and RM82 were cultured in complete RPMI medium. All parental cell lines were tested twice per year for mycoplasma contamination and authenticated by STR during this project. For cell culture experiments, SGC-CLK-1 (Structural Genomics Consortium) inhibitor was incubated with cells for 24 hours. DMSO was used as a negative control.

**Oligonucleotide synthesis**

Homotrimer phosphoramidites were purchased as a custom product from Metkinen Chemistry (Finland).

scCOLOR-seq beads were synthesised as described previously (Philpott, M. et al. Nanopore sequencing of single-cell transcriptomes with scCOLOR-seq. Nat Biotechnol 39, 1517-1520 (2021)).

Primers containing Common Molecular Identifiers (CMI) were synthesised by Sigma Aldrich (Burlington, USA) using the following sequences polyA oligonucleotide: 5' - AAGCAGTGGTATCAACGCAGAGTACNNNNNNNNNNTTT TTTTTTTTTTTTTTTTTTTT TTTTTTT-3 Template switch oligonucleotide: 5' - AAGCAGTGGTATCAACGCAGAGTNNNNNNNNNNGAATrGrGrG-3'. The oligonucleotides were PAGE purified and shipped lyophilized.

**Simulated UMI data**

UMI data of length 30 (10 blocks of nucleotide trimers) was simulated to confirm the accuracy of our UMI correction methodology. The PCR amplification and sequencing errors seen with ONT sequencing were mimicked, as this sequencing methodology suffers from indels and basecalling errors more frequently than PacBio or Illumina sequencing. UMIs were generated following an approach that was first described by UMI-tools. Briefly, homotrimer nucleotide blocks of UMIs were simulated randomly, with a uniform amplification (0.8-1.0) and then PCR cycles were simulated so that each UMI was duplicated to the probability of amplification. PCR errors were then randomly added and assigned new probabilities of amplification. A defined number of UMIs were randomly sampled to simulate sequencing depth and sequencing errors introduced with a specified probability. Finally, errors were detected by assessing the complementarity of homotrimers across the full UMI sequence. If no errors were detected, then the homotrimers were collapsed into single nucleotide bases. However, if errors were identified then collapsing into single nucleotides was performed using the most common nucleotide within the trimer. If a most common nucleotide could not be determined, then a single nucleotide was selected at random for collapsing. The following values were used as values within our simulations. Sequencing depth 10-400; number of UMIs 10-100; UMI-length 6 - 16; PCR error rate $1\times10^{-3}$ - $1\times10^{-5}$; sequencing error rate $1\times10^{-1}$- $1\times10^{-7}$ and number of PCR cycles 4-12.

**Removal of errors within the UMI**

UMI errors were detected by identifying trimer complementarity across the full length of the UMI sequence. First, indels were removed by tiling a 3 base sliding window across the UMI and looking for potential one base insertions and then removing them. Next, point mutational were removed by choosing the most common base within the homotrimers across the full length of the sequence. At this stage, homotrimer correction approach was resistant to one base pair insertions or deletions so insertions or deletions are automatically corrected. Unless otherwise stated, reads were removed where greater than or equal to three errors were detected in any UMI.

**Simulating chimeric artefacts in UMI sequences**

Reads mixed with chimeric artefacts were simulated using the *resimpy_umi transloc* module in Resimpy. Briefly, a total of 50 reads were generated, each attached with a UMI included at the 3' end and a UMI at the 5' end. Each randomly simulated UMI of homotrimer nucleotide blocks was 30bp in length. UMIs with at least a 3-base edit distance away from one another were simulated. The chimeric frequency was set to 2% of the total read count. The number of wrongly synthesized bases due to DNA polymerase errors and sequencing errors were estimated from our bulk data using negative binomial models. All other parameters for simulation were kept the same as the simulation of UMIs as above. To detect chimeric artefacts, two methods were proposed, umiRarity and umiRarityCR, which both merge UMIs at 3' and 5' ends at first and then leverage the rarity of the merged UMIs in terms of their unique counts specific to reads that it is mostly likely impossible to synthesize during PCR amplification. umiRarity and umiRarityCR are both threshold-dependent methods by setting a frequency of UMI-UMI pairs, below which reads were considered chimeric and above which non-chimeric otherwise. Frequency thresholds ranging from 1-5 were explored so as to understand their influence on the detection of chimeric artefacts. The performance of chimeric artefact removal was evaluated by comparisons between the percentage of successfully removed chimeric artefacts and the percentage of sacrificed real reads, and between the percentage of successfully removed chimeric artefacts and the percentage of successfully detected PCR duplicates. For comparison purposes, UMIs at the single end (3') are taken as the control group to be compared with umiRarity and umiRarityCR.

**Generating homotrimer UMI tagged cDNA**

Total mRNA was isolated using a Quick-RNA MiniPrep kit (Zymo), following the manufacturers protocol. The RNA sample quality and quantity was measured using an RNA screen tape on the TapeStation (Agilent). cDNA synthesis was performed with modification to the SMART approach (Zhu, Y.Y. et al. Reverse transcriptase template switching: a SMART approach for full-length cDNA library construction. BioTechniques 30, 892-897 (2001)). An oligo(dT) containing adaptor containing a homotrimer 30-base DNA sequence and a SMART primer sequence was used to initiate a reverse transcriptase reaction. Briefly, RNA was denatured at 72°C for 2 minutes and then reverse transcribed with Maxima H minus reverse transcriptase (2000 U) in a total volume of 50uL with the buffer, 1mM dNTPs, 2mM dithiothreitol (DTT) and 4% Ficoll PM-400. The reaction was performed for 90 minutes at 42 °C and then the enzyme was heat inactivated at 80 °C for 5 minutes. The library was then purified using 0.8X SPRI bead (Beckman Coulter) clean-up followed by PCR using the SMART PCR primer (AAGCAGTGGTATCAACGCAGAGT) before being purified using SPRI beads. To achieve a high concentration of cDNA the input was subjected to 25 cycles (Examples 1 to 4) or 30 cycles (Example 6) of PCR amplification followed by a second clean up. Then PCR was repeated for a further 12 cycles of PCR (Examples 1-4) or optionally, 10ng of PCR product was subjected to 12 further cycles of PCR using primers that contained trimer sample barcodes (Supplementary Table 1). Further 3-5 PCR cycles were performed using a biotin oligonucleotide (5-PCBio-AAGCAGTGGTATCAACGCAGAGT) and then cDNA was enriched using DynabeadsTM MyOneTM streptavidin T1 magnetic beads (Invitrogen). The beads were washed in 2X binding buffer (10mM Tric-HCL ph7.5, 1mM EDTA and 2M NaCl) then samples were added to an equi-volume amount of 2X binding buffer and incubated at room temperature for 10 mins. Beads were placed in a magnetic rack and then washed with twice with 1X binding buffer. The beads were resuspended in H2O and incubated at room temperature and subjected to long-wave UV light (~366 nm) for 10 minutes. Magnetic beads were removed, and library was quantified using the QubitTM High sensitivity kit. Libraries were then prepared before sequencing.

| Barcode | Sequence |
|---|---|
| 1 | **AAATTTGGGCCC**AAGCTGTGGTATCAACGCAGAGT |
| 2 | **TTTCCCAAAGGG**AAGCTGTGGTATCAACGCAGAGT |
| 3 | **GGGAAACCCTTT**AAGCTGTGGTATCAACGCAGAGT |
| 4 | **CCCGGGTTTAAA**AAGCTGTGGTATCAACGCAGAGT |

**Oxford Nanopore Technologies (ONT) bulk RNA seq library preparation and sequencing**

A total of 1,200ng of purified cDNA was used as a template for ONT library preparation. Both the SQK-LSK-109 and SQK-LSK112 (also referred to as Q20+ chemistry) Ligation sequencing kits were used, following the manufacturers protocol. Samples were sequenced using a minION™ device using R9.4.1 (FLO-MIN106D), R10.3 (FLO-MIN111) and R10.4 (FLO-MIN112) flow cells. Barcoding using the Native Barcoding Amplicon kit (EXP-NDB104) was performed for RM82 cells treated with DMSO or CLK1 inhibitor treatment. Unlike the other libraries, these samples were sequencing using the PromethION™ sequencing platform using R9.4.1 FLO-PRO002 flow cells at the Deep Seq facility at the University of Nottingham and CD Genomics.

**PacBio bulk RNA seq library preparation and sequencing**

A total of 1,200ng of purified cDNA was used as a template for PacBio library preparation and sequencing at the Centre for Genomic Research at the University of Liverpool. cDNA was end repair and A tailed with T4 polynucleotide kinase (New England Biolabs). The sequencing library was prepared using SMRTbell Express Template Prep Kit 2.0 following the standard protocol. Sequencing was then performed on a sequel II using a Sequel II SMRT Cell 8M ion CCS mode, following the standard protocol. CCS reads were generated using CCS v6.3.0 (https://github.com/PacificBiosciences/ccs) using default settings.

**Illumina bulk RNA seq library preparation and sequencing**

Purified cDNA was used as an input for the Nextera XT DNA library preparation kit (New England Biolabs). Library quality and size was determined using a TapeStation (Agilent Technologies) High Sensitivity D1000 tape and then sequenced on a NextSeq 500 sequencer (Illumina) using a 75-cycle High Output kit using a custom read1 primer (G CCTGTCCGCGGAAGCAGTGGTATCAACGCAGAGTAC). Read1 length was 30bp and read2 length was 52bp long.

**Dropseq and 10X Chomium ONT sequencing**

A total of 1,000ng of purified cDNA was used as a template for ONT library preparation. Unless otherwise stated within the text, library preparation was performed using the SQK-LSK114 (kit V14) ligation sequencing kit, following the manufacturers protocol. Samples were then sequenced on either a minION™ device using an R9.4.1 (FLO-MIN106D) or a promethION™ device using R10.4 (FLO-PRO114M) flow cells.

**10X Chromium library preparation**

We prepared a single-cell suspension using JJN3 and 5TGM1 cells using the standard protocol. Briefly, cells were filtered into a single-cell suspension using a 40 μM Flomi cell strainer before being counted. We performed 10X Chromium library preparation following the manufacturers protocol. Briefly, we loaded 3,300 JJN3:5TGM1 cells at a 50:50 split into a single channel of the 10X Chromium instrument. Cells were barcoded and reverse transcribed into cDNA using the Chromium Single Cell 3' library kit and get bead v3.1. We performed 10 cycles of PCR amplification before cleaning up the library using 0.6X SPRI Select beads. The library was split and a further 20 or 25 PCR cycles were performed using a biotin oligonucleotide (5-PCBio-CTACACGACGCTCTTCCGATCT) and then cDNA was enriched using DynabeadsTM MyOneTM streptavidin T1 magnetic beads (Invitrogen). The beads were washed in 2X binding buffer (10mM Tric-HCL ph7.5, 1mM EDTA and 2M NaCl) then samples were added to an equi-volume amount of 2X binding buffer and incubated at room temperature for 10 mins. Beads were placed in a magnetic rack and then washed with twice with 1X binding buffer. The beads were resuspended in H2O and incubated at room temperature and subjected to long-wave UV light (~366 nm) for 10 minutes. Magnetic beads were removed, and library was quantified using the QubitTM High sensitivity kit. Libraries were then prepared before sequencing.

**Dropseq library preparation**

Single-cell capture and reverse transcription were performed as previously described (Macosko, E.Z. et al. Highly Parallel Genome-wide Expression Profiling of Individual Cells Using Nanoliter Droplets. Cell 161, 1202-1214 (2015)). Briefly, JJN3 and 5TGM1 cells (20:80 ratio) were filtered into a single-cell suspension using a 40 μM Flomi cell strainer before being counted. Cells were loaded into the DolomiteBio Nadia Innovate system at a concentration of 310 cells per μL. Custom synthesised beads were loaded into the microfluidic cartridge at a concentration of 620,000 beads per mL. Cell capture was then performed using the standard Nadia Innovate protocol according to manufacturer's instructions. The droplet emulsion was then incubated for 10 mins before being disrupted with 1H, 1H,2H,2H-perfluoro-1-octanol (Sigma) and beads were released into aqueous solution. After several washes, the beads were subjected to reverse transcription. Prior to PCR amplification, beads were treated with ExoI exonuclease for 45 min. PCR amplification was then performed using the SMART PCR primer (AAGCAGTGGTATCAACGCAGAGT) and cDNA was subsequently purified using AMPure beads (Beckman Coulter). The library was split and a further 20 or 25 PCR cycles were performed using a biotin oligonucleotide (5-PCBio-AAGCAGTGGTATCAACGCAGAGT) and then cDNA was enriched using DynabeadsTM MyOneTM streptavidin T1 magnetic beads (Invitrogen). The beads were washed in 2X binding buffer (10mM Tric-HCL ph7.5, 1mM EDTA and 2M NaCl) then samples were added to an equi-volume amount of 2X binding buffer and incubated at room temperature for 10 mins. Beads were placed in a magnetic rack and then washed with twice with 1X binding buffer. The beads were resuspended in H2O and incubated at room temperature and subjected to long-wave UV light (~366 nm) for 10 minutes. Magnetic beads were removed, and library was quantified using the QubitTM High sensitivity kit. Libraries were then prepared for sequencing.

**scCOLOR-seq library preparation and Dropseq and 10X Chomium ONT sequencing**

A total of 1,200ng of purified cDNA was used as a template for ONT library preparation. Unless otherwise stated within the text, library preparation was performed using the SQK-LSK112 or the SQK-LSK114 (kit V14) Ligation sequencing kit, following the manufacturers protocol. Samples were then sequenced on either a minION™ device using an R9.4.1 (FLO-MIN106D) or a promethION™ device using R10.4 (FLO-PRO114M) flow cell.

**Basecalling ONT data**

ONT Basecalling was performed on the raw fast5 data to generate fastq files using Guppy (v6.0.1+652ffd179) (guppy _basecaller -compress-fastq -c [configuration _file]-x "cuda:1") in GPU mode from ONT running on a GeForce RTX 3090 graphics card. Depending upon the flow cell and chemistry used, basecalling was performed using either the dna _r9.4.1_450bps_hac.cfg or dna_r10_450bps_hac.cfg configuration files. Where specifically specified, the high accuracy dna_r10_450bps_hac.cfg model was used.

**ONT and PacBio bulk RNA sequencing workflow**

The data was processed using a custom pipeline *'pipeline_counting'* written using cgatcore and included within the Bulk-tallyNNN repository (Cribbs, A. et al. CGAT-core: a python framework for building scalable, reproducible computational biology workflows [version 1; peer review: 1 approved, 1 approved with reservations]. F1000Research 8 (2019)). Briefly, the quality of each fastq file was evaluated using fastqc toolkit and summary statistics were collated using Multiqc (Andrews, S. FastQC: a quality control tool for high throughput sequence data. (2010); Ewels, P. et al. MultiQC: summarize analysis results for multiple tools and samples in a single report. Bioinformatics 32, 3047-3048 (2016)). The polyA associated UMI sequence was identified by searching for the poly A region and reverse complementing the read if it does not appear in the correct orientation. The 30bp UMI was then identified upstream of the SMART primer by pattern matching for GTACTCTGCGTTGATACCACTGCTT. Errors were corrected or the read removed based the number of UMI errors, and then the UMI was added to the read name. Next, the TSO associated UMI is identified using the SMART primer sequence AAGCAGTGGTATCAACGCAGAGTAAT. The 30bp UMI sequence was then corrected for errors or remove the read based the number of UMI errors and then the UMI was added to the read name. Both the TSO and polyA associated UMIs and primer sequences were removed from the read sequence. For transcrip0t level analysis, the fastq file is then mapped against the transcriptome using minimap2 (v2.22) with the following settings: -ax map-ont -p 0.9 --end-bonus 10 -N 3. The resulting sam file was then sorted and indexed using samtools (Li, H. et al. The Sequence Alignment/Map format and SAMtools. Bioinformatics 25, 2078-2079 (2009)). A custom script was then used to add the transcript name to the XT tag of the samfile for downstream counting by homotrimer deduplication, UMI-tools or mclUMI. For gene level analysis, the fastq data was mapped using minimap2 using the following setting: -ax splice -k 14 --sam-hit-only --secondary=no --junc-bed. The resulting sam file was then sorted and indexed followed by feature annotation using featurecounts (v2.0.1) using the following settings to generate an annotated bam file: featureCounts -a (gtf) -o (output) -R BAM (Liao, Y. et al. featureCounts: an efficient general purpose program for assigning sequence reads to genomic features. Bioinformatics 30, 923-930 (2014)). This bam file was then used for downstream counting by UMI-tools or mclUMI. The reference transcriptome and genomes used for the analysis were hg38_ensembl98 and mm10_ensembl88.

**Illumina bulk RNA sequencing analysis**

The data was processed using a custom cgatcore written pipeline 'pipeline _illumina'. Briefly, the UMIs contained in read1 were corrected based on homotrimer complementarity or were removed from the analysis depending upon a set error threshold. The paired fastq files were then mapped using hisat2 (v2.2.1) (Kim, D. et al. Graph-based genome alignment and genotyping with HISAT2 and HISAT-genotype. Nat Biotechnol 37, 907-915 (2019)) before features being counted using featureCounts using the following commands: featureCounts -a (gtf) -o (output) -R BAM. The resulting XT tagged bam file was then used for downstream counting using homotrimer deduplication, UMI-tools or mclUMI. Basic homotrimer deduplication, where a "majority vote" of each homotrigram is performed, can also be ran prior to UMI-tools and mclUMI by modifying the configuration file according to the documentation.

**UMI-tools deduplication**

Following gene or transcript level mapping, the UMI was extracted from the read. Since UMI-tools was not designed to correct homotrimer sequences, the UMI was collapsed into single nucleotide sequence by selecting the first base within the trimer sequence (i.e. each of the individual trimers). Reads were then deduplicated using the following command: umi_tools count -per-gene -gene-tag=XT.

**Homotrimer deduplication**

Following gene or transcript level mapping, the UMI was extracted from the read and collapsed into single nucleotide sequence using the majority vote approach where applicable or resolve inconsistencies through a combinatorial optimization scheme otherwise. Briefly, the read was first filtered to exclude reads in which there were more than 3 errors in the UMI sequence. For each trimer within the UMI sequence/ UMI sequences where each trimer contains at least two identical nucleotides, a majority vote was then performed to collapse the trimer into a monomer. For Examples 1 to 4, for trimers that could not be resolved by the majority vote, a base was selected from the trimer by random. The collapsed name was then appended to the name of the Fastq file, which was then used within downstream counting using a unique approach that groups reads that share the same UMI or the directional adjacency approach used by UMI-tools. For Example 5, if at least one trimer was inconclusive and contained three different nucleotides, each UMI sequence is no longer treated independently when collapsing trimers into monomers. Instead, one of the nucleotides in each trimer nucleotide block is selected to achieve maximal consistency between duplicates, i.e. to minimize the number of distinct collapsed UMI sequences. This task is formulated as a set cover problem for each gene as follows. Let S be the set of sequenced homotrimer UMIs of a given gene (in a given cell). For $s \in S$ let $C(s)$ denote the set of collapsed UMIs that can be obtained by combining single nucleotides that occur in each trimer nucleotide block of $s$. Each such collapsed sequence $c \in C(s)$, for some $s \in S$, can explain potentially multiple homotrimer UMIs $s'$ if c is also contained in $C(s')$. Therefore include one subset $S_c \subseteq S$ for each $c \in \cup_{\{s \in S\}} C(s)$ that contains all $s \in S$ for which $c \in C(s)$. The collection of sets $S_c$ of smallest cardinality that together include ("cover") all sequenced UMIs in $S$ therefore corresponds to the smallest set of collapsed UMIs that explain all $s \in S$. To find this smallest set of collapsed UMIs, employ a greedy algorithm that starts from the empty set and in each iteration adds the subset $S_c$ (i.e. collapsed UMI $c$) that explains the largest number of yet unexplained sequenced UMIs. The solution returned by this algorithm is guaranteed to be within a logarithmic factor of the optimal solution[23]. In the experiments escribed herein, the solution of the greedy approach was identical to the optimal solution for more than 90% of the genes. The optimal solution was computed using an integer linear programming approach, where decision variables model the inclusion or exclusion of sets $S_c$ and linear inequalities enforce each sequenced UMI to be covered by at least one such set, i.e. to be explained by at least one collapsed UMI.

**Settings for simulated UMIs**

We simulated UMI data of length 30 (10 blocks of nucleotide trimers) to confirm the accuracy of our UMI correction methodology by using the ResimPy tool. We mimicked the PCR amplification and sequencing errors seen with ONT sequencing, as this sequencing methodology suffers from indels and basecalling errors more frequently than PacBio or Illumina sequencing. UMIs were generated following an approach that was first described by UMI-tools[10]. Briefly, we simulated homotrimer nucleotide blocks of UMIs at random, with an amplification rate (-ampl_rate) ranging between 0.8-1.0 and then simulated PCR cycles so that each UMI was duplicated to the probability of amplification. PCR errors were then randomly added and assigned new probabilities of amplification. A defined number of UMIs were randomly sampled to simulate sequencing depth and sequencing errors introduced with a specified probability. Finally, errors were detected by assessing the complementarity of homotrimers across the full UMI sequence. If no errors are detected, then the homotrimers are collapsed into single nucleotide bases. However, if errors are identified then collapsing into single nucleotides is performed using the most common nucleotide within the trimer. If a most common nucleotide cannot be determined, then a single nucleotide is selected at random for collapsing. The following values were used as values within our simulations. Sequencing depth 10-400; number of UMIs 10-100 (-umi_num); UMI-length 6 - 16 (-umi_len); PCR error rate $1\times10^{-3}$ - $1\times10^{-5}$ (-seq_err); sequencing error rate $1\times10^{-1}$ - $1\times10^{-7}$ and number of PCR cycles 4-12 (-pcr_num); permutation tests 10-100 (-perm_num).

**ResimPy - Simulating chimeric artefacts in UMI sequences**

We developed a UMI simulation package called ResimPy. The number of UMI sequences m to be amplified at PCR i in ResimPy is described as the Galton-Watson branching (GWB) process

$$m^{(i+1)} = \sum_{j=1}^{m^{(i)}} (1 + \sigma_j^{(i)})$$

where $\sigma_j^{(i)}$ is the Kronecker symbol to represent the presence or absence of the *jth* sequence at PCR i with an amplification rate $\alpha$, given by

$$\begin{cases} 0, & p \in (0,1-\alpha) \\ 1, & p \in (\alpha, 1) \end{cases}$$

We used a binomial distribution $Binom(m^{(i)}, \alpha)$ to generate $m^{(i+1)}$. The $m_a^{(i+1)}$ UMI sequences were chosen from its preceding PCR cycle i based on a uniform distribution $U(0, m^{(i)})$. To simulate PCR errors, we also implemented another GWB process. The total number of base errors $n_e^{(i+1)}$ at PCR cycle i + 1 is modelled by

$$n_e^{(i+1)} = \sum_{k=1}^{n_e^{(i)}} (1 + \gamma_k^{(i)})$$

where $\gamma_k^{(i)}$ is the Kronecker symbol to indicate a binary state (*i.e.,* erroneous or correct) of a position of sequences at PCR cycle i,

$$\begin{cases} 0, & p \in (0,1-p_e) \\ 1, & p \in (p_e, 1) \end{cases}$$

where $p_e$ is the PCR error rate. We used a negative binomial distribution $NBinom(q \times n^{(i)}, q)$ to generate $n_e^{(i+1)}$, where $q = 1 - p_e$. These $n_e^{(i+1)}$ sequence positions were then randomly sampled based on a uniform distribution $U(0, n^{(i+1)})$ where $n^{(i+1)}$ represents the total number of bases at PCR cycle i + 1. Each PCR base error determined above is finally substituted by one of the remaining three types of bases to be drawn from a uniform distribution U(0,3). Likewise, we adopted the same way to simulate sequencing errors.

We simulated reads mixed with chimeric artefacts using the *resimpy_umi transloc* module in Resimpy. Briefly, a total of 50 reads were generated, each attached with a UMI included at the 3' end and a UMI at the 5' end. Each randomly simulated UMI of homotrimer nucleotide blocks was 30bp in length. We simulated UMIs with at least a 3-base edit distance away from one another. The chimeric frequency was set to 2% of the total read count. The number of wrongly synthesized bases due to DNA polymerase errors and sequencing errors were estimated from our bulk data using negative binomial models as given in the section above. All other parameters for simulation were kept the same as the simulation of UMIs as above.

**Removal of 5' and 3' UMI tagged chimeric artefacts**

To remove chimeric artefacts we developed umiRarity to detect chimeric artefacts through combining UMIs at 3' and 5' ends into one merged sequence. We leverage the fact that chimeric artefacts will generate UMI pairs at a significantly lower frequency than real translocations. The combination of UMIs at both ends can further bring the frequency down to a level which is lower than that only using UMI at either 5' or 3' end. To this end, we counted the combined homotrimer UMIs and also the combined monomer UMIs corrected from their homotrimer UMIs. We then set a count threshold to remove reads tagged by the combined UMIs below this threshold and retain reads above this threshold. The removed reads are considered as chimeric artefacts and the remaining reads are classified as true translocated reads. Methodological details of the chimeric artefact detection process are illustrated in Supplementary Fig. 5. The performance of chimeric artefact removal was evaluated by comparisons between the percentage of successfully removed chimeric artefacts and the percentage of sacrificed true reads, and between the percentage of successfully removed chimeric artefacts and the percentage of successfully detected PCR duplicates. The count thresholds used in our study range from 1-5. The umiRarity method is available as an independent module in our package mclUMI, which can be accessed by 'mclumi dechimeric -m dc_by_cnt'.

**Common Molecular Identifiers (CMI) and error evaluation in bulk sequencing**

To measure the error rate and evaluate the accuracy of our UMIs following library preparation and sequencing we synthesised a common sequence (GGGAAACCCTTTGGGCCCTTTAAACCCTTT) in replacement of a UMI to our polyA capture oligonucleotide. Following sequencing the CMI sequence was identified upstream of the SMART primer by pattern matching for GTACTCTGCGTTGATACCACTGCTT. The accuracy of our CMI was then determined by comparing the expected synthesised sequence to the extracted CMI sequence. The percent of CMI that show full complementary with the expected sequence were counted and the number of errors were determined for the inaccurate CMIs.

**Comparison between UMI-tools and homotrimer CMI deduplication methods**

After mapping the reads to the reference genome at the gene level, the data was either processed using UMI-tools, mclUMI or by homotrimer deduplication. For homotrimer deduplication, the full length of the CMI sequence was used, while for UMI-tools the CMI was collapsed into a monomer by selecting the first base for each trimer nucleotide block. The inclusion of the CMI sequence to the reads provides an experimental ground truth with which to evaluate the accuracy of each deduplication strategy. The accuracy of the final deduplicated counts from each method was compared to the expected ground truth CMI gene count of 1.

**Identification of fusion transcripts within ONT sequencing data**

Following gene level mapping of the ONT sequencing data, the sam file was filtered using samtools to remove all non-primary alignments and supplementary alignments. mclUMI was then ran to remove the chimeric artefacts from true genomic chimeric fusions using the following settings: mclumi dechimeric -m dc_by_cnt -ibam (infile) -tcthres 5 -obam dechimeric.bam -obam_c chimerical.bam. Chimeric reads were identified based upon the sam flag tag SA. All chimeric fusions were then annotated using the bed file of genes and genomic coordinates followed by filtering and then counting using pysam and the collections. Counter module in Python.

**scCOLOR-seq analysis workflow**

The data was processed using a custom written cgatcore pipeline (Cribbs, A. et al. CGAT-core: a python framework for building scalable, reproducible computational biology workflows [version 1; peer review: 1 approved, 1 approved with reservations]. F1000Research 8 (2019)). The workflow for scCOLOR sequencing analysis has previously been described (Philpott, M. et al. Nanopore sequencing of single-cell transcriptomes with scCOLOR-seq. Nat Biotechnol 39, 1517-1520 (2021)). Briefly, the barcode and dimer UMI sequences were identified by searching for the polyA region and flanking regions before and after the barcode/UMI. The trimer UMI is identified based upon the primer sequence AAGCAGTGGTATCAACGCAGAGTAAT and allowing for two mismatches. Both homodimer UMI and homotrimer UMI correction are performed prior to mapping using minimap2 (v2.22) ( Li, H. Minimap2: pairwise alignment for nucleotide sequences. Bioinformatics 34, 3094-3100 (2018)) with the following settings: -ax splice -uf -MD -sam-hit-only -junc-bed and using the reference transcriptome for human hg38 and mouse mm10. The resulting sam file was sorted and indexed using samtools. Counting was then performed using UMI-tools to count features to cells before being converted to a market matrix format. When using UMI-tools to correct homodimer UMIs, a modified version of the algorithm was used.

Raw transcript expression matrices generated by UMI-tools count were processed using R/Bioconductor (v4.0.3) and the Seurat package (v3.1.4). Gene matrices were cell-level scaled and log-transformed. The top 2000 highly variable genes were then selected based on variance stabilising transformation which was used for principal component analysis (PCA). Clustering was performed within Seurat using the Louvain algorithm. To visualise the single-cell data, we projected data onto a Uniform Manifold Approximation and Projection (UMAP). Cell type determination was performed using clustifyr v1.0.0 to identify correlated gene expression between single-cells and bulk RNA-seq gene lists from the harmonize database

**10X analysis workflow**

To process the 10X chromium data, a custom cgatcore pipeline was used (https://github.com/cribbslab/TallyNNN/blob/main/tallynnn/pipeline_10x.py) (Cribbs, A. et al. CGAT-core: a python framework for building scalable, reproducible computational biology workflows [version 1; peer review: 1 approved, 1 approved with reservations]. F1000Research 8 (2019)). First the orientation of the reads was determined and if a polyT sequence was detected the read was we reverse complemented. Next, the barcode and umi were identified based on the pairwise alignment of the sequence AGATCGGAAGAGCGT and AAAAAAAAA and the sequence between these alignments was identified. Next reads that were greater or equal to 28 bp were removed and the barcode was isolated as the first 16 bp and the UMI the following 12 bp. The barcode and UMI sequence were then appended to the name of the fastq read using the underscore delimiter. Next, to remove barcode errors the barcodes were parsed from each read in the fastq file and then the most common barcode sequences were selected using the number of expected cells in the library as the threshold. Next, for every read in the fastq file the closest barcode match for each read was identified, allowing for two mismatches. Mapping was performed using minimap2 (v2.22)[25], with the following settings: -ax splice -uf -MD -sam-hit-only -junc-bed and using the reference transcriptome for human hg38 and mouse mm10. The resulting bam file was sorted and indexed before adding the transcript name to the XT tag within the bam file. Counting was then performed using UMI-tools -method=unique before being converted to a market matrix format. Raw transcript expression matrices generated by UMI-tools count were processed using R/Bioconductor (v4.0.3) and the Seurat package (v3.1.4). Transcript matrices were cell-level scaled and log-transformed. The top 2000 highly variable genes were then selected based on variance stabilising transformation which was used for principal component analysis (PCA). Clustering was performed within Seurat using the Louvain algorithm. To visualise the single-cell data, we projected data onto a Uniform Manifold Approximation and Projection (UMAP).

**Drop-seq analysis workflow**

To process the drop-seq data, a custom cgatcore pipeline was used (https://github.com/cribbslab/Bulk-TallyNNN) (Cribbs, A. et al. CGAT-core: a python framework for building scalable, reproducible computational biology workflows [version 1; peer review: 1 approved, 1 approved with reservations]. F1000Research 8 (2019)). The workflow previously described for identifying barcodes and UMIs using scCOLOR-seq sequencing analysis was followed. Briefly, to determine the orientation of reads, the presence of a polyA sequence or a polyT sequence was searched for. In cases were the polyT was identified, the reverse complemented was read. Next the barcode sequence was identified by searching for the polyA region and flanking regions before and after the barcode. The trimer UMI was identified based upon the primer sequence GTACTCTGCGTT at the TSO distal end of the read, allowing for two mismatches. Barcodes and UMIs that had a length less than 48 base pairs were filtered. To conduct monomer-based analyses, a random base was selected from each homotrimer in the UMI or CMI and collapsed into a monomer. Homotrimer UMI correction was performed following mapping using minimap2 (v2.22). Mapping settings were as follows: -ax splice -uf - MD -sam-hit-only -junc-bed and using the reference transcriptome for human hg38 and mouse mm10. The resulting sam file was sorted and indexed using samtools. For monomer UMI, counting was performed using UMI-tools before being converted to a market matrix format. For homotrimer UMI correction, the counting was performed using the script *greedy.py* within the TallyNNN repository. Raw transcript expression matrices generated by UMI-tools count and *greedy.py* were processed using R/Bioconductor (v4.0.3) and custom scripts were used to generate barnyard plots showing the proportion of mouse and human cells. Transcript matrices were cell-level scaled and centre log ratio transformed. The top 3000 highly variable genes were then selected based on variance stabilising transformation which was used for principal component analysis (PCA). Clustering was performed within Seurat using the Louvain algorithm. To visualise the single-cell data, we projected data onto a Uniform Manifold Approximation and Projection (UMAP).

## Claims

**1.** A method for detecting chimeric artefact polynucleotides produced during amplification of a mixed sample of RNA molecules, the method comprising:

(i) capturing RNA molecules of the sample on a set of capture polynucleotides, wherein the capture polynucleotides each comprise a sample RNA capture region, one of a mixed pool of first identifier sequences, and a PCR handle sequence;
(ii) performing reverse transcription of captured sample RNA molecules using a template switch reverse polymerase and primed using the capture polynucleotides to generate cDNA polynucleotides having 3' end non-templated nucleotides;
(iii) annealing a set of template switch oligonucleotides (TSOs) to the 3' end non-templated nucleotides, wherein the TSOs each comprise one of a mixed pool of second identifier sequences, wherein the first identifier sequences and/or the second identifier sequences each comprise a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two nucleotide substitutions;
(iv) amplifying the cDNA using the capture polynucleotide PCR handle sequences and TSO PCR handle sequences to generate a library of amplified cDNA flanked by first and second identifier sequences;
(v) sequencing the library;
(vi) identifying pairings of first and second identifier sequences flanking the library cDNA molecules;
(vii) counting library cDNA molecules having identified pairings of first and second identifier sequences and identifying pairings that are under-represented in the library; and/or identifying pairings as mismatch pairings when they comprise

a. a first identifier sequence in common with another pairing in the library having a different second identifier sequence to the mismatch pairing; and/or
b. a second identifier sequence in common with another pairing in the library having a different first identifier sequence to the mismatch pairing; and

(viii) identifying a library cDNA molecule that is:

a. flanked by an underrepresented pairing of first and second identifier sequences; and/or
b. flanked by a mismatch pairing of first and second identifier sequences;

as a chimeric artefact polynucleotide.

**2.** The method of claim 1, wherein:

(a) each nucleotide block sequence in the pool differs from each other nucleotide block in the pool by at least three nucleotide substitutions;
(b) the nucleotide block sequences are homodimers or homotrimers;
(c) the same mixed pool of nucleotide block sequences is used for each nucleotide block of each of the first and/or second identifier sequences;
(d) the pool of first and/or second identifier sequences has been generated by degenerate polynucleotide synthesis from a single mixed pool of nucleotide blocks; and/or
(e) all of the first identifier sequences of the pool of first identifier sequences have a different sequence length, and/or a different number of nucleotide blocks, from all of the second identifier sequences of the pool of second identifier sequences.

**3.** The method of claim 1 or 2, wherein the capture polynucleotides are provided on one or more micro-particles, wherein the micro-particles each comprise a micro-bead and an array of capture polynucleotides, optionally wherein the capture polynucleotides of each micro-particle further comprise a barcode sequence, wherein the capture polynucleotides of the same micro-particle each have the same barcode sequence.

**4.** The method of any one of claims 1 to 3, wherein:

(a) the PCR handle sequence, or a portion thereof, and/or the sample polynucleotide sequence, or a portion thereof, and/or the barcode sequence, or a portion thereof, is used together with an identifier sequence of the same library cDNA molecule to deduplicate an identifier sequence and/or to identify pairings of first and second identifier sequences in the library cDNA;
(b) the sample RNA molecules comprise full-length mRNA molecules and/or the sequencing is full-length mRNA sequencing; and/or
(c) the sample RNA molecules are from a plurality of single cells, wherein sample RNA molecules from each cell are contacted with and captured by a separate array of the capture polynucleotides, optionally wherein each array of capture polynucleotides is associated with a separate micro-particle, wherein the capture polynucleotides comprise barcode sequences, and wherein each capture polynucleotide of an array associated with a micro-particle comprises the same barcode sequence as each other capture polynucleotide of the same array and of the same micro particle.

**5.** A method of producing a library of cDNA molecules of mixed sequence from RNA molecules in a sample, the method comprising:

(i) capturing RNA molecules of the sample on a set of capture polynucleotides, wherein the capture polynucleotides each comprise a sample RNA capture region, one of a mixed pool of first identifier sequences and a PCR handle sequence;
(ii) performing reverse transcription of captured sample RNA molecules using a template switch reverse polymerase and primed using the capture polynucleotides to generate cDNA polynucleotides having 3' end non-templated nucleotides;
(iii) annealing a set of template switch oligonucleotides (TSOs) to the 3' end non-templated nucleotides, wherein each TSO comprises a PCR handle sequence and one of a mixed pool of second identifier sequences, wherein the first identifier sequences and/or the second identifier sequences each comprise a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two nucleotide substitutions; and
(iv) amplifying the cDNA using the capture polynucleotide PCR handle sequences and TSO PCR handle sequences to generate a library of amplified cDNA flanked by first and second identifier sequences.

**6.** The method of claim 5, wherein:

(a) each nucleotide block sequence in the pool differs from each other nucleotide block in the pool by at least three nucleotide substitutions; and/or
(b) the nucleotide block sequences are homodimers or homotrimers.

**7.** The method of claim 5 or 6, wherein:

(a) the same mixed pool of nucleotide block sequence is used for each nucleotide block of each of the first and/or second identifier sequences;
(b) the pool of first and/or second identifier sequences has been generated by degenerate polynucleotide synthesis from a single mixed pool of nucleotide blocks; and/or
(c) all of the first identifier sequences of the pool of first identifier sequences have a different sequence length, and/or a different number of nucleotide blocks, from all of the second identifier sequences of the pool of second identifier sequences.

**8.** The method of any one of claims 5 to 7, wherein:

(a) the capture polynucleotides are provided on one or more micro-particles, wherein the micro-particles each comprise a micro-bead and an array of capture polynucleotides, optionally wherein the capture polynucleotides of each micro-particle further comprise a barcode sequence, wherein the capture polynucleotides of the same micro-particle each have the same barcode sequence;
(b) the PCR handle sequence, or a portion thereof, and/or the sample RNA sequence, or a portion thereof, and/or the barcode sequence, or a portion thereof, is used together with an identifier sequence of the same library cDNA molecule to deduplicate an identifier sequence and/or to identify pairings of first and second identifier sequences in library polynucleotides;
(c) the sample RNA molecules comprise full-length mRNA molecules and/or the sequencing is full-length mRNA sequencing; and/or
(d) the sample RNA molecules are from a plurality of single cells, wherein sample RNA molecules from each cell are contacted with and captured by a separate array of the capture polynucleotides, optionally wherein each array of capture polynucleotides is associated with a separate micro-particle, wherein the capture polynucleotides comprise barcode sequences, and wherein each capture polynucleotide of an array associated with a micro-particle comprises the same barcode sequence as each other capture polynucleotide of the same array and of the same micro particle.

**9.** The method of any one of claims 1 to 8, wherein:

(I) the capture polynucleotide comprises a purification tag, and the method comprises separating tagged polynucleotides from non-tagged polynucleotides after step (ii); and/or
(II) step (iv) comprises amplifying the cDNA using one or more primers comprising a purification tag, and separating tagged polynucleotides from non-tagged polynucleotides,

optionally wherein the purification tag is biotin and the tagged polynucleotides are separated from non-tagged polynucleotides by streptavidin pull down.

**10.** The method of any one of claims 5 to 9, further comprising sequencing the library.

**11.** A library produced by the method of claim 7.

**12.** An array of template switch oligonucleotides (TSOs), wherein the TSOs comprise an identifier sequence, wherein the array comprises a pool of different identifier sequences, wherein each identifier sequence of the pool comprises a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two nucleotide substitutions.

**13.** The array of TSOs of claim 12, wherein:

(a) each nucleotide block sequence in the pool differs from each other nucleotide block in the pool by at least three nucleotide substitutions;
(b) the nucleotide block sequences are homodimers or homotrimers;
(c) the same mixed pool of nucleotide block sequence is used for each nucleotide block of each of the first and/or second identifier sequences; and/or
(d) the pool of first and/or second identifier sequences has been generated by degenerate polynucleotide synthesis from a single mixed pool of nucleotide blocks.

**14.** A kit for generating a library of polynucleotides, the kit comprising:

(i) a set of capture polynucleotides, wherein each capture polynucleotide comprises an RNA capture region, one of a mixed pool of first identifier sequences in the set of capture polynucleotides, and a PCR handle sequence; and
(ii) a set of template switch oligonucleotides (TSOs), wherein each TSO comprises a PCR handle sequence, one of a mixed pool of second identifier sequences and a 3' end template switching sequence that can hybridize to 3' non-templated nucleotides added to a cDNA strand by a reverse transcriptase;

wherein each identifier sequence of the pool in the capture polynucleotides and/or the TSOs comprises a series of discrete nucleotide blocks, wherein the discrete nucleotide blocks are from a mixed pool of nucleotide blocks of known sequence, and wherein each nucleotide block sequence in the pool differs from each other nucleotide block in the pool by at least two nucleotide substitutions.

**15.** The kit of claim 14 wherein:

(a) each nucleotide block sequence in the pool differs from each other nucleotide block in the pool by at least three nucleotide substitutions;
(b) each nucleotide block sequence in the pool differs from each other nucleotide block in the pool by at least three nucleotide substitutions;
(c) the nucleotide block sequences are homodimers or homotrimers;
(d) the same mixed pool of nucleotide block sequence is used for each nucleotide block of each of the first and/or second identifier sequences;
(e) the pool of first and/or second identifier sequences has been generated by degenerate polynucleotide synthesis from a single mixed pool of nucleotide blocks;
(f) all of the first identifier sequences of the pool of first identifier sequences have a different sequence length, and/or a different number of nucleotide blocks, from all of the second identifier sequences of the pool of second identifier sequences; and/or
(g) the capture polynucleotides are provided on one or more micro-particles, wherein the micro-particles each comprise a micro-bead and an array of capture polynucleotides, optionally wherein the capture polynucleotides of each micro-particle further comprise a barcode sequence, wherein the capture polynucleotides of the same micro-particle each have the same barcode sequence.

## Patentansprüche

**1.** Verfahren zum Nachweisen chimärer Artefaktpolynukleotide, die während der Amplifikation einer gemischten Probe von RNA-Molekülen produziert werden, wobei das Verfahren umfasst:

(i) Einfangen von RNA-Molekülen der Probe an einem Satz von Einfangpolynukleotiden, wobei die Einfangpolynukleotide jeweils eine Proben-RNA-Einfangregion, eine aus einem gemischten Pool von ersten Kennungssequenzen und eine PCR-Handle-Sequenz umfassen;
(ii) Durchführen einer reversen Transkription von eingefangenen Proben-RNA-Molekülen unter Verwendung einer reversen Polymerase mit Matrizenwechsel und unter Verwendung der Einfangpolynukleotide geprimed, um cDNA-Polynukleotide mit Nukleotiden ohne Matrize an dem 3'-Ende zu erzeugen;
(iii) Binden eines Satzes von Oligonukleotiden mit Matrizenwechsel (TSOs) an die Nukleotide ohne Matrize an dem 3'-Ende, wobei die TSOs jeweils eine aus einem gemischten Pool von zweiten Kennungssequenzen umfassen, wobei die ersten Kennungssequenzen und/oder die zweiten Kennungssequenzen jeweils eine Reihe von diskreten Nukleotidblöcken umfassen, wobei die diskreten Nukleotidblöcke aus einem gemischten Pool von Nukleotidblöcken mit bekannter Sequenz stammen und wobei sich jede Nukleotidblocksequenz in dem Pool von jedem anderen Nukleotidblock in dem Pool durch wenigstens zwei Nukleotidsubstitutionen unterscheidet;
(iv) Amplifizieren der cDNA unter Verwendung der PCR-Handle-Sequenzen des Einfangpolynukleotids und der TSO-PCR-Handle-Sequenzen, um eine Bibliothek von amplifizierter cDNA zu erzeugen, die von ersten und zweiten Kennungssequenzen flankiert wird;
(v) Sequenzieren der Bibliothek;
(vi) Identifizieren von Paaren aus ersten und zweiten Kennungssequenzen, die die cDNA-Moleküle der Bibliothek flankieren;
(vii) Zählen der cDNA-Moleküle der Bibliothek, die identifizierte Paare von ersten und zweiten Kennungssequenzen aufweisen, und Identifizieren von Paaren, die in der Bibliothek unterrepräsentiert sind; und/oder Identifizieren von Paaren als Nichtübereinstimmungspaare, wenn sie umfassen:

a. eine erste Kennungssequenz, die mit einem anderen Paar in der Bibliothek gemeinsam ist, das eine andere zweite Kennungssequenz als das Nichtübereinstimmungspaar aufweist; und/oder
b. eine zweite Kennungssequenz, die mit einem anderen Paar in der Bibliothek gemeinsam ist, das eine andere erste Kennungssequenz als das Nichtübereinstimmungspaar aufweist; und

(viii) Identifizieren eines cDNA-Moleküls aus der Bibliothek, das:

a. flankiert von einem unterrepräsentierten Paar erster und zweiter Kennungssequenzen ist; und/oder
b. flankiert von einem Nichtübereinstimmungspaar aus erster und zweiter Kennungssequenz ist;

als ein chimäres Artefaktpolynukleotid.

**2.** Verfahren nach Anspruch 1, wobei:

(a) sich jede Nukleotidblocksequenz in dem Pool von jedem anderen Nukleotidblock in dem Pool durch wenigstens drei Nukleotidsubstitutionen unterscheidet;
(b) die Nukleotidblocksequenzen Homodimere oder Homotrimere sind;
(c) derselbe gemischte Pool von Nukleotidblocksequenzen für jeden Nukleotidblock jeder der ersten und/oder zweiten Kennungssequenzen verwendet wird;
(d) der Pool von ersten und/oder zweiten Kennungssequenzen durch degenerierte Polynukleotidsynthese aus einem einzigen gemischten Pool von Nukleotidblöcken erzeugt wurde; und/oder
(e) alle ersten Kennungssequenzen des Pools der ersten Kennungssequenzen eine andere Sequenzlänge und/oder eine andere Anzahl von Nukleotidblöcken aufweisen als alle zweiten Kennungssequenzen des Pools der zweiten Kennungssequenzen.

**3.** Verfahren nach Anspruch 1 oder 2, wobei die Einfangpolynukleotide auf einem oder mehreren Mikropartikeln bereitgestellt werden, wobei die Mikropartikel jeweils ein Mikrokügelchen und eine Anordnung von Einfangpolynukleotiden umfassen, wobei die Einfangpolynukleotide jedes Mikropartikels optional ferner eine Barcode-Sequenz umfassen, wobei die Einfangpolynukleotide desselben Mikropartikels jeweils dieselbe Barcode-Sequenz aufweisen.

**4.** Verfahren nach einem der Ansprüche 1 bis 3, wobei:

(a) die PCR-Handle-Sequenz oder ein Abschnitt davon und/oder die Probenpolynukleotidsequenz oder ein Abschnitt davon und/oder die Barcode-Sequenz oder ein Abschnitt davon zusammen mit einer Kennungssequenz desselben Bibliotheks-cDNA-Moleküls verwendet wird, um eine Kennungssequenz zu deduplizieren und/oder um Paare von ersten und zweiten Kennungssequenzen in der Bibliotheks-cDNA zu identifizieren;
(b) die Proben-RNA-Moleküle mRNA-Moleküle in voller Länge umfassen und/oder die Sequenzierung eine Sequenzierung der mRNA in voller Länge ist; und/oder
(c) die Proben-RNA-Moleküle aus einer Mehrzahl von einzelnen Zellen stammen, wobei die Proben-RNA-Moleküle aus jeder Zelle mit einer separaten Anordnung der Einfangpolynukleotide in Berührung gebracht und von dieser eingefangen werden, wobei optional jede Anordnung von Einfangpolynukleotiden mit einem separaten Mikropartikel assoziiert ist, wobei die Einfangpolynukleotide Barcode-Sequenzen umfassen und wobei jedes Einfangpolynukleotid einer Anordnung, die mit einem Mikropartikel assoziiert ist, dieselbe Barcode-Sequenz wie jedes andere Einfangpolynukleotid derselben Anordnung und desselben Mikropartikels umfasst.

**5.** Verfahren zum Herstellen einer Bibliothek von cDNA-Molekülen mit gemischter Sequenz aus RNA-Molekülen in einer Probe, wobei das Verfahren umfasst:

(i) Einfangen von RNA-Molekülen der Probe an einem Satz von Einfangpolynukleotiden, wobei die Einfangpolynukleotide jeweils eine Proben-RNA-Einfangregion, eine aus einem gemischten Pool von ersten Kennungssequenzen und eine PCR-Handle-Sequenz umfassen;
(ii) Durchführen einer reversen Transkription von eingefangenen Proben-RNA-Molekülen unter Verwendung einer reversen Polymerase mit Matrizenwechsel und unter Verwendung der eingefangenen Polynukleotide geprimed, um cDNA-Polynukleotide mit Nukleotiden ohne Matrize an dem 3'-Ende zu erzeugen;
(iii) Binden eines Satzes von Oligonukleotiden mit Matrizenwechsel (TSOs) an die Nukleotide ohne Matrize an dem 3'-Ende, wobei jedes TSO eine PCR-Handle-Sequenz und eine aus einem gemischten Pool von zweiten Kennungssequenzen umfasst, wobei die ersten Kennungssequenzen und/oder die zweiten Kennungssequenzen jeweils eine Reihe von diskreten Nukleotidblöcken umfassen, wobei die diskreten Nukleotidblöcke aus einem gemischten Pool von Nukleotidblöcken mit bekannter Sequenz stammen und wobei sich jede Nukleotidblocksequenz in dem Pool von jedem anderen Nukleotidblock in dem Pool durch wenigstens zwei Nukleotidsubstitutionen unterscheidet; und
(iv) Amplifizieren der cDNA unter Verwendung der PCR-Handle-Sequenzen des Einfangpolynukleotids und der TSO-PCR-Handle-Sequenzen, um eine Bibliothek von amplifizierter cDNA zu erzeugen, die von ersten und zweiten Kennungssequenzen flankiert wird.

**6.** Verfahren nach Anspruch 5, wobei:

(a) sich jede Nukleotidblocksequenz in dem Pool von jedem anderen Nukleotidblock in dem Pool durch wenigstens drei Nukleotidsubstitutionen unterscheidet; und/oder
(b) die Nukleotidblocksequenzen Homodimere oder Homotrimere sind.

**7.** Verfahren nach Anspruch 5 oder 6, wobei:

(c) derselbe gemischte Pool einer Nukleotidblocksequenz für jeden Nukleotidblock jeder der ersten und/oder zweiten Kennungssequenzen verwendet wird;
(b) der Pool von ersten und/oder zweiten Kennungssequenzen durch degenerierte Polynukleotidsynthese aus einem einzigen gemischten Pool von Nukleotidblöcken erzeugt wurde; und/oder
(c) alle ersten Kennungssequenzen des Pools der ersten Kennungssequenzen eine andere Sequenzlänge und/oder eine andere Anzahl von Nukleotidblöcken aufweisen als alle zweiten Kennungssequenzen des Pools der zweiten Kennungssequenzen.

**8.** Verfahren nach einem der Ansprüche 5 bis 7, wobei:

(a) die Einfangpolynukleotide auf einem oder mehreren Mikropartikeln bereitgestellt werden, wobei die Mikropartikel jeweils ein Mikrokügelchen und eine Anordnung von Einfangpolynukleotiden umfassen, wobei die Einfangpolynukleotide jedes Mikropartikels optional ferner eine Barcode-Sequenz umfassen, wobei die Einfangpolynukleotide desselben Mikropartikels jeweils dieselbe Barcode-Sequenz aufweisen;
(a) die PCR-Handle-Sequenz oder ein Abschnitt davon und/oder die Proben-RNA-Sequenz oder ein Abschnitt davon und/oder die Barcode-Sequenz oder ein Abschnitt davon zusammen mit einer Kennungssequenz desselben Bibliotheks-cDNA-Moleküls verwendet wird, um eine Kennungssequenz zu deduplizieren und/oder um Paare von ersten und zweiten Kennungssequenzen in Bibliothekspolynukleotiden zu identifizieren;
(c) die Proben-RNA-Moleküle mRNA-Moleküle in voller Länge umfassen und/oder die Sequenzierung eine Sequenzierung der mRNA in voller Länge ist; und/oder
(d) die Proben-RNA-Moleküle aus einer Mehrzahl von einzelnen Zellen stammen, wobei die Proben-RNA-Moleküle aus jeder Zelle mit einer separaten Anordnung der Einfangpolynukleotide in Berührung gebracht und von dieser eingefangen werden, wobei optional jede Anordnung von Einfangpolynukleotiden mit einem separaten Mikropartikel assoziiert ist, wobei die Einfangpolynukleotide Barcode-Sequenzen umfassen und wobei jedes Einfangpolynukleotid einer Anordnung, die mit einem Mikropartikel assoziiert ist, dieselbe Barcode-Sequenz wie jedes andere Einfangpolynukleotid derselben Anordnung und desselben Mikropartikels umfasst.

**9.** Verfahren nach einem der Ansprüche 1 bis 8, wobei:

(I) das Einfangpolynukleotid eine Reinigungsmarkierung umfasst und das Verfahren das Trennen der markierten Polynukleotide von den nicht markierten Polynukleotiden nach Schritt (ii) umfasst; und/oder
(II) Schritt (iv) das Amplifizieren der cDNA unter Verwendung eines oder mehrerer Primer, die eine Reinigungsmarkierung umfassen, und das Trennen markierter Polynukleotide von nicht markierten Polynukleotiden umfasst,

wobei die Reinigungsmarkierung optional Biotin ist und die markierten Polynukleotide von den nicht markierten Polynukleotiden durch Streptavidin-Pulldown getrennt werden.

**10.** Verfahren nach einem der Ansprüche 5 bis 9, ferner umfassend das Sequenzieren der Bibliothek.

**11.** Bibliothek, die durch das Verfahren nach Anspruch 7 hergestellt wird.

**12.** Anordnung von Oligonukleotiden mit Matrizenwechsel (TSOs), wobei die TSOs eine Kennungssequenz umfassen, wobei die Anordnung einen Pool von verschiedenen Kennungssequenzen umfasst, wobei jede Kennungssequenz des Pools eine Reihe von diskreten Nukleotidblöcken umfasst, wobei die diskreten Nukleotidblöcke aus einem gemischten Pool von Nukleotidblöcken mit bekannter Sequenz stammen und wobei sich jede Nukleotidblocksequenz in dem Pool von jedem anderen Nukleotidblock in dem Pool durch wenigstens zwei Nukleotidsubstitutionen unterscheidet.

**13.** Anordnung von TSOs nach Anspruch 12, wobei:

(a) sich jede Nukleotidblocksequenz in dem Pool von jedem anderen Nukleotidblock in dem Pool durch wenigstens drei Nukleotidsubstitutionen unterscheidet;

(b) die Nukleotidblocksequenzen Homodimere oder Homotrimere sind;
(c) derselbe gemischte Pool einer Nukleotidblocksequenz für jeden Nukleotidblock jeder der ersten und/oder zweiten Kennungssequenzen verwendet wird; und/oder
(d) der Pool von ersten und/oder zweiten Kennungssequenzen durch degenerierte Polynukleotidsynthese aus einem einzigen gemischten Pool von Nukleotidblöcken erzeugt wurde.

**14.** Kit zum Erzeugen einer Bibliothek von Polynukleotiden, wobei der Kit umfasst:

(i) einen Satz von Einfangpolynukleotiden, wobei jedes Einfangpolynukleotid eine RNA-Einfangregion, eine aus einem gemischten Pool von ersten Kennungssequenzen in dem Satz von Einfangpolynukleotiden und eine PCR-Handle-Sequenz umfasst; und
(ii) einen Satz von Oligonukleotiden mit Matrizenwechsel (TSOs), wobei jedes TSO eine PCR-Handle-Sequenz, eine aus einem gemischten Pool von zweiten Kennungssequenzen und eine Sequenz mit Matrizenwechsel an dem 3'-Ende umfasst, die an 3'-Nukleotide ohne Matrizenwechsel hybridisieren kann, die durch eine reverse Transkriptase zu einem cDNA-Strang hinzugefügt wurden;

wobei jede Kennungssequenz des Pools in den Einfangpolynukleotiden und/oder TSOs eine Reihe von diskreten Nukleotidblöcken umfasst, wobei die diskreten Nukleotidblöcke aus einem gemischten Pool von Nukleotidblöcken mit bekannter Sequenz stammen und wobei sich jede Nukleotidblocksequenz in dem Pool von jedem anderen Nukleotidblock in dem Pool durch wenigstens zwei Nukleotidsubstitutionen unterscheidet.

**15.** Kit nach Anspruch 14, wobei:

(a) sich jede Nukleotidblocksequenz in dem Pool von jedem anderen Nukleotidblock in dem Pool durch wenigstens drei Nukleotidsubstitutionen unterscheidet;
(b) sich jede Nukleotidblocksequenz in dem Pool von jedem anderen Nukleotidblock in dem Pool durch wenigstens drei Nukleotidsubstitutionen unterscheidet;
(c) die Nukleotidblocksequenzen Homodimere oder Homotrimere sind;
(d) derselbe gemischte Pool einer Nukleotidblocksequenz für jeden Nukleotidblock jeder der ersten und/oder zweiten Kennungssequenzen verwendet wird;
(e) der Pool von ersten und/oder zweiten Kennungssequenzen durch degenerierte Polynukleotidsynthese aus einem einzigen gemischten Pool von Nukleotidblöcken erzeugt wurde;
(f) alle ersten Kennungssequenzen des Pools der ersten Kennungssequenzen eine andere Sequenzlänge und/oder eine andere Anzahl von Nukleotidblöcken aufweisen als alle zweiten Kennungssequenzen des Pools der zweiten Kennungssequenzen; und/oder
(g) die Einfangpolynukleotide auf einem oder mehreren Mikropartikeln bereitgestellt werden, wobei die Mikropartikel jeweils ein Mikrokügelchen und eine Anordnung von Einfangpolynukleotiden umfassen, wobei die Einfangpolynukleotide jedes Mikropartikels optional ferner eine Barcode-Sequenz umfassen, wobei die Einfangpolynukleotide desselben Mikropartikels jeweils dieselbe Barcode-Sequenz aufweisen.

## Revendications

**1.** Procédé de détection de polynucléotides artéfacts chimériques produits pendant l'amplification d'un échantillon mixte de molécules d'ARN, le procédé comprenant :

(i) la capture de molécules d'ARN de l'échantillon sur un ensemble de polynucléotides de capture, dans lequel les polynucléotides de capture comprennent chacun une région de capture d'ARN d'échantillon, l'une d'un fonds mixte de premières séquences d'identification, et une séquence de manipulation PCR ;
(ii) la réalisation d'une transcription inverse de molécules d'ARN d'échantillon capturées à l'aide d'une polymérase inverse de commutateur de matrice et amorcée à l'aide des polynucléotides de capture pour générer des polynucléotides d'ADNc ayant des nucléotides non matrice d'extrémité 3' ;
(iii) l'annelage d'un ensemble d'oligonucléotides commutateurs de matrice (TSO) aux nucléotides non matrice d'extrémité 3', dans lequel les TSO comprennent chacun l'une d'un fonds mixte de deuxièmes séquences d'identification, dans lequel les premières séquences d'identification et/ou les deuxièmes séquences d'identification comprennent chacune une série de blocs nucléotidiques discrets, dans lequel les blocs nucléotidiques discrets proviennent d'un fonds mixte de blocs nucléotidiques de séquence connue, et dans lequel chaque séquence de bloc nucléotidique du fonds diffère de chaque autre bloc nucléotidique du fonds d'au moins deux substitutions nucléotidiques ;

(iv) l'amplification de l'ADNc à l'aide des séquences de manipulation PCR de polynucléotides de capture et des séquences de manipulation PCR de TSO afin de générer une banque d'ADNc amplifié encadrée par des premières et deuxièmes séquences d'identification ;

(v) le séquençage de la banque ;

(vi) l'identification des paires de premières et deuxièmes séquences d'identification encadrant la banque de molécules d'ADNc ;

(vii) le comptage de molécules d'ADNc de banque ayant des paires identifiées des premières et deuxièmes séquences d'identification et l'identification des paires qui sont sous-représentées dans la banque ; et/ou l'identification des paires comme paires de mésappariement lorsqu'elles comprennent

a. une première séquence d'identification commune avec une autre paire dans la banque ayant une deuxième séquence d'identification différente de la paire de mésappariement ; et/ou

b. une deuxième séquence d'identification commune avec une autre paire dans la banque ayant une première séquence d'identification différente de la paire de mésappariement ; et

(viii) l'identification d'une molécule d'ADNc de banque qui est :

a. encadrée par une paire sous-représentée de première et deuxième séquences d'identification ; et/ou

b. encadrée par une paire de mésappariement de première et deuxième séquences d'identification ;

en tant que polynucléotide artéfact chimérique.

**2.** Procédé selon la revendication 1, dans lequel :

(a) chaque séquence de bloc nucléotidique dans le fonds diffère de chaque autre bloc nucléotidique dans le fonds d'au moins trois substitutions nucléotidiques ;

(b) les séquences de blocs nucléotidiques sont des homodimères ou des homotrimères ;

(c) le même fonds mixte de séquences de blocs nucléotidiques est utilisé pour chaque bloc nucléotidique de chacune des premières et/ou deuxièmes séquences d'identification ;

(d) le fonds de premières et/ou deuxièmes séquences d'identification a été généré par synthèse de polynucléotides dégénérés à partir d'un seul fonds mixte de blocs nucléotidiques ; et/ou

(e) l'ensemble des premières séquences d'identification du fonds de premières séquences d'identification ont une longueur de séquence différente, et/ou un nombre de blocs nucléotidiques différent, de l'ensemble des deuxièmes séquences d'identification du fonds de deuxièmes séquences d'identification.

**3.** Procédé selon la revendication 1 ou 2, dans lequel les polynucléotides de capture sont fournis sur une ou plusieurs microparticules, dans lequel les microparticules comprennent chacune une microbille et un réseau de polynucléotides de capture, éventuellement dans lequel les polynucléotides de capture de chaque microparticule comprennent en outre une séquence code-barres, dans lequel les polynucléotides de capture d'une même microparticule ont tous la même séquence code-barres.

**4.** Procédé selon l'une quelconque des revendications 1 à 3, dans lequel :

(a) la séquence de manipulation PCR, ou une portion de celle-ci, et/ou la séquence de polynucléotide d'échantillon, ou une portion de celle-ci, et/ou la séquence code-barres, ou une portion de celle-ci, sont utilisées conjointement avec une séquence d'identification de la même molécule d'ADNc de banque pour dédupliquer une séquence d'identification et/ou pour identifier des paires des premières et deuxièmes séquences d'identification dans l'ADNc de banque ;

(b) les molécules d'ARN d'échantillon comprennent des molécules d'ARNm de pleine longueur et/ou le séquençage est un séquençage d'ARNm de pleine longueur ; et/ou

(c) les molécules d'ARN d'échantillon proviennent d'une pluralité d'unicellulaires, dans lequel des molécules d'ARN d'échantillon issues de chaque cellule sont mises en contact avec et capturées par un réseau distinct de polynucléotides de capture, éventuellement dans lequel chaque réseau de polynucléotides de capture est associé à une microparticule distincte, dans lequel les polynucléotides de capture comprennent des séquences codes-barres, et dans lequel chaque polynucléotide de capture d'un réseau associé à une microparticule comprend la même séquence code-barres que chaque autre polynucléotide de capture du même réseau et de la même microparticule.

**5.** Procédé de production d'une banque de molécules d'ADNc de séquence mixte à partir de molécules d'ARN dans un échantillon, le procédé comprenant :

(i) la capture de molécules d'ARN de l'échantillon sur un ensemble de polynucléotides de capture, dans lequel les polynucléotides de capture comprennent chacun une région de capture d'ARN d'échantillon, l'une d'un fonds mixte de premières séquences d'identification et une séquence de manipulation PCR ;
(ii) la réalisation d'une transcription inverse de molécules d'ARN d'échantillon capturées à l'aide d'une polymérase inverse de commutateur de matrice et amorcée à l'aide des polynucléotides de capture pour générer des polynucléotides d'ADNc ayant des nucléotides non matrice d'extrémité 3' ;
(iii) l'annelage d'un ensemble d'oligonucléotides commutateurs de matrice (TSO) aux nucléotides non matrice d'extrémité 3', dans lequel chaque TSO comprend une séquence de manipulation PCR et l'une d'un fonds mixte de deuxièmes séquences d'identification, dans lequel les premières séquences d'identification et/ou les deuxièmes séquences d'identification comprennent chacune une série de blocs nucléotidiques discrets, dans lequel les blocs nucléotidiquesdiscrets proviennent d'un fonds mixte de blocs nucléotidiques de séquence connue, et dans lequel chaque séquence de bloc nucléotidique du fonds diffère de chaque autre bloc nucléotidique du fonds d'au moins deux substitutions nucléotidiques ; et
(iv) l'amplification de l'ADNc à l'aide des séquences de manipulation PCR de polynucléotides de capture et des séquences de manipulation PCR de TSO afin de générer une banque d'ADNc amplifié encadrée par des première et deuxième séquences d'identification.

**6.** Procédé selon la revendication 5, dans lequel :

(a) chaque séquence de bloc nucléotidique dans le fonds diffère de chaque autre bloc nucléotidique dans le fonds d'au moins trois substitutions nucléotidiques ; et/ou
(b) les séquences de blocs nucléotidiques sont des homodimères ou des homotrimères.

**7.** Procédé selon la revendication 5 ou 6, dans lequel :

(a) le même fonds mixte de séquence de bloc nucléotidique est utilisé pour chaque bloc nucléotidique de chacune des premières et/ou deuxièmes séquences d'identification ;
(b) le fonds de premières et/ou deuxièmes séquences d'identification a été généré par synthèse de polynucléotides dégénérés à partir d'un seul fonds mixte de blocs nucléotidiques ; et/ou
(c) l'ensemble des premières séquences d'identification du fonds de premières séquences d'identification ont une longueur de séquence différente, et/ou un nombre différent de blocs nucléotidiques, de l'ensemble des deuxièmes séquences d'identification du fonds de deuxièmes séquences d'identification.

**8.** Procédé selon l'une quelconque des revendications 5 à 7, dans lequel :

(a) les polynucléotides de capture sont fournis sur une ou plusieurs microparticules, dans lequel les microparticules comprennent chacune une microbille et un réseau de polynucléotides de capture, éventuellement dans lequel les polynucléotides de capture de chaque microparticule comprennent en outre une séquence code-barres, dans lequel les polynucléotides de capture de la même microparticule ont chacun la même séquence code-barres ;
(b) la séquence de manipulation PCR, ou une portion de celle-ci, et/ou la séquence d'ARN d'échantillon, ou une portion de celle-ci, et/ou la séquence code-barres, ou une portion de celle-ci, sont utilisées conjointement avec une séquence d'identification de la même molécule d'ADNc de banque pour dédupliquer une séquence d'identification et/ou pour identifier des paires des premières et deuxièmes séquences d'identification dans des polynucléotides de banque ;
(c) les molécules d'ARN d'échantillon comprennent des molécules d'ARNm de pleine longueur et/ou le séquençage est un séquençage d'ARNm de pleine longueur ; et/ou
(d) les molécules d'ARN d'échantillon proviennent d'une pluralité d'unicellulaires, dans lequel des molécules d'ARN d'échantillon issue de chaque cellule sont mises en contact avec et capturées par un réseau distinct de polynucléotides de capture, éventuellement dans lequel chaque réseau de polynucléotides de capture est associé à une microparticule distincte, dans lequel les polynucléotides de capture comprennent des séquences codes-barres, et dans lequel chaque polynucléotide de capture d'un réseau associé à une microparticule comprend la même séquence code-barres que chaque autre polynucléotide de capture du même réseau et de la même microparticule.

**9.** Procédé selon l'une quelconque des revendications 1 à 8, dans lequel :

(1) le polynucléotide de capture comprend une étiquette de purification, et le procédé comprend la séparation de polynucléotides étiquetés de polynucléotides non étiquetés après l'étape (ii) ; et/ou
(II) l'étape (iv) comprend l'amplification de l'ADNc à l'aide d'une ou plusieurs amorces comprenant une étiquette de purification, et la séparation de polynucléotides étiquetés de polynucléotides non étiquetés,

éventuellement dans lequel l'étiquette de purification est la biotine et les polynucléotides étiquetés sont séparés de polynucléotides non étiquetés par interaction avec la streptavidine (« pull down »).

**10.** Procédé selon l'une quelconque des revendications 5 à 9, comprenant en outre le séquençage de la banque.

**11.** Banque produite par le procédé de la revendication 7.

**12.** Réseau d'oligonucléotides commutateurs de matrice (TSO), dans lequel les TSO comprennent une séquence d'identification, dans lequel le réseau comprend un fonds de séquences d'identification différentes, dans lequel chaque séquence d'identification du fonds comprend une série de blocs nucléotidiques discrets, dans lequel les blocs nucléotidiques discrets proviennent d'un fonds mixte de blocs nucléotidiques de séquence connue, et dans lequel chaque séquence de bloc nucléotidique du fonds diffère de chaque autre bloc nucléotidique du fonds d'au moins deux substitutions nucléotidiques.

**13.** Réseau de TSO selon la revendication 12, dans lequel :

(a) chaque séquence de bloc nucléotidique dans le fonds diffère de chaque autre bloc nucléotidique dans le fonds d'au moins trois substitutions nucléotidiques ;
(b) les séquences de blocs nculéotidiques sont des homodimères ou des homotrimères ;
(c) le même fonds mixte de séquence de bloc nucléotidique est utilisé pour chaque bloc nucléotidique de chacune des premières et/ou deuxièmes séquences d'identification ; et/ou
(d) le fonds de premières et/ou deuxièmes séquences d'identification a été généré par synthèse de polynucléotides dégénérés à partir d'un seul fonds mixte de blocs nucléotidiques.

**14.** Kit de génération d'une banque de polynucléotides, le kit comprenant :

(i) un ensemble de polynucléotides de capture, dans lequel chaque polynucléotide de capture comprend une région de capture d'ARN, l'une d'un fonds mixte de premières séquences d'identification dans l'ensemble de polynucléotides de capture, et une séquence de manipulation PCR ; et
(ii) un ensemble d'oligonucléotides commutateurs de matrice (TSO), dans lequel chaque TSO comprend une séquence de manipulation PCR, l'une d'un fonds mixte de deuxièmes séquences d'identification et une séquence de commutation de matrice d'extrémité 3' qui peut s'hybrider à des nucléotides non matrice 3' ajoutés à un brin d'ADNc par une transcriptase inverse ;

dans lequel chaque séquence d'identification du fonds dans les polynucléotides de capture et/ou les TSO comprend une série de blocs nucléotidiques discrets, dans lequel les blocs nucléotidiques discrets proviennent d'un fonds mixte de blocs nucléotidiques de séquence connue, et dans lequel chaque séquence de bloc nucléotidique du fonds diffère de chaque autre bloc nucléotidique du fonds d'au moins deux substitutions nucléotidiques.

**15.** Kit selon la revendication 14 dans lequel :

(a) chaque séquence de bloc nucléotidique dans le fonds diffère de chaque autre bloc nucléotidique dans le fonds d'au moins trois substitutions nucléotidiques ;
(b) chaque séquence de bloc nucléotidique dans le fonds diffère de chaque autre bloc nucléotidique dans le fonds d'au moins trois substitutions nucléotidiques ;
(c) les séquences de blocs nucléotidiques sont des homodimères ou des homotrimères ;
(d) le même fonds mixte de séquence de bloc nucléotidique est utilisé pour chaque bloc nucléotidique de chacune des premières et/ou deuxièmes séquences d'identification ;
(e) le fonds de premières et/ou deuxièmes séquences d'identification a été généré par synthèse de polynucléotides dégénérés à partir d'un seul fonds mixte de blocs nucléotidiques ;
(f) l'ensemble des premières séquences d'identification du fonds de premières séquences d'identification ont une longueur de séquence différente, et/ou un nombre différent de blocs nucléotidiques, de l'ensemble des deuxièmes séquences d'identification du fonds de deuxièmes séquences d'identification ; et/ou
(g) les polynucléotides de capture sont fournis sur une ou plusieurs microparticules, dans lequel les microparticules comprennent chacune une microbille et un réseau de polynucléotides de capture, éventuellement dans lequel les polynucléotides de capture de chaque microparticule comprennent en outre une séquence code-barres, dans lequel les polynucléotides de capture de la même microparticule ont chacun la même séquence code-barres.

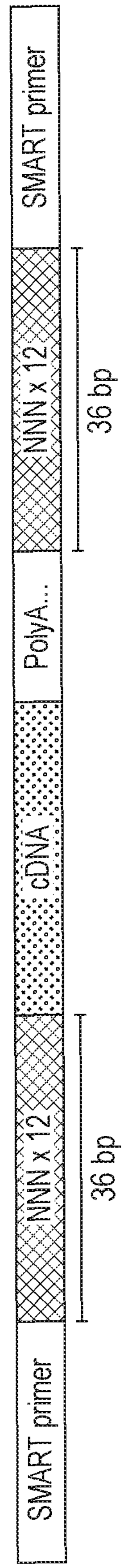


Fig. 1a

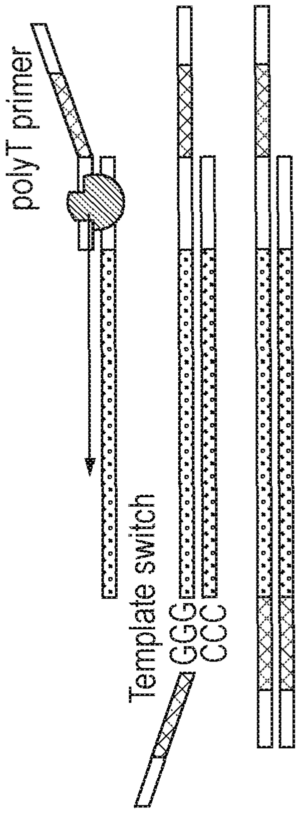


Fig. 1b

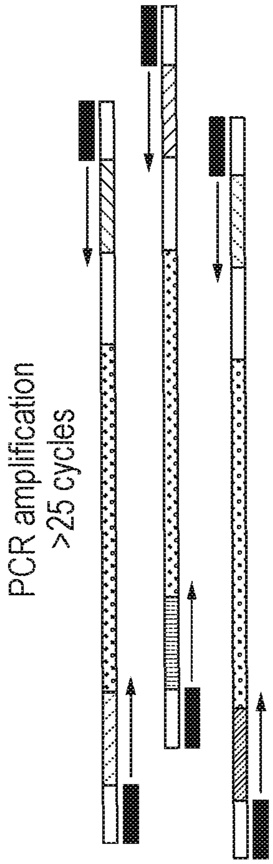


Fig. 1c

# Fig. 1d

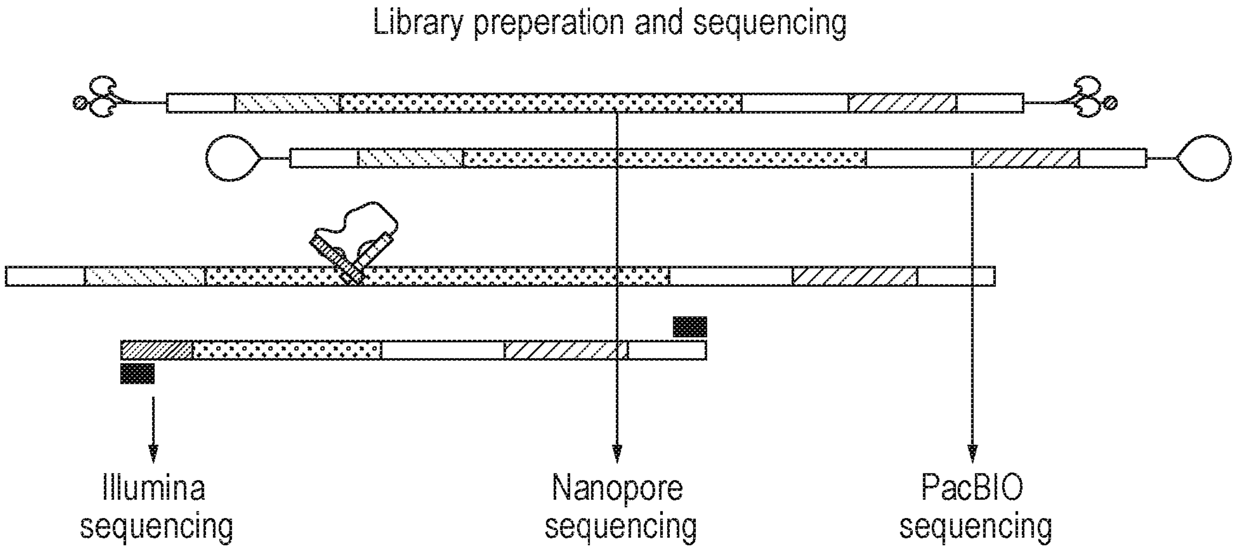
Library preperation and sequencing
Illumina sequencing
Nanopore sequencing
PacBIO sequencing

# Fig. 1e

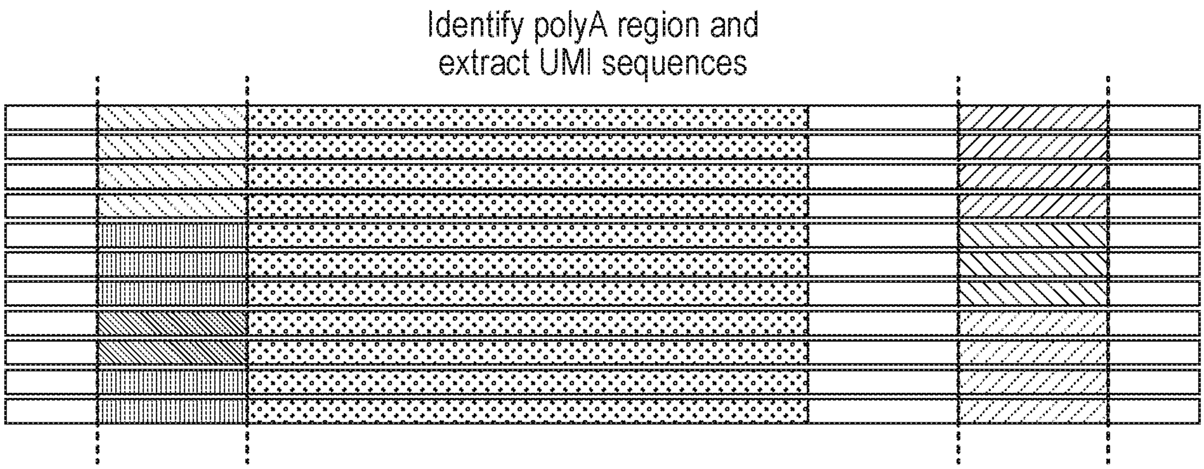
Identify polyA region and extract UMI sequences

Fig. 1f

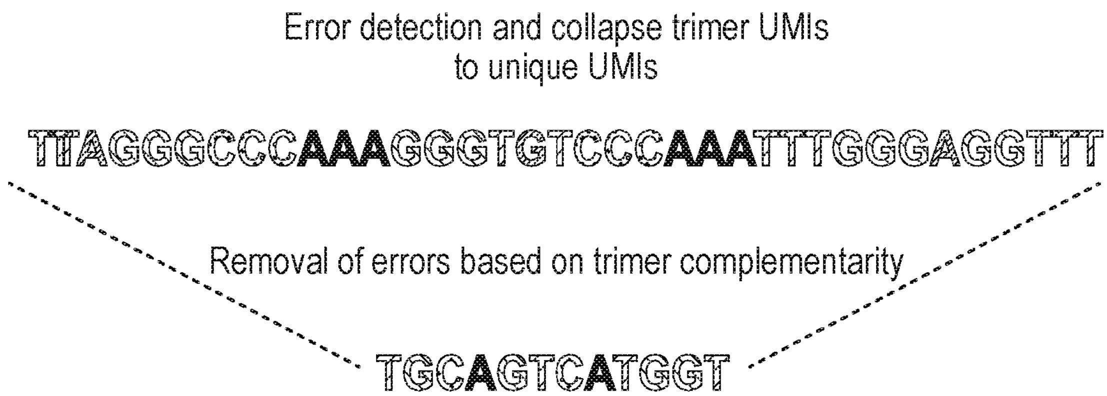
Error detection and collapse trimer UMIs
to unique UMIs
TTTAGGGCCCAAAGGGTGTCCCAAATTTGGGAGGTTT
Removal of errors based on trimer complementarity
TGCAGTCATGGT

Fig. 1g

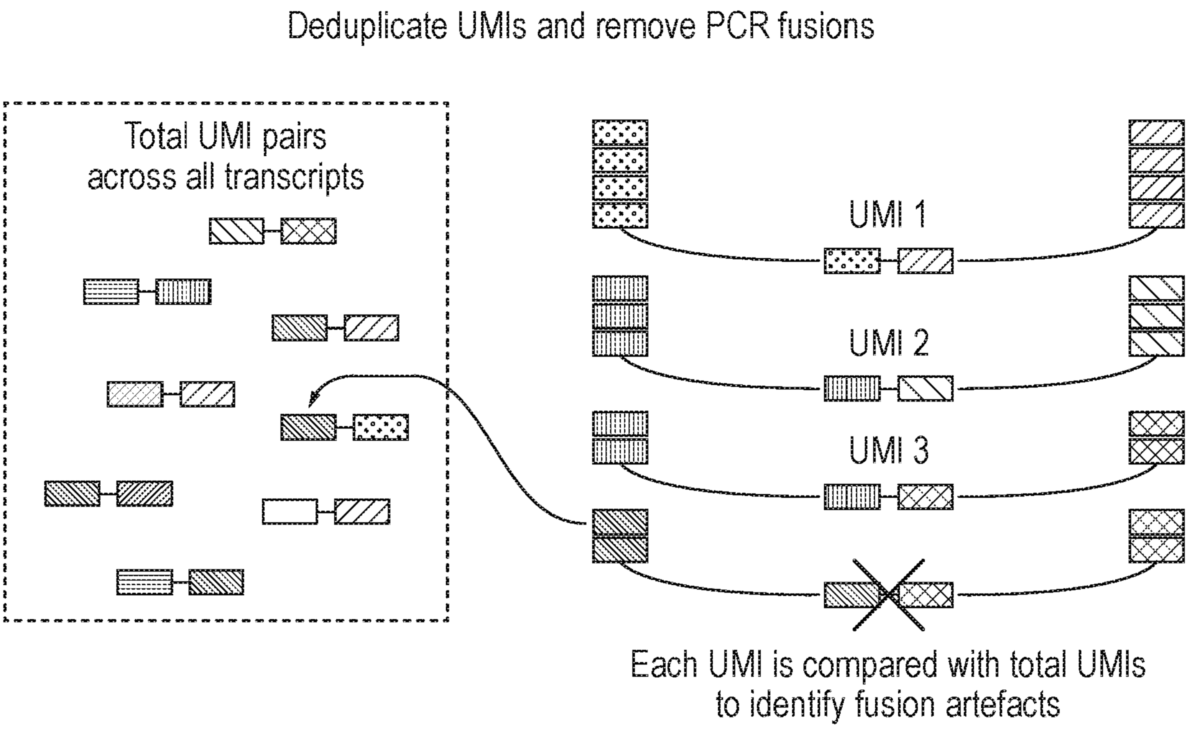
Deduplicate UMIs and remove PCR fusions
Total UMI pairs
across all transcripts
UMI 1
UMI 2
UMI 3
Each UMI is compared with total UMIs
to identify fusion artefacts

Fig. 1h

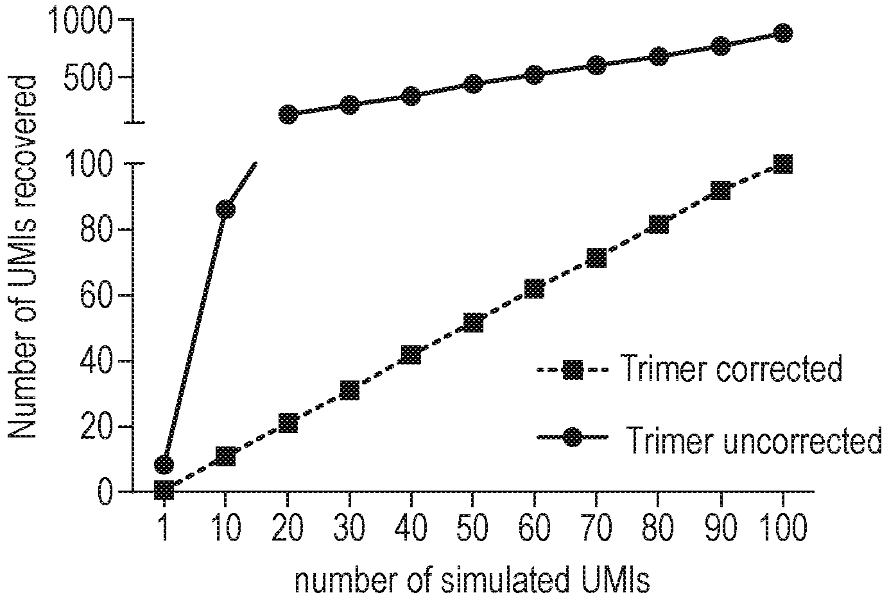
1000
500
100
80
60
40
20
0
Number of UMIs recovered
1
10
20
30
40
50
60
70
80
90
100
number of simulated UMIs
Trimer corrected
Trimer uncorrected

Fig. 1i

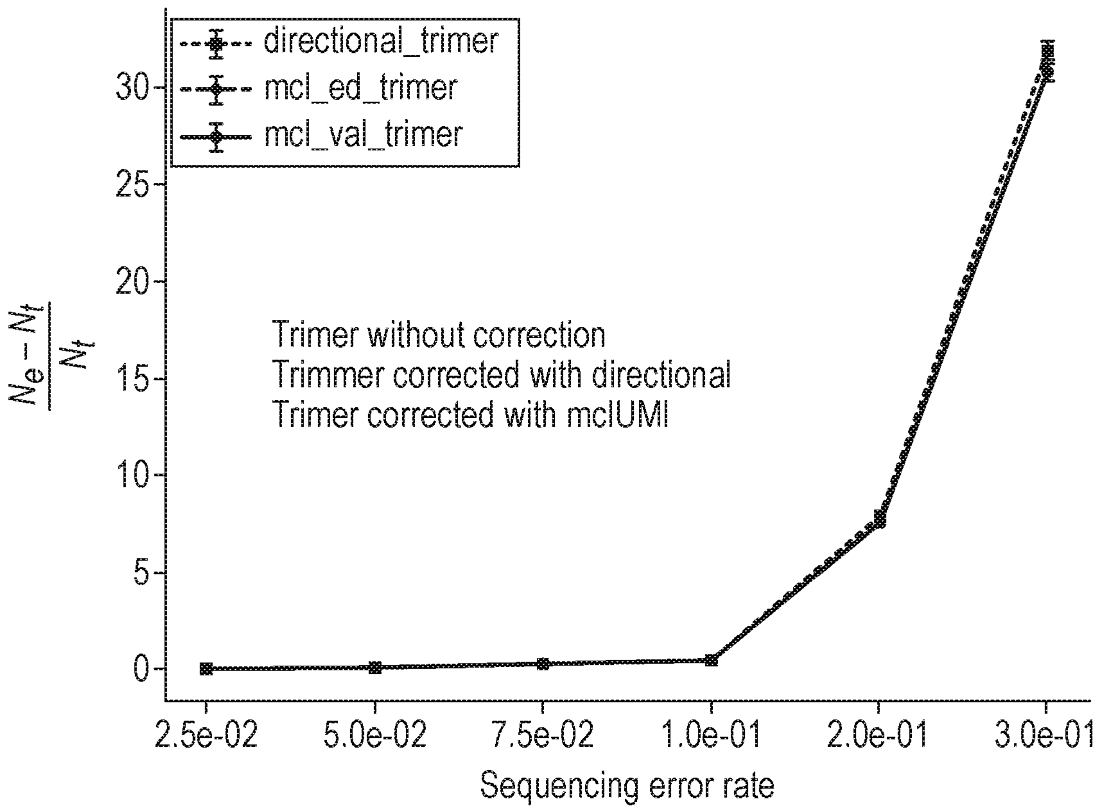
directional_trimer
mcl_ed_trimer
mcl_val_trimer
30
25
20
15
10
5
0
$\frac{N_e - N_t}{N_t}$
Trimer without correction
Trimmer corrected with directional
Trimer corrected with mclUMI
2.5e-02
5.0e-02
7.5e-02
1.0e-01
2.0e-01
3.0e-01
Sequencing error rate

Fig. 1j

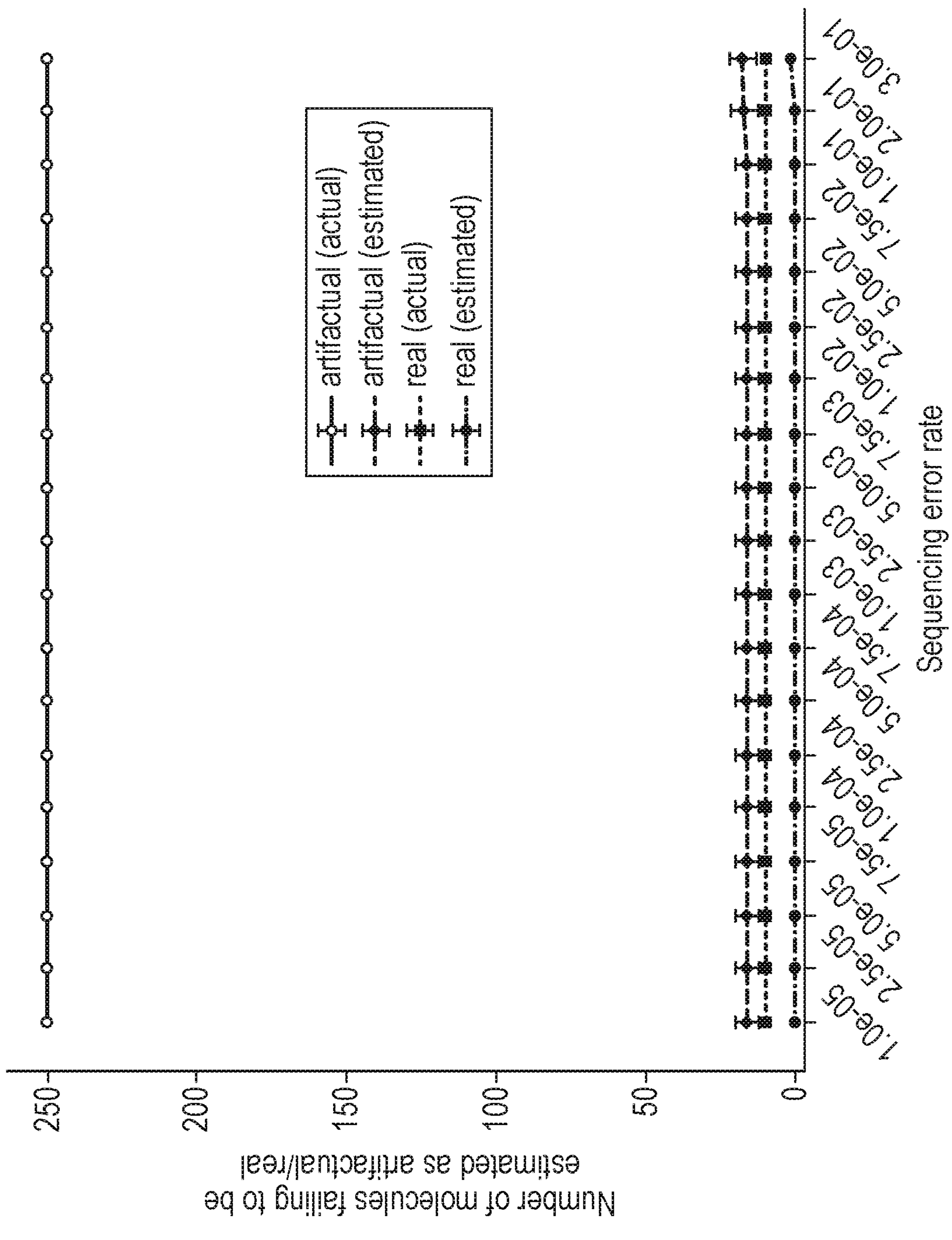
artifactual (actual)
artifactual (estimated)
real (actual)
real (estimated)
Number of molecules failing to be estimated as artifactual/real
0
50
100
150
200
250
Sequencing error rate
1.0e-05
2.5e-05
5.0e-05
7.5e-05
1.0e-04
2.5e-04
5.0e-04
7.5e-04
1.0e-03
2.5e-03
5.0e-03
7.5e-03
1.0e-02
2.5e-02
5.0e-02
7.5e-02
1.0e-01
2.0e-01
3.0e-01

Fig. 2a

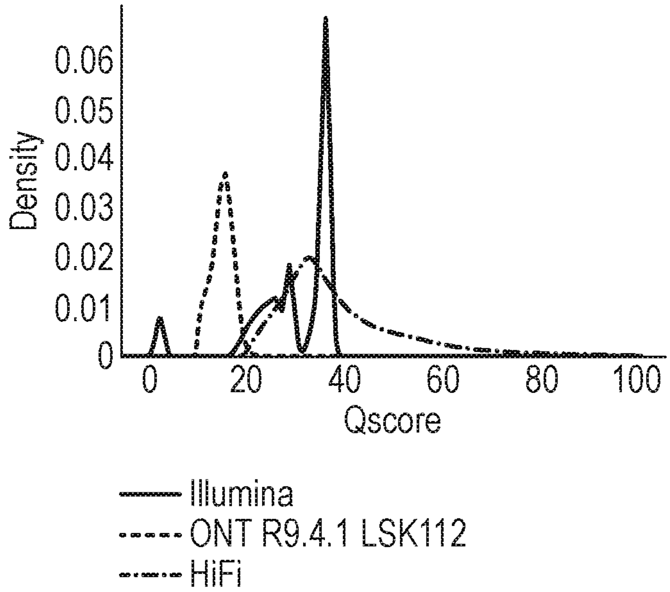


Fig. 2b

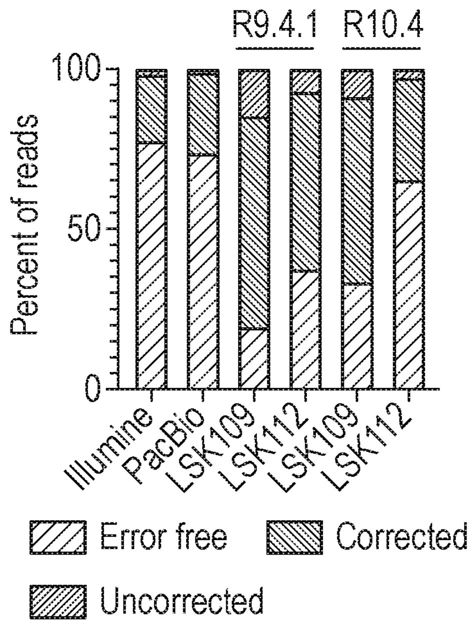


Fig. 2c

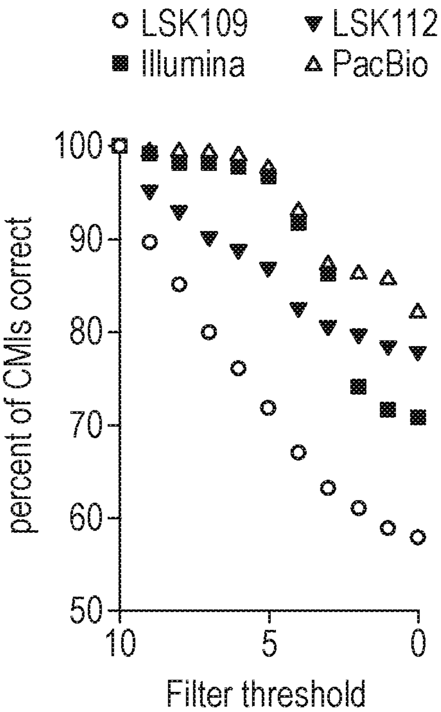

Fig. 2d

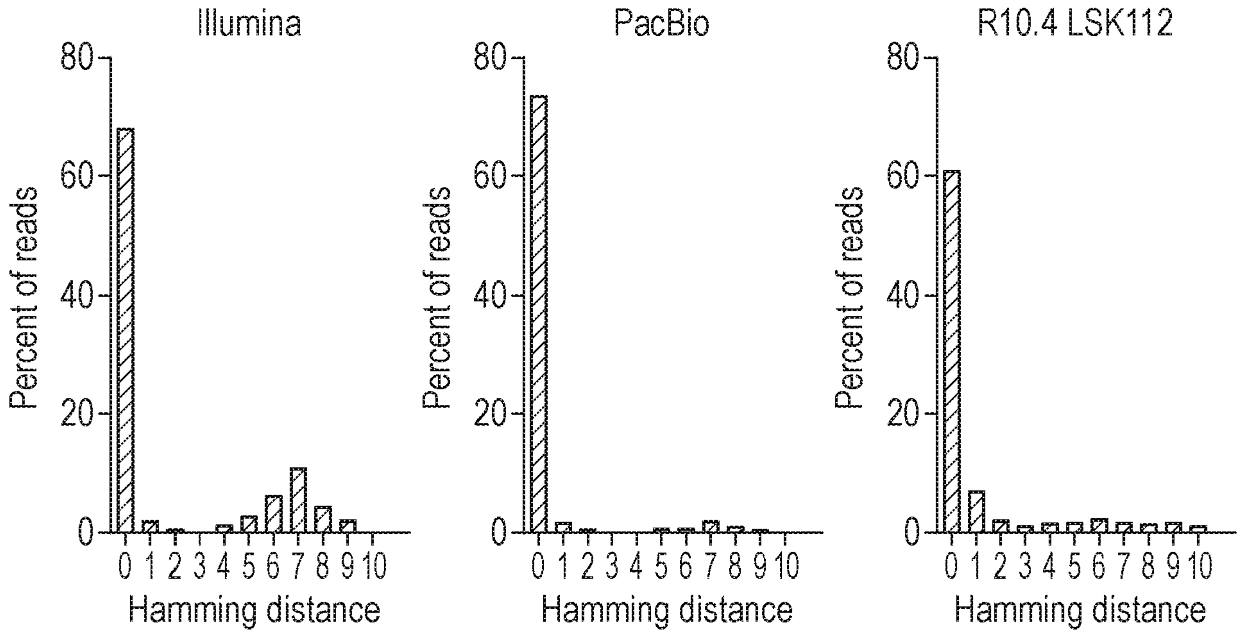


Fig. 2e

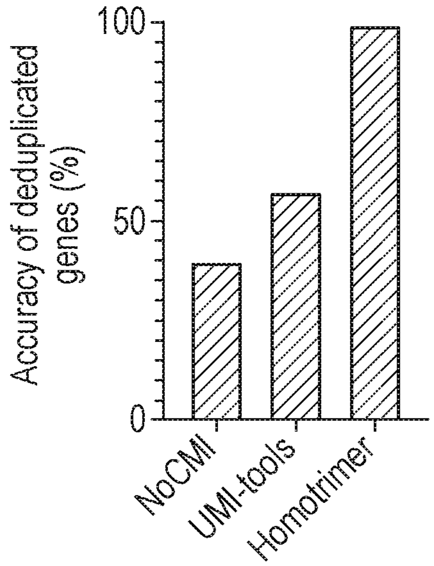


Fig. 2f

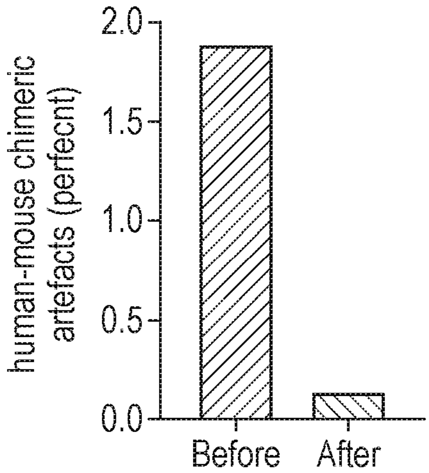

Fig. 3

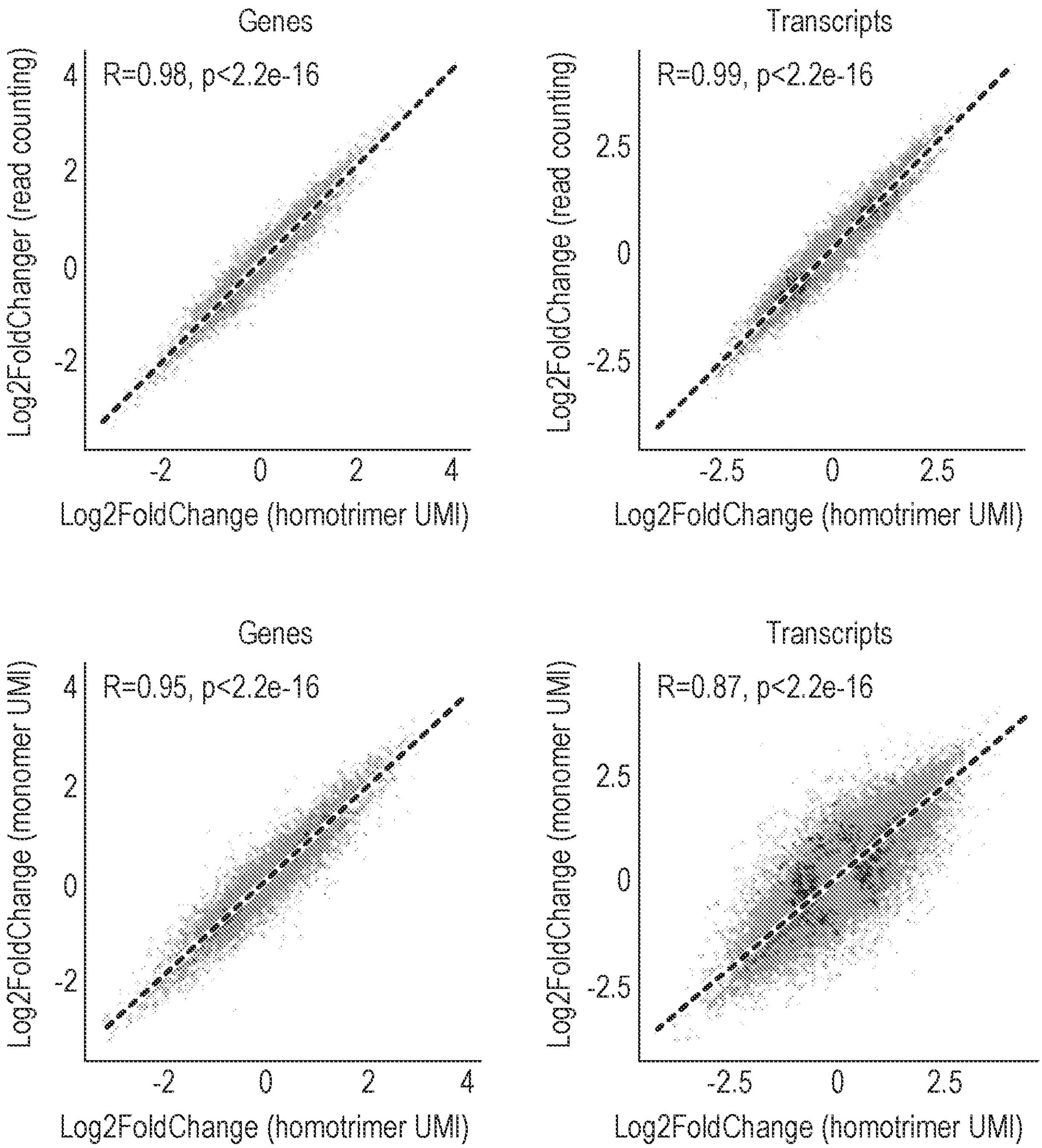
Genes
R=0.98, p<2.2e-16
Log2FoldChanger (read counting)
Log2FoldChange (homotrimer UMI)
4
2
0
-2
-2
0
2
4
Transcripts
R=0.99, p<2.2e-16
Log2FoldChange (read counting)
Log2FoldChange (homotrimer UMI)
2.5
0
-2.5
-2.5
0
2.5
Genes
R=0.95, p<2.2e-16
Log2FoldChange (monomer UMI)
Log2FoldChange (homotrimer UMI)
4
2
0
-2
-2
0
2
4
Transcripts
R=0.87, p<2.2e-16
Log2FoldChange (monomer UMI)
Log2FoldChange (homotrimer UMI)
2.5
0
-2.5
-2.5
0
2.5

Fig. 4a

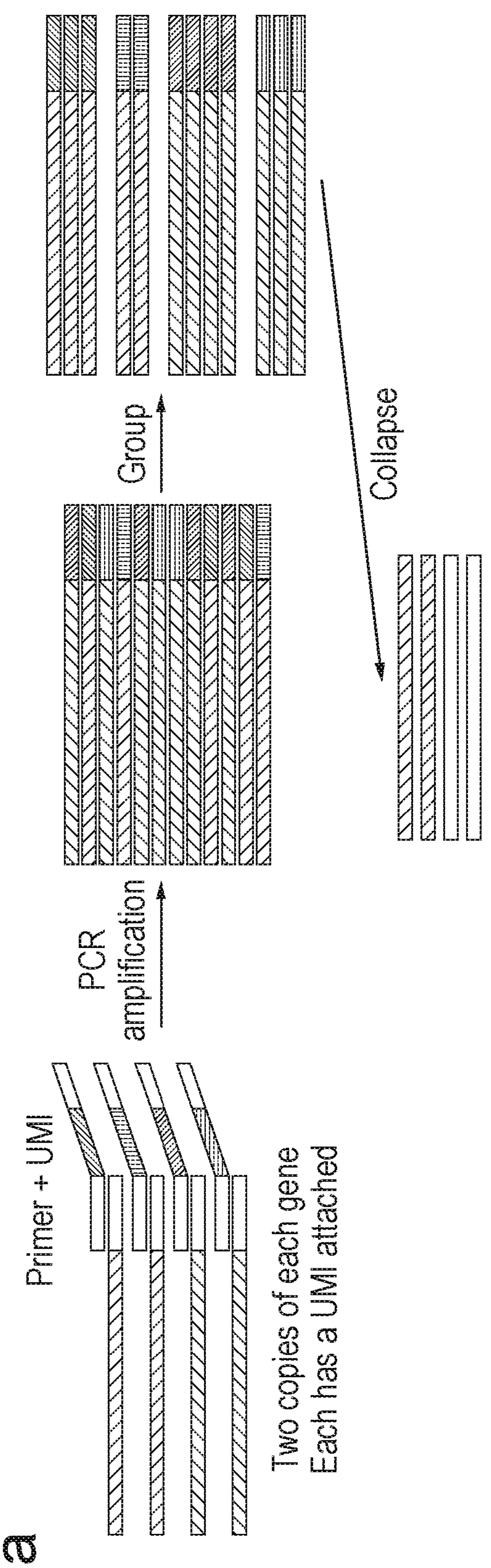
Primer + UMI
Two copies of each gene
Each has a UMI attached
PCR amplification
Group
Collapse

Fig. 4b

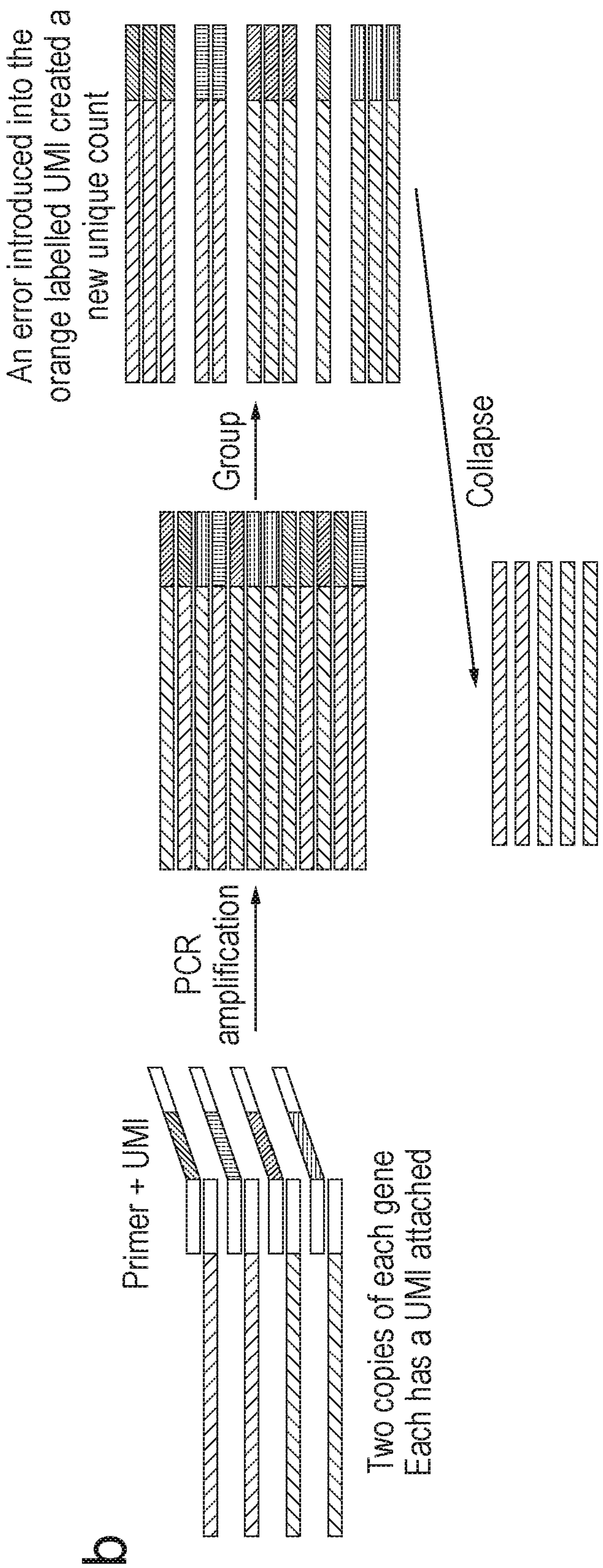
Primer + UMI
Two copies of each gene
Each has a UMI attached
PCR amplification
Group
An error introduced into the orange labelled UMI created a new unique count
Collapse

Fig. 5

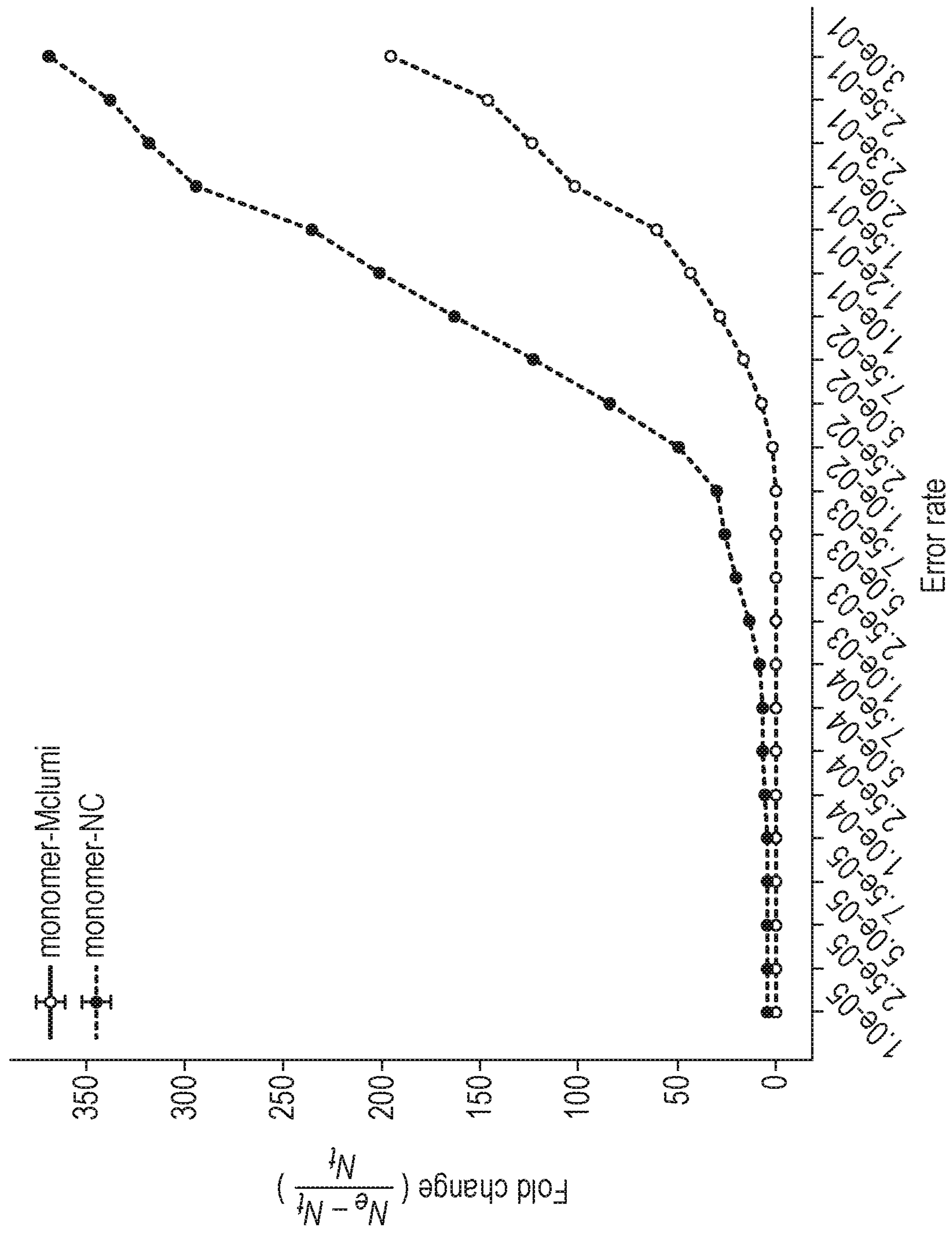
monomer-Mclumi
monomer-NC
Fold change ($\frac{N_e - N_t}{N_t}$)
350
300
250
200
150
100
50
0
Error rate
1.0e-05
2.5e-05
5.0e-05
7.5e-05
1.0e-04
2.5e-04
5.0e-04
7.5e-04
1.0e-03
2.5e-03
5.0e-03
7.5e-03
1.0e-02
2.5e-02
5.0e-02
7.5e-02
1.0e-01
1.2e-01
1.5e-01
2.0e-01
2.3e-01
2.5e-01
3.0e-01

Fig. 6a

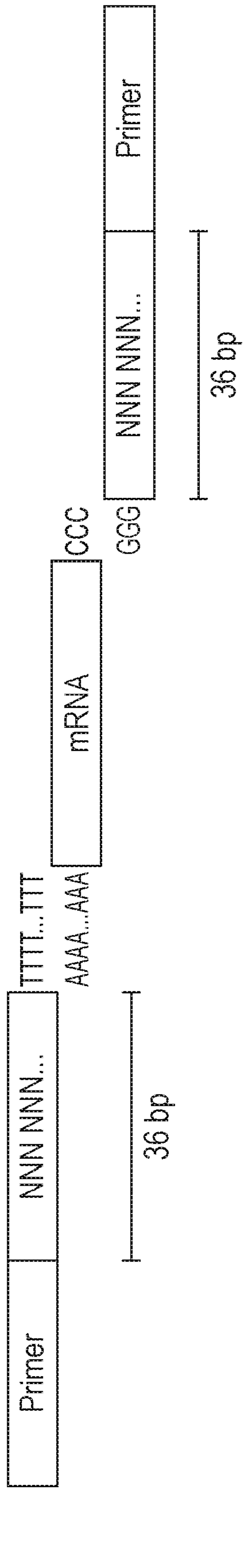
Primer
NNN NNN...
36 bp
TTTT...TTT
AAAA...AAA
mRNA
CCC
GGG
NNN NNN...
36 bp
Primer

Fig. 6b

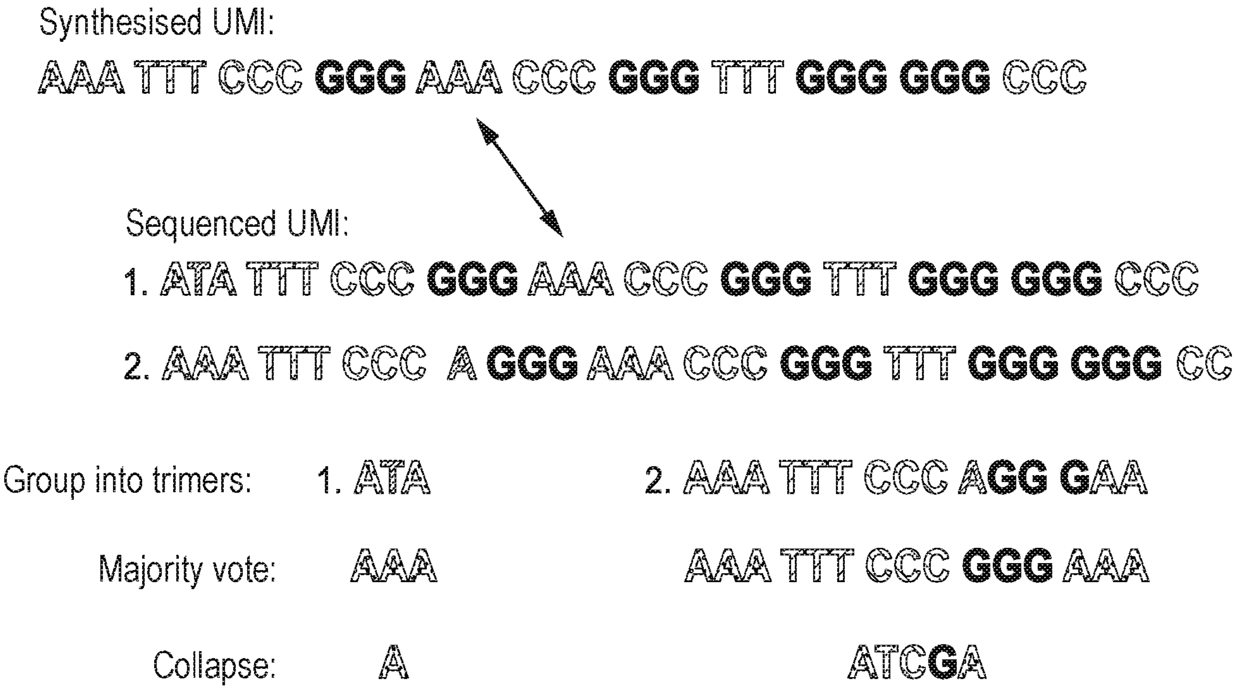
Synthesised UMI:
AAA TTT CCC GGG AAA CCC GGG TTT GGG GGG CCC
Sequenced UMI:
1. ATA TTT CCC GGG AAA CCC GGG TTT GGG GGG CCC
2. AAA TTT CCC A GGG AAA CCC GGG TTT GGG GGG CC
Group into trimers:
1. ATA
2. AAA TTT CCC AGG GAA
Majority vote:
AAA
AAA TTT CCC GGG AAA
Collapse:
A
ATCGA

Fig. 6c

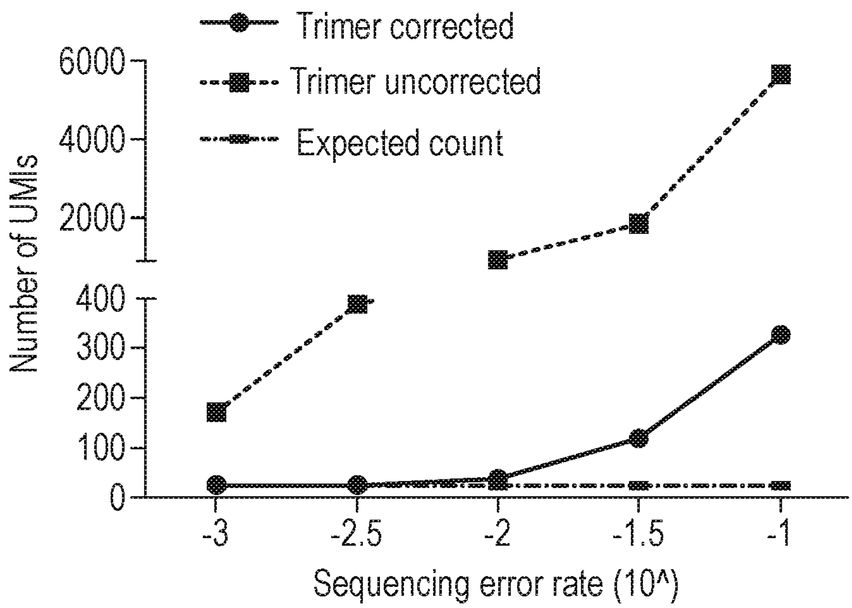
Trimer corrected
Trimer uncorrected
Expected count
Number of UMIs
6000
4000
2000
400
300
200
100
0
-3
-2.5
-2
-1.5
-1
Sequencing error rate (10^)

Fig. 7

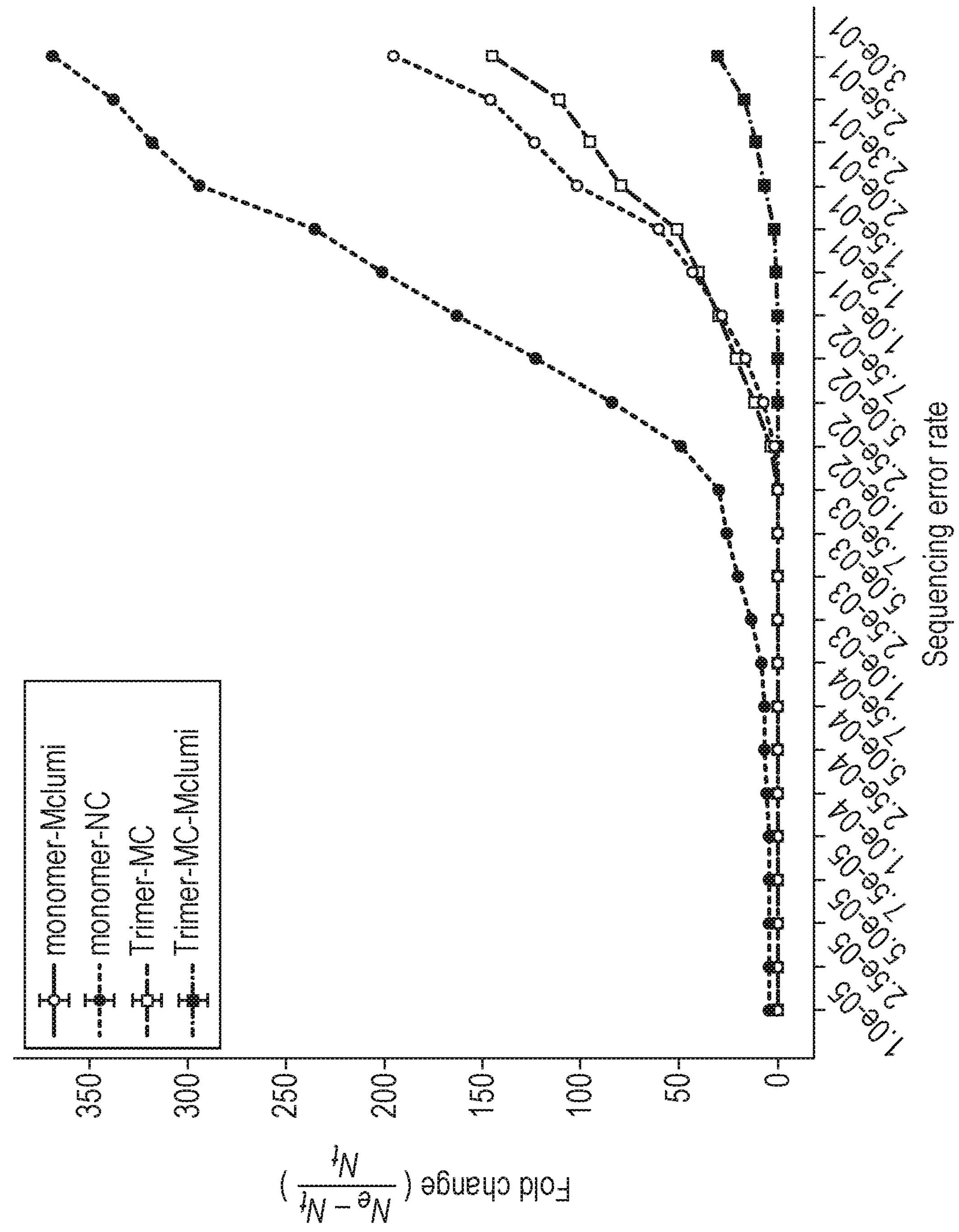
monomer-Mclumi
monomer-NC
Trimer-MC
Trimer-MC-Mclumi
Fold change ( $\frac{N_e - N_t}{N_t}$ )
Sequencing error rate
0
50
100
150
200
250
300
350
1.0e-05
2.5e-05
5.0e-05
7.5e-05
1.0e-04
2.5e-04
5.0e-04
7.5e-04
1.0e-03
2.5e-03
5.0e-03
7.5e-03
1.0e-02
2.5e-02
5.0e-02
7.5e-02
1.0e-01
1.2e-01
1.5e-01
2.0e-01
2.3e-01
2.5e-01
3.0e-01

# Fig. 8

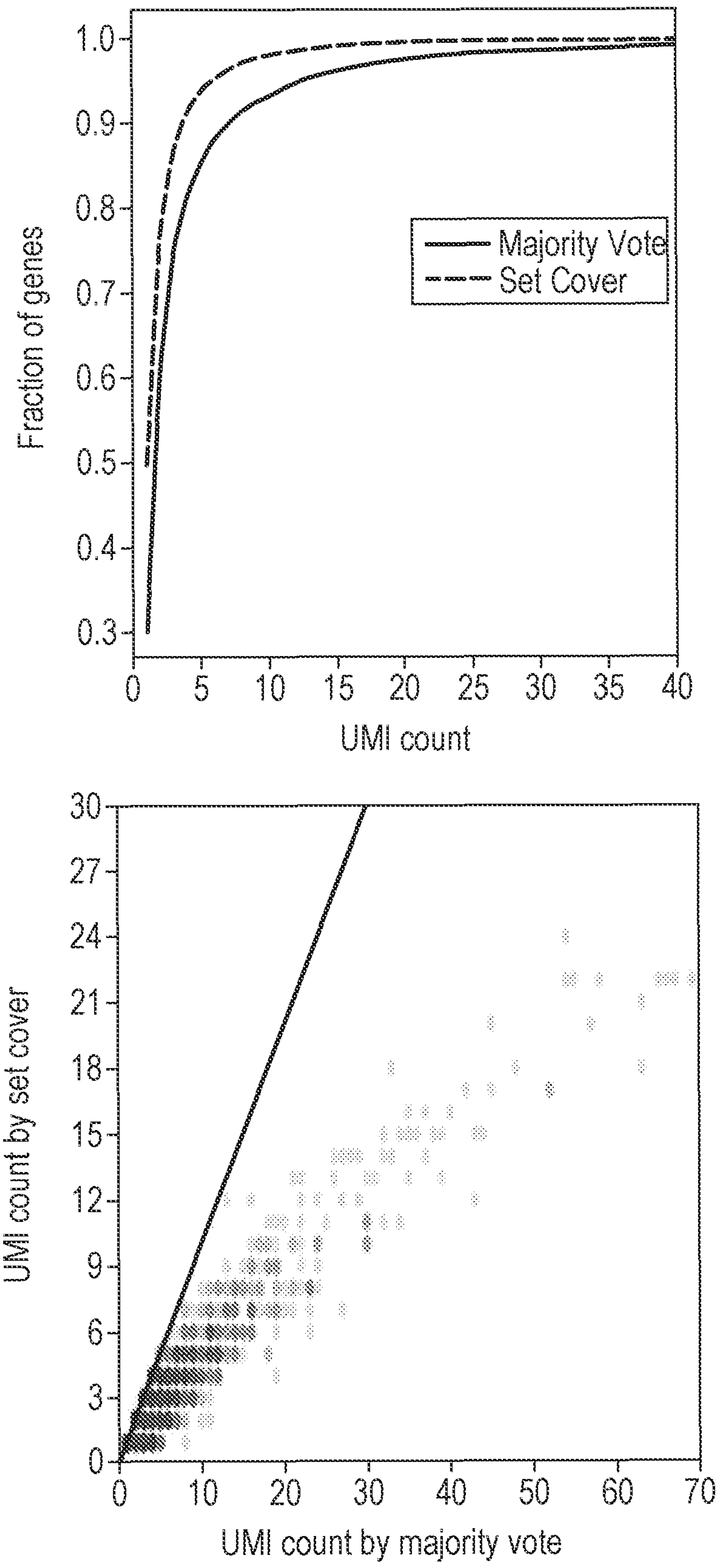
Fraction of genes
1.0
0.9
0.8
0.7
0.6
0.5
0.4
0.3
Majority Vote
Set Cover
0
5
10
15
20
25
30
35
40
UMI count
UMI count by set cover
30
27
24
21
18
15
12
9
6
3
0
0
10
20
30
40
50
60
70
UMI count by majority vote

Fig. 9a

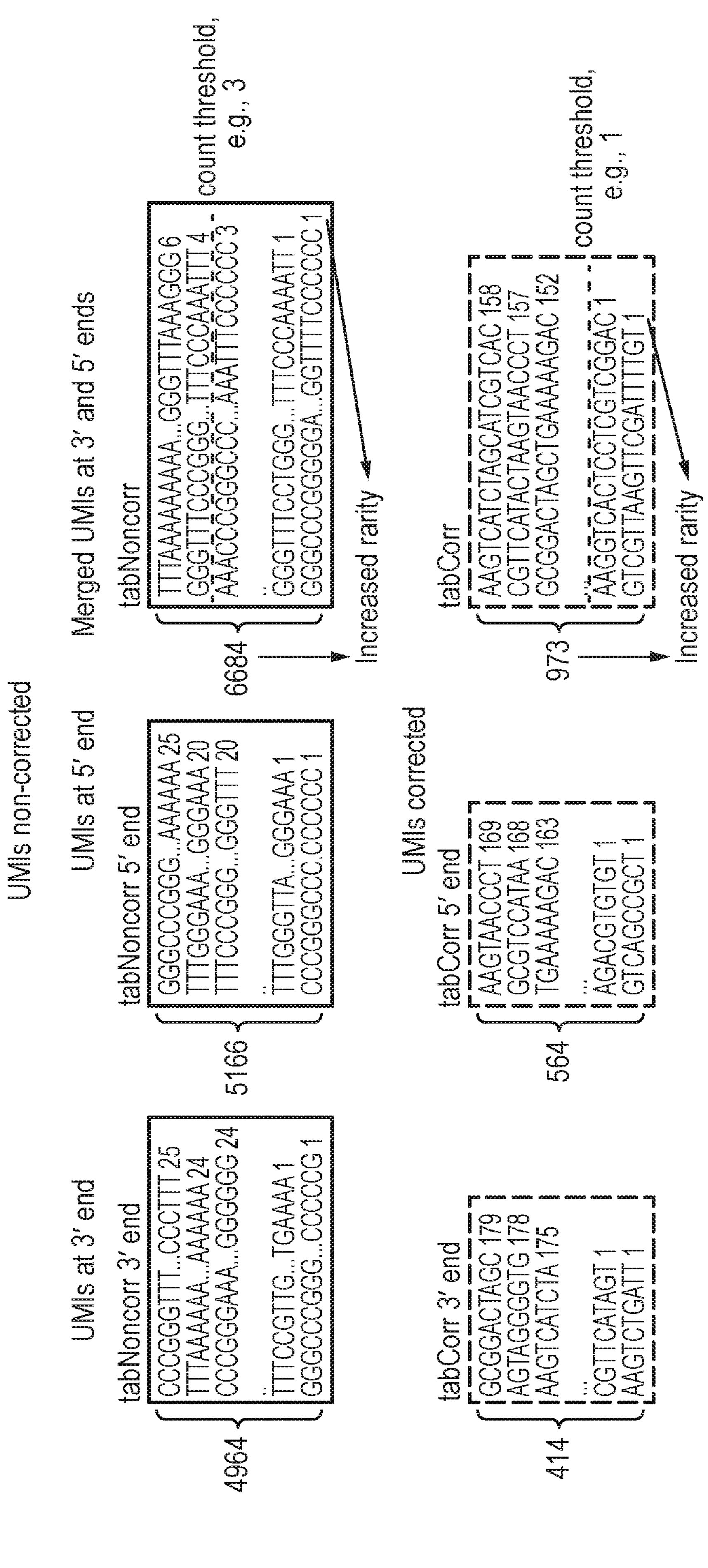
UMIs non-corrected
UMIs at 3′ end
tabNoncorr 3′ end
CCCGGGTTT...CCCTTT 25
TTTAAAAAA...AAAAAA 24
CCCGGGAAA...GGGGGG 24
..
TTTCCGTTG...TGAAAA 1
GGGCCCGGG...CCCCCG 1
4964
UMIs at 5′ end
tabNoncorr 5′ end
GGGCCCGGG...AAAAAA 25
TTTGGGAAA...GGGAAA 20
TTTCCCGGG...GGGTTT 20
..
TTTGGGTTA...GGGAAA 1
CCCGGGCCC.CCCCCC 1
5166
Merged UMIs at 3′ and 5′ ends
tabNoncorr
TTTAAAAAAAAA...GGGTTTAAAGGG 6
GGGTTTCCCGGG...TTTCCCAAATTT 4
AAACCCGGGCCC...AAATTTCCCCCC 3
..
GGGTTTCCTGGG...TTTCCCAAAATT 1
GGGCCCGGGGGA...GGTTTTCCCCCC 1
6684
Increased rarity
count threshold, e.g., 3
UMIs corrected
tabCorr 3′ end
GCGGACTAGC 179
AGTAGGGGTG 178
AAGTCATCTA 175
...
CGTTCATAGT 1
AAGTCTGATT 1
414
tabCorr 5′ end
AAGTAACCCT 169
GCGTCCATAA 168
TGAAAAAGAC 163
...
AGACGTGTGT 1
GTCAGCCGCT 1
564
tabCorr
AAGTCATCTAGCATCGTCAC 158
CGTTCATACTAAGTAACCCT 157
GCGGACTAGCTGAAAAAGAC 152
...
AAGGTCACTCCTCGTCGGAC 1
GTCGTTAAGTTCGATTTTGT 1
973
Increased rarity
count threshold, e.g., 1

Fig. 9b

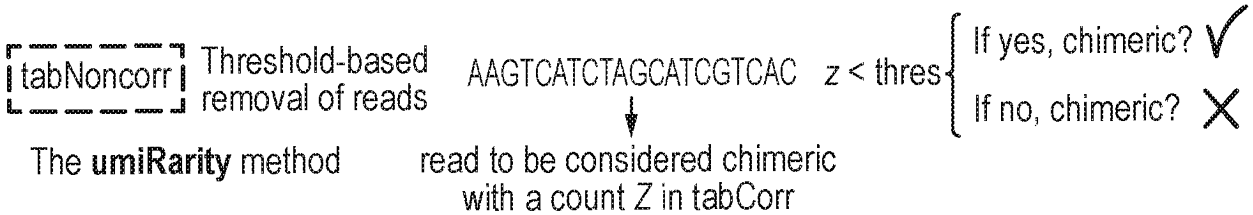
tabNoncorr
Threshold-based removal of reads
The **umiRarity** method
AAGTCATCTAGCATCGTCAC
read to be considered chimeric with a count Z in tabCorr
z < thres
If yes, chimeric?
If no, chimeric?

Fig. 9c

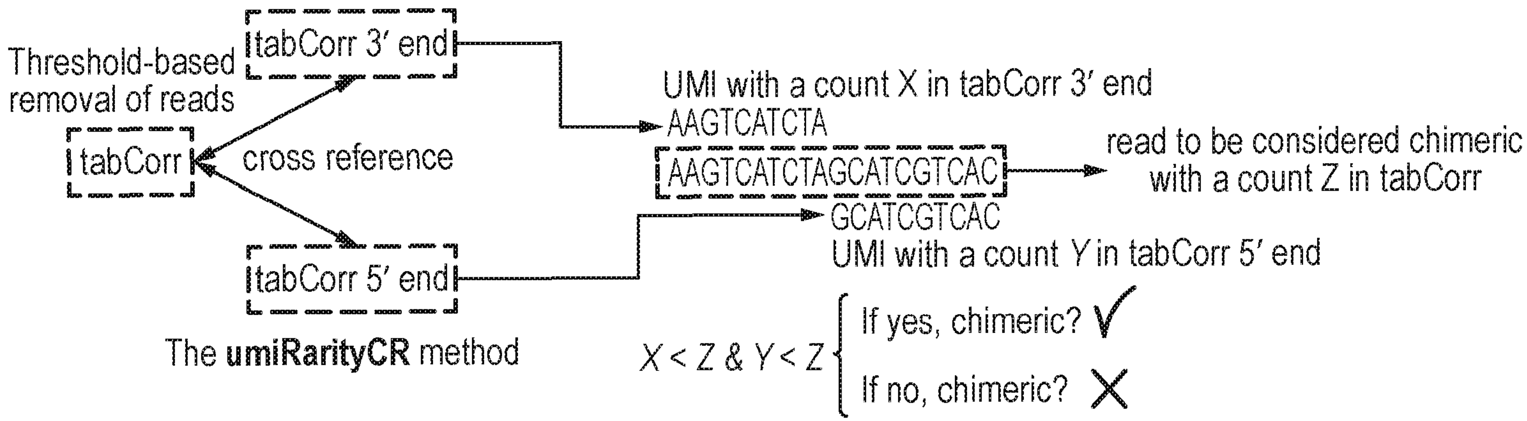
Threshold-based removal of reads
tabCorr
cross reference
tabCorr 3′ end
tabCorr 5′ end
The **umiRarityCR** method
UMI with a count X in tabCorr 3′ end
AAGTCATCTA
AAGTCATCTAGCATCGTCAC
GCATCGTCAC
UMI with a count Y in tabCorr 5′ end
read to be considered chimeric with a count Z in tabCorr
X < Z & Y < Z
If yes, chimeric?
If no, chimeric?

Fig. 10a

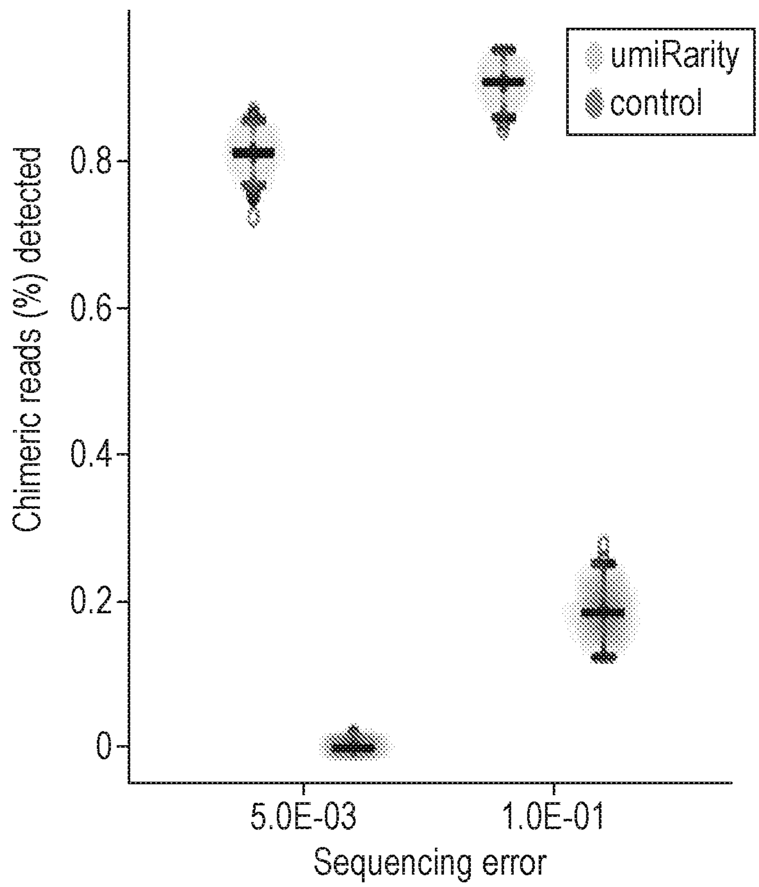
umiRarity
control
Chimeric reads (%) detected
0.8
0.6
0.4
0.2
0
5.0E-03
1.0E-01
Sequencing error

Fig. 10b

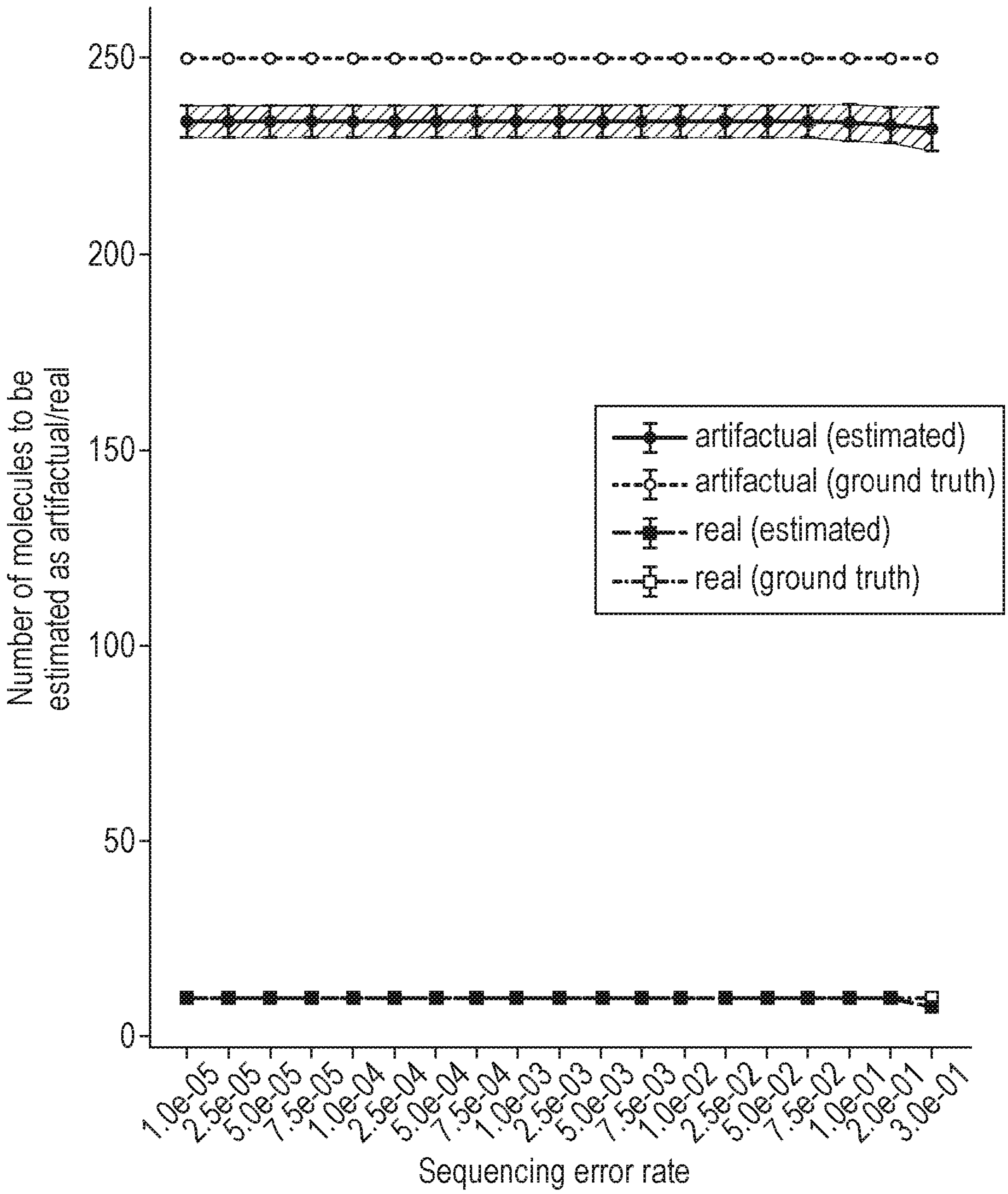
Number of molecules to be estimated as artifactual/real
250
200
150
100
50
0
artifactual (estimated)
artifactual (ground truth)
real (estimated)
real (ground truth)
1.0e-05
2.5e-05
5.0e-05
7.5e-05
1.0e-04
2.5e-04
5.0e-04
7.5e-04
1.0e-03
2.5e-03
5.0e-03
7.5e-03
1.0e-02
2.5e-02
5.0e-02
7.5e-02
1.0e-01
2.0e-01
3.0e-01
Sequencing error rate

Fig. 11a

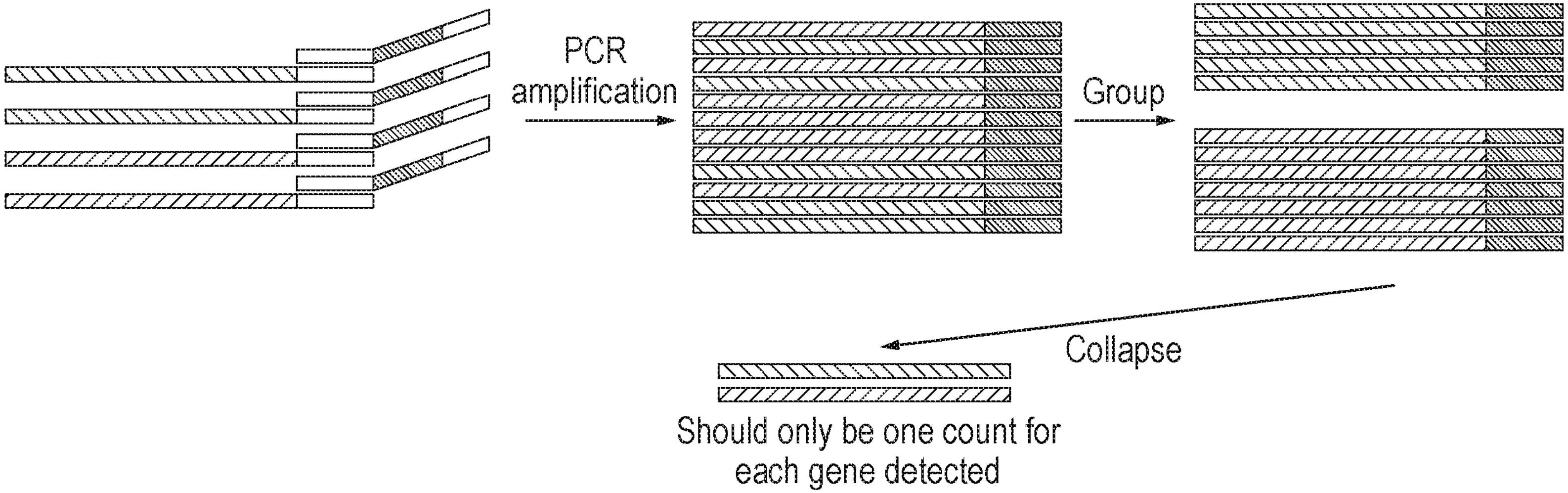


Fig. 11b

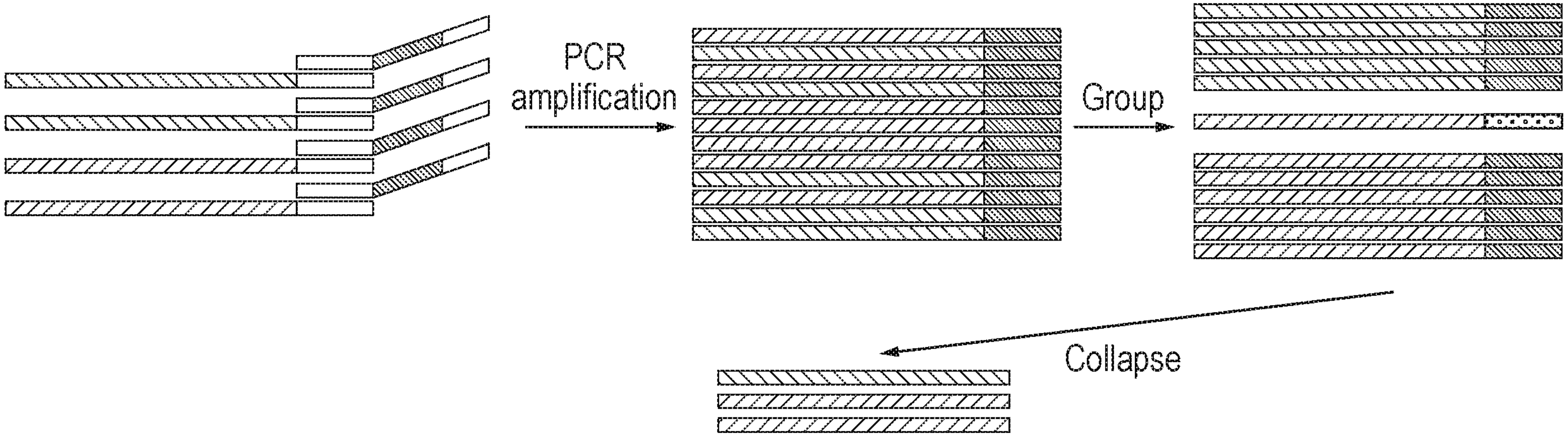

Fig. 12

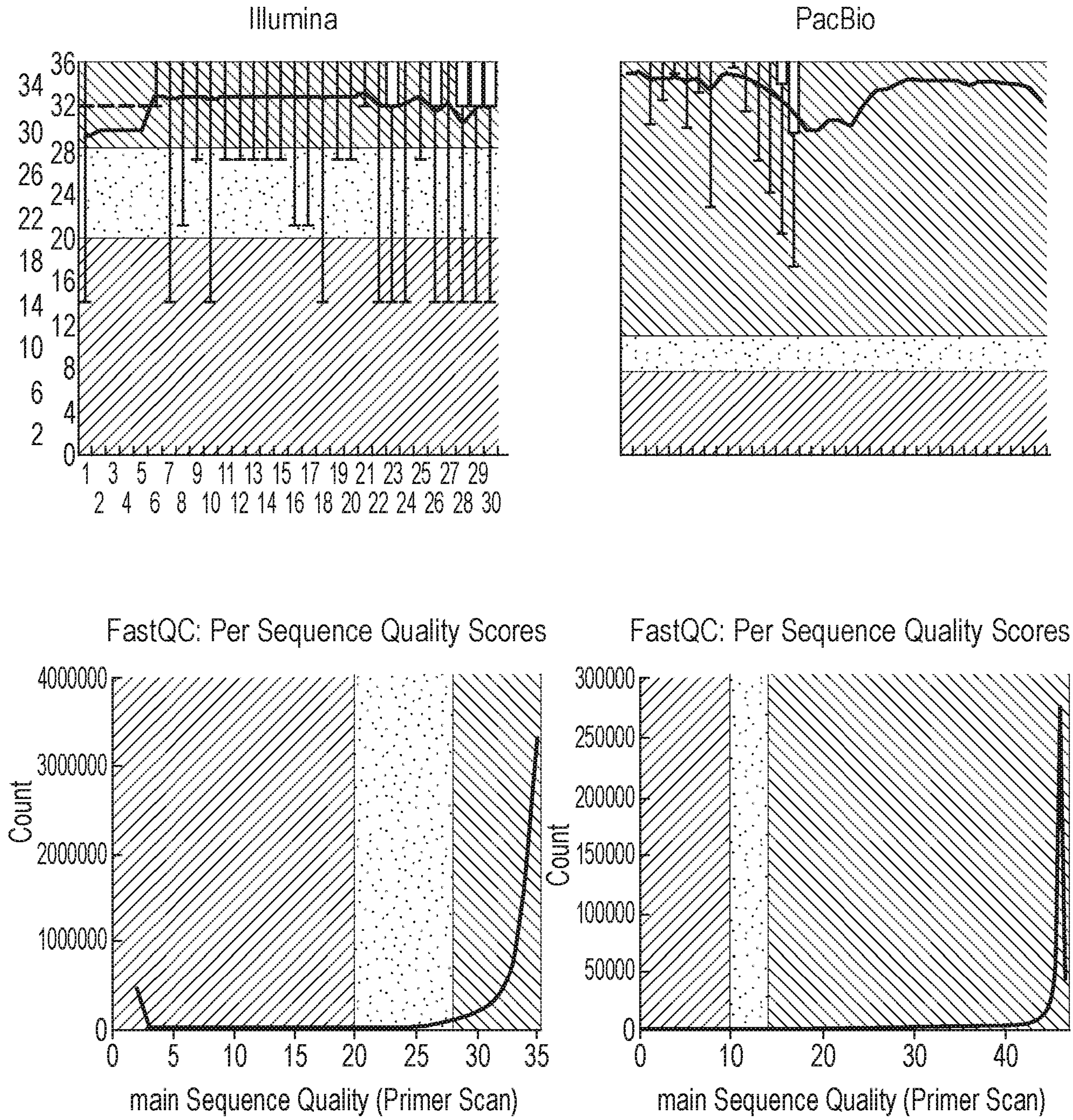
Illumina
PacBio
36
34
32
30
28
26
24
22
20
18
16
14
12
10
8
6
4
2
0
1 3 5 7 9 11 13 15 17 19 21 23 25 27 29
2 4 6 8 10 12 14 16 18 20 22 24 26 28 30
FastQC: Per Sequence Quality Scores
4000000
3000000
2000000
1000000
0
Count
0 5 10 15 20 25 30 35
main Sequence Quality (Primer Scan)
FastQC: Per Sequence Quality Scores
300000
250000
200000
150000
100000
50000
0
Count
0 10 20 30 40
main Sequence Quality (Primer Scan)

# Fig. 12 (Cont.)

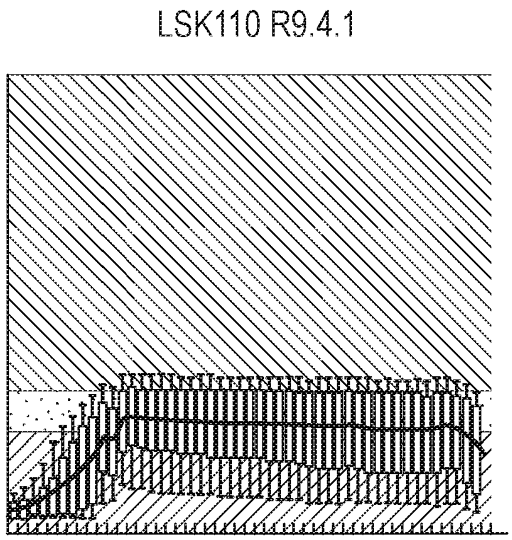
LSK110 R9.4.1

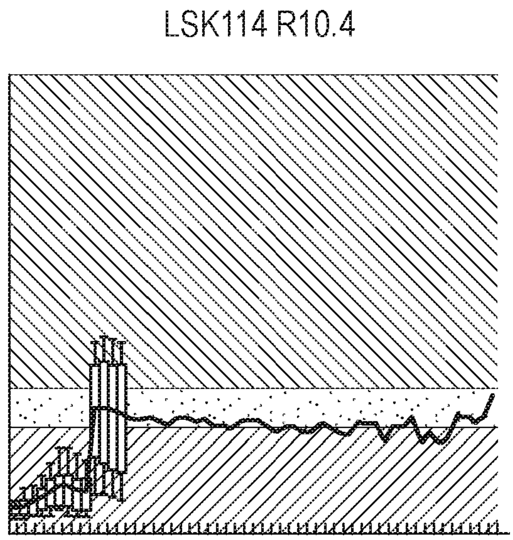
LSK114 R10.4

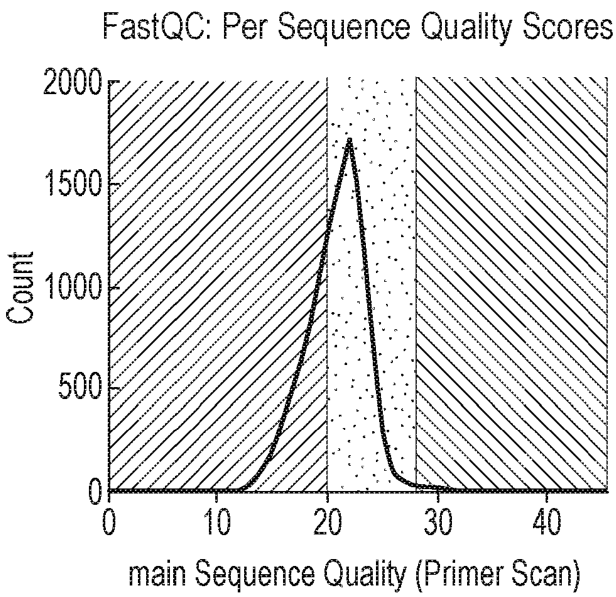
FastQC: Per Sequence Quality Scores
2000
1500
1000
500
0
Count
0
10
20
30
40
main Sequence Quality (Primer Scan)

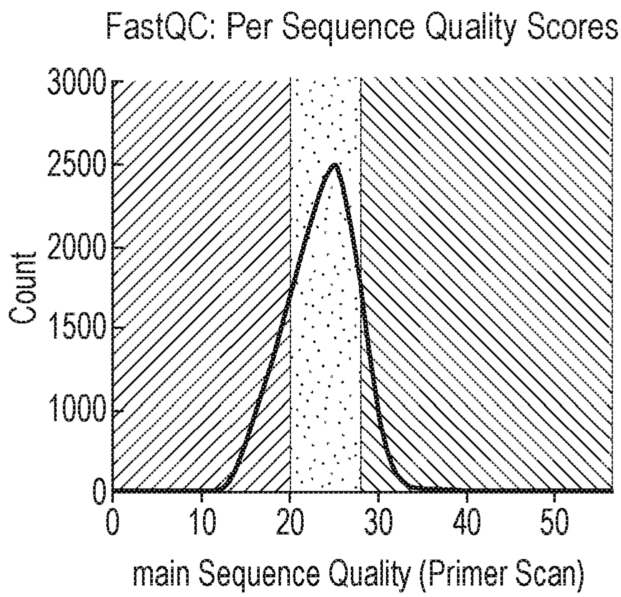
FastQC: Per Sequence Quality Scores
3000
2500
2000
1500
1000
0
Count
0
10
20
30
40
50
main Sequence Quality (Primer Scan)

# Fig. 13a

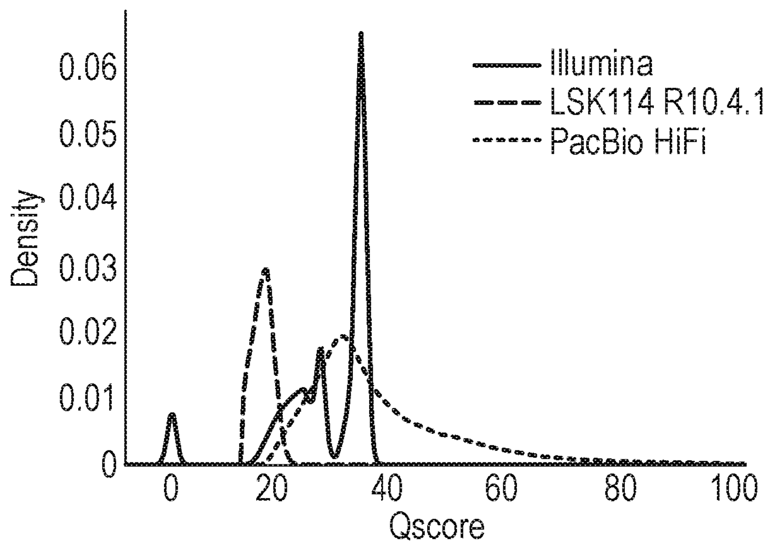


# Fig. 13b

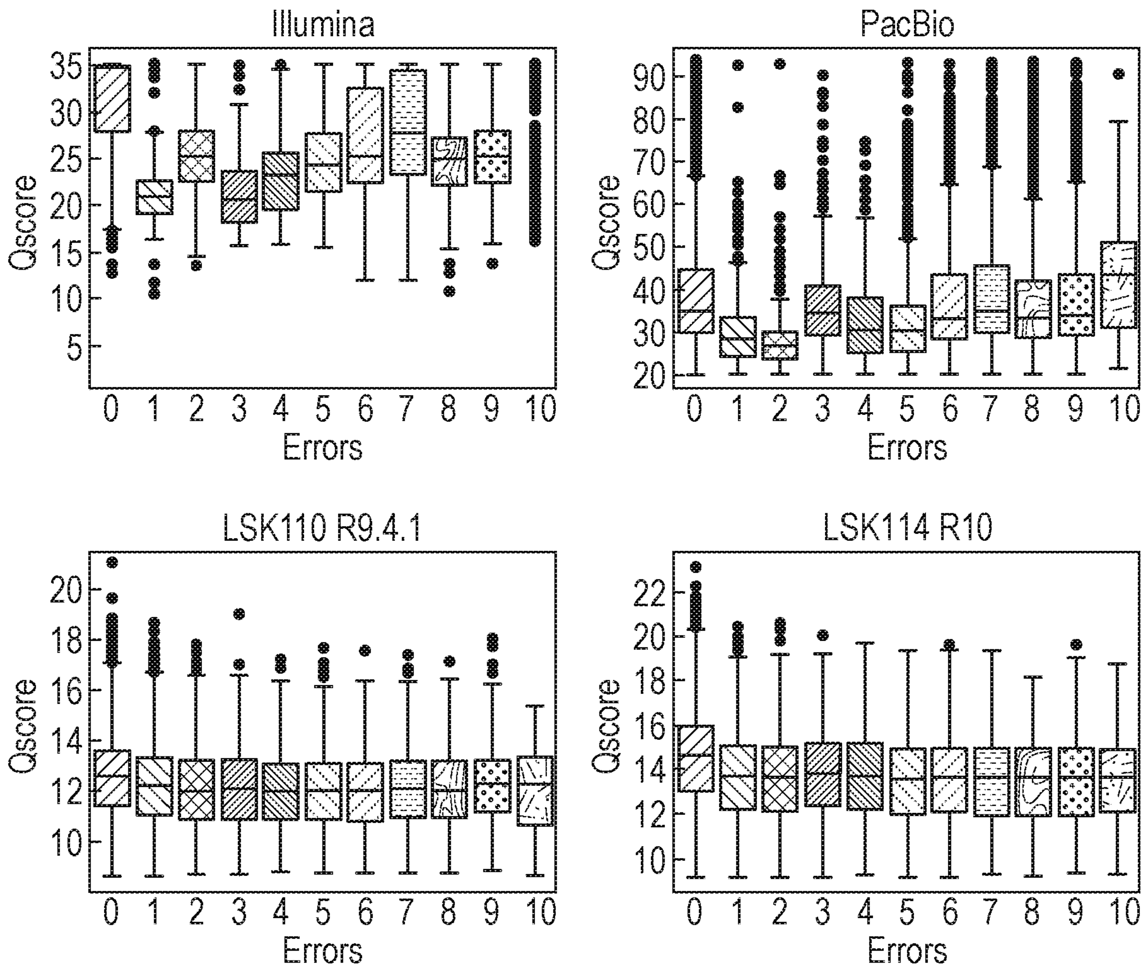

Fig. 14a

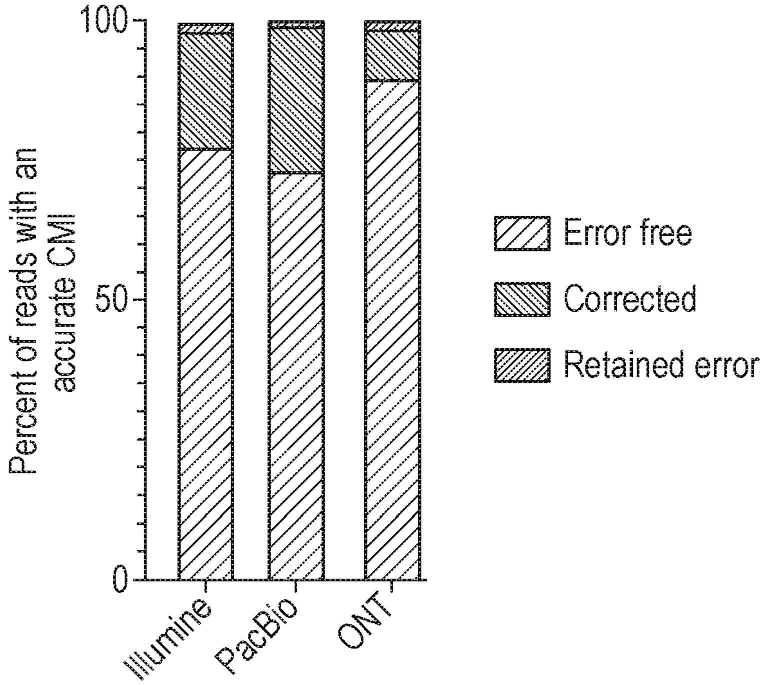


Fig. 14b

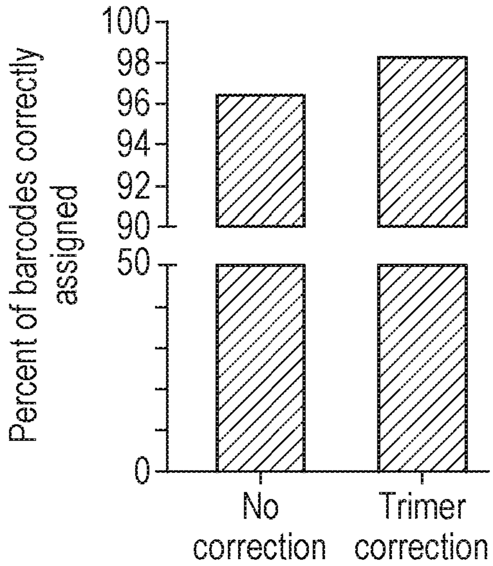

Fig. 15

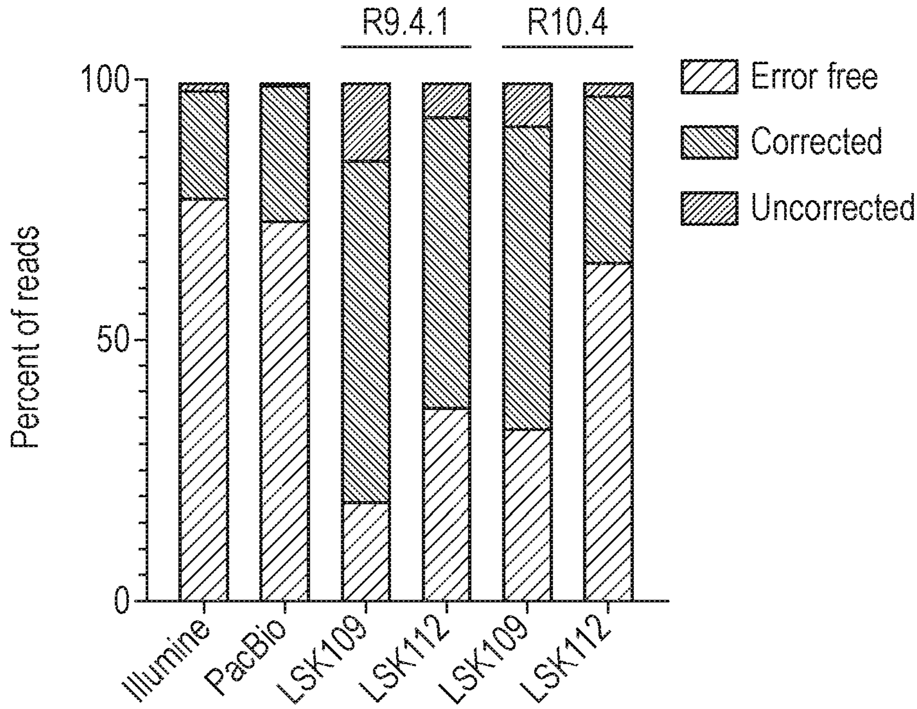

R9.4.1
R10.4
100
50
0
Percent of reads
Illumine
PacBio
LSK109
LSK112
LSK109
LSK112
Error free
Corrected
Uncorrected


Fig. 16

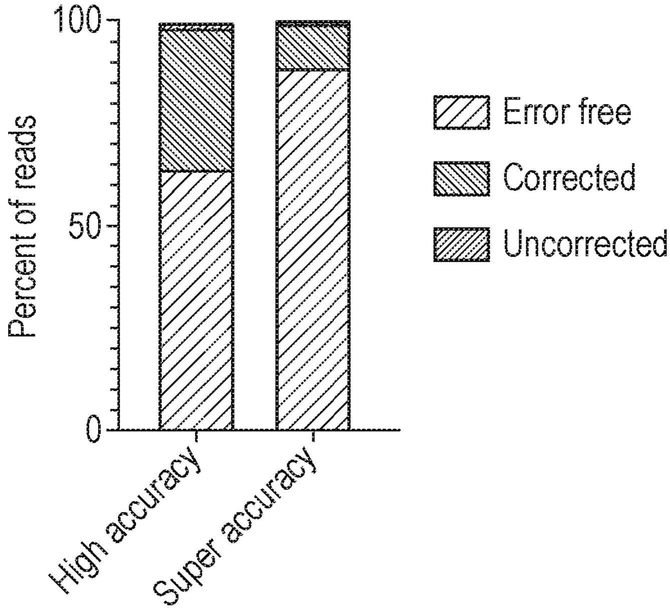

100
50
0
Percent of reads
High accuracy
Super accuracy
Error free
Corrected
Uncorrected

Fig. 17a

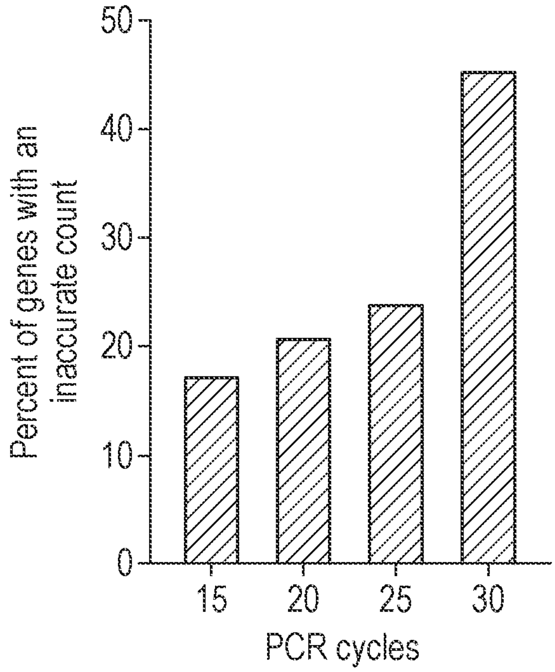


Fig. 17b

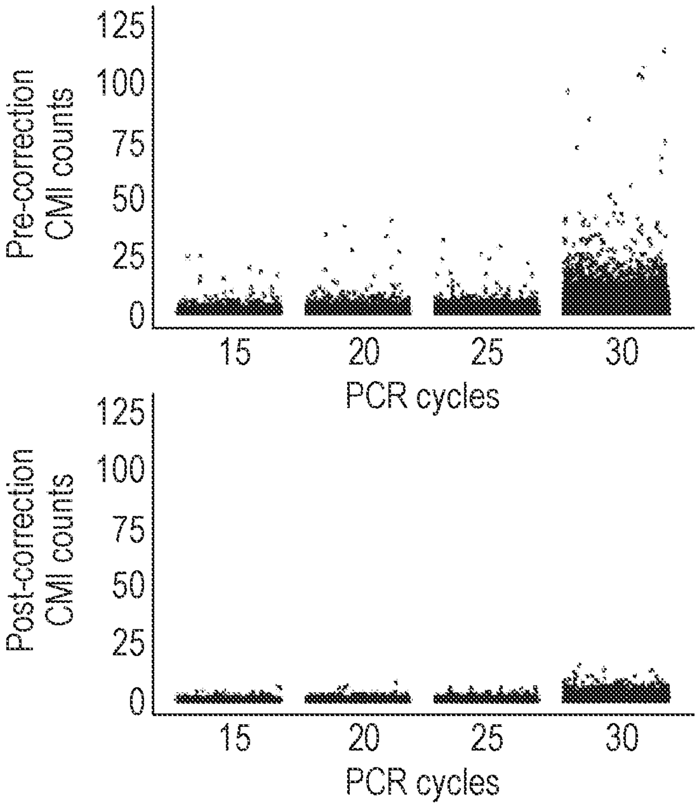

Fig. 18a

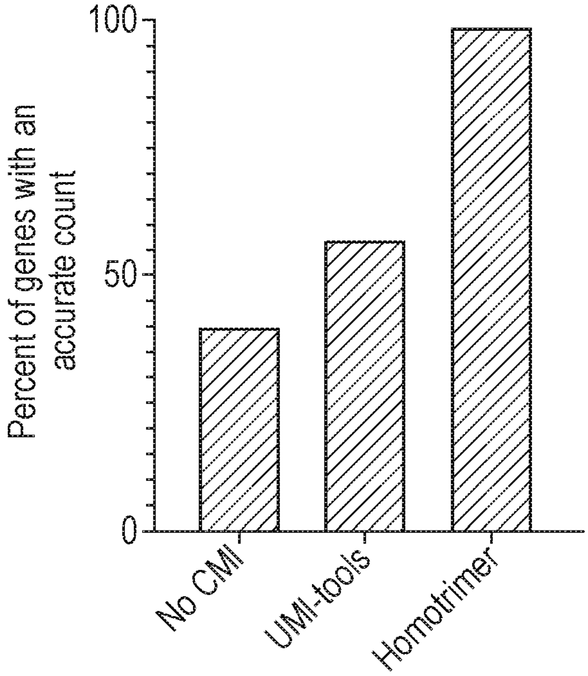


Fig. 18b

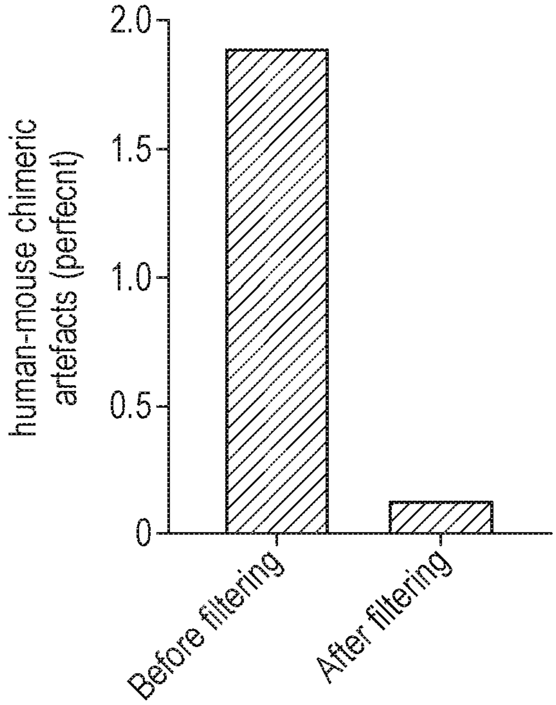

Fig. 19

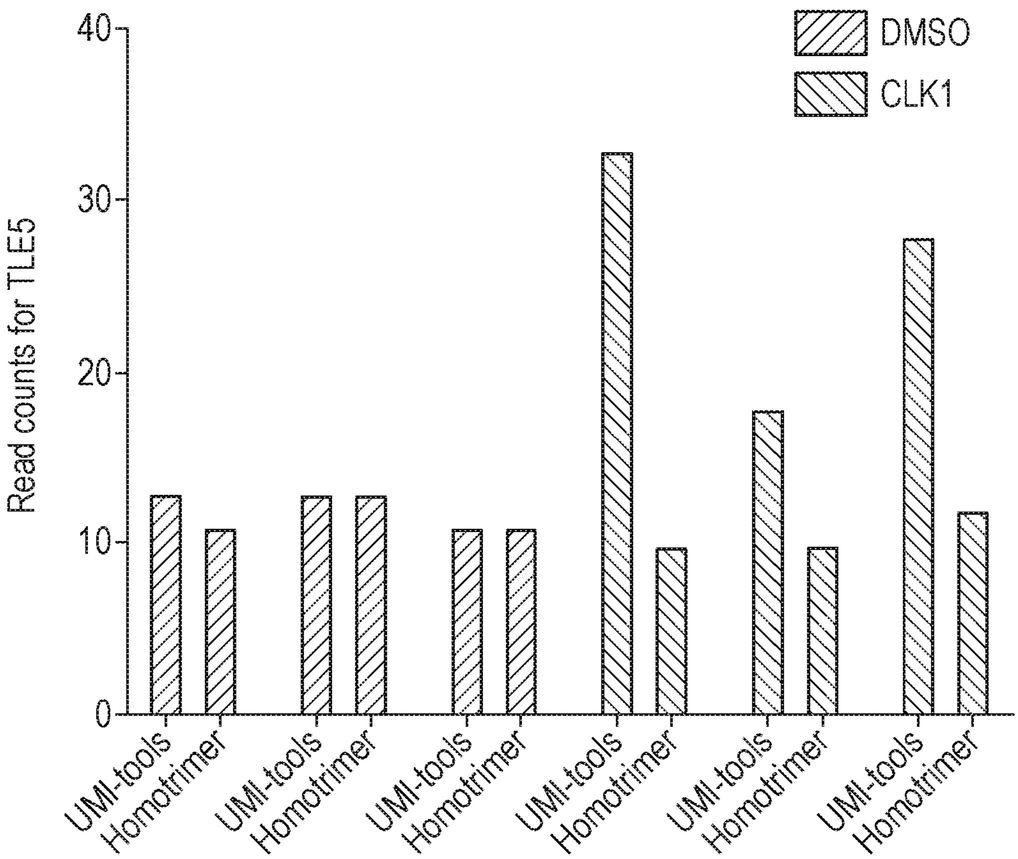
DMSO
CLK1
Read counts for TLE5
40
30
20
10
0
UMI-tools
Homotrimer
UMI-tools
Homotrimer
UMI-tools
Homotrimer
UMI-tools
Homotrimer
UMI-tools
Homotrimer
UMI-tools
Homotrimer

Fig. 20a

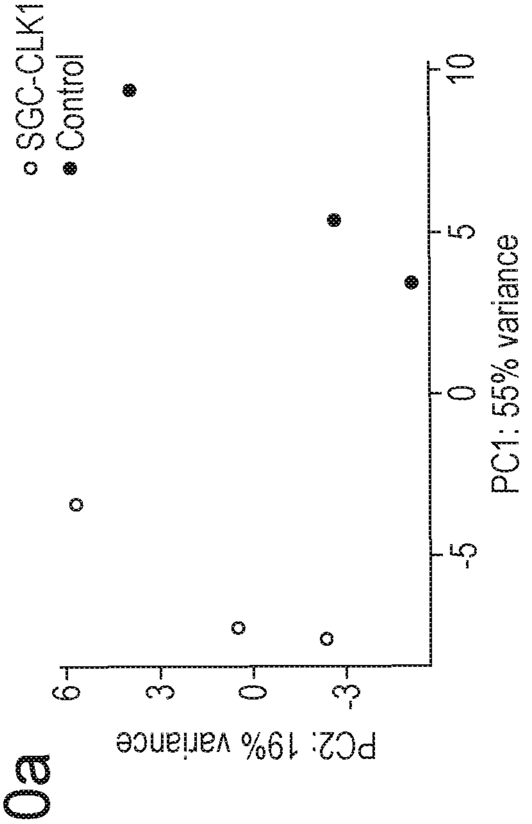


Fig. 20b

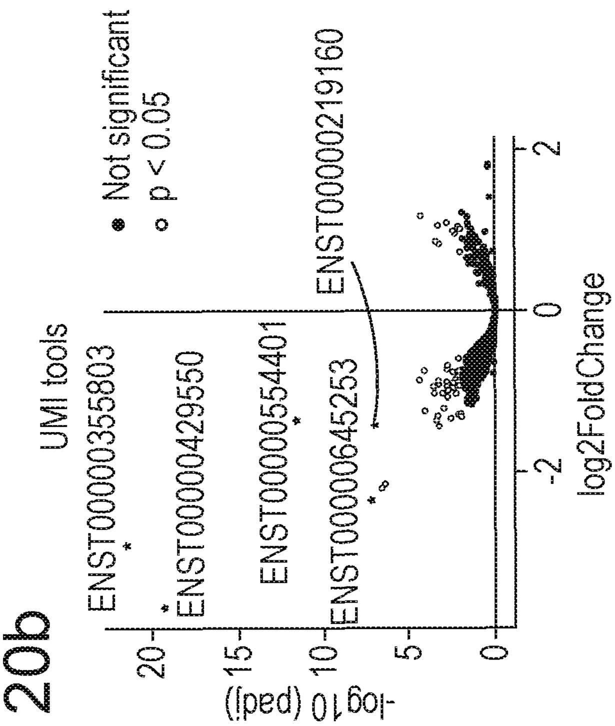


Fig. 20c

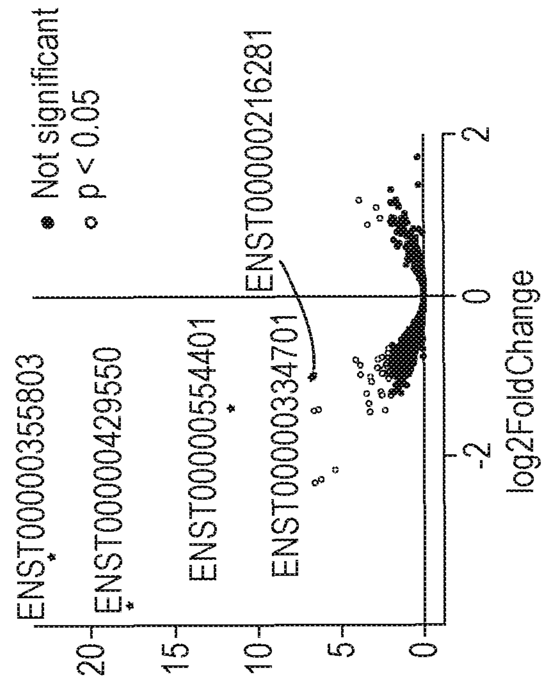

Fig. 21a

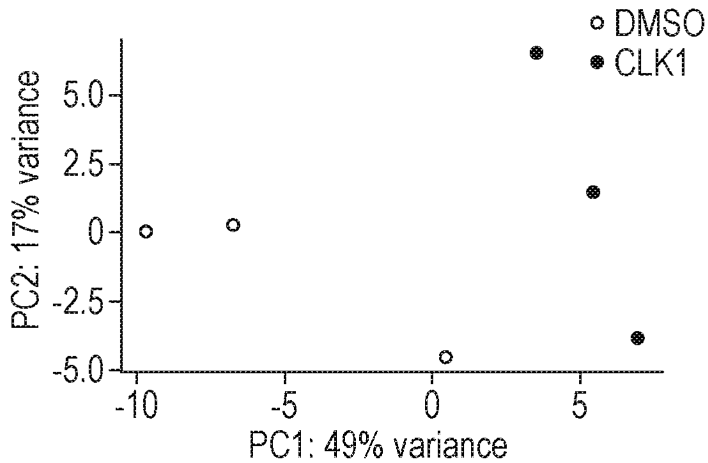


Fig. 21b

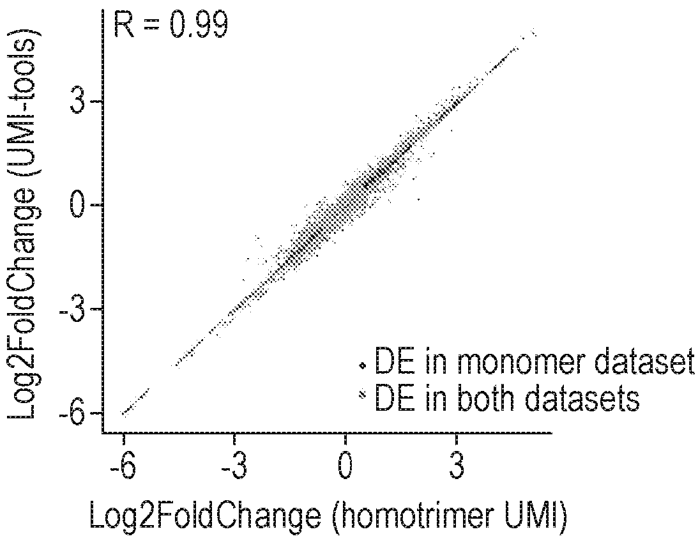

# Fig. 21c

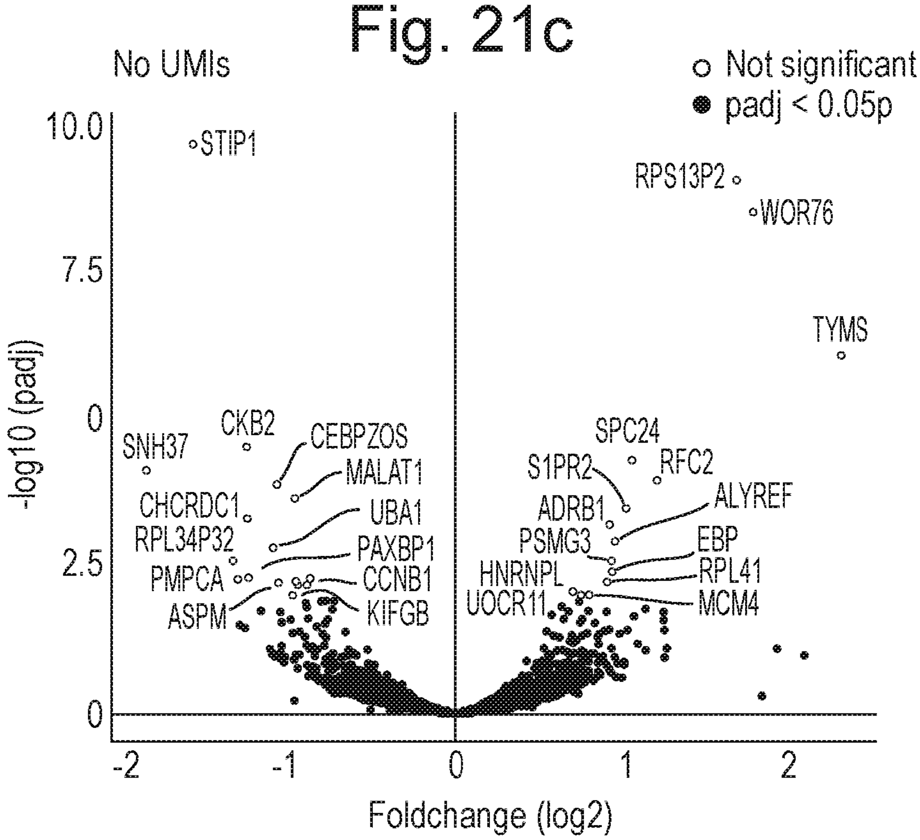


# Fig. 21d

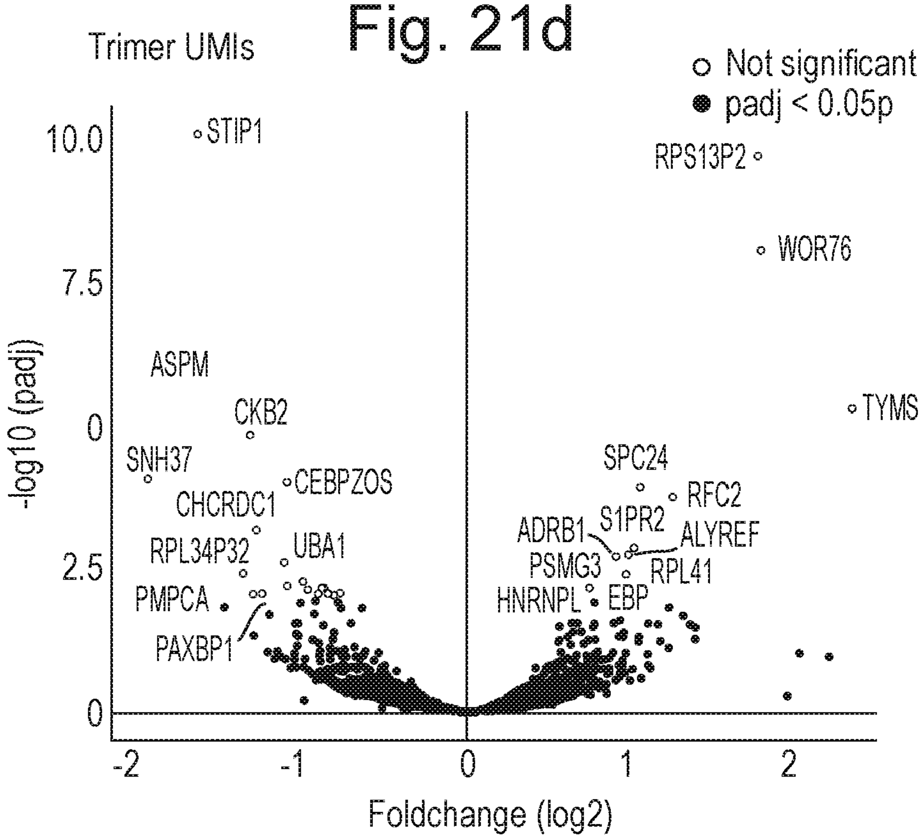

Fig. 22a

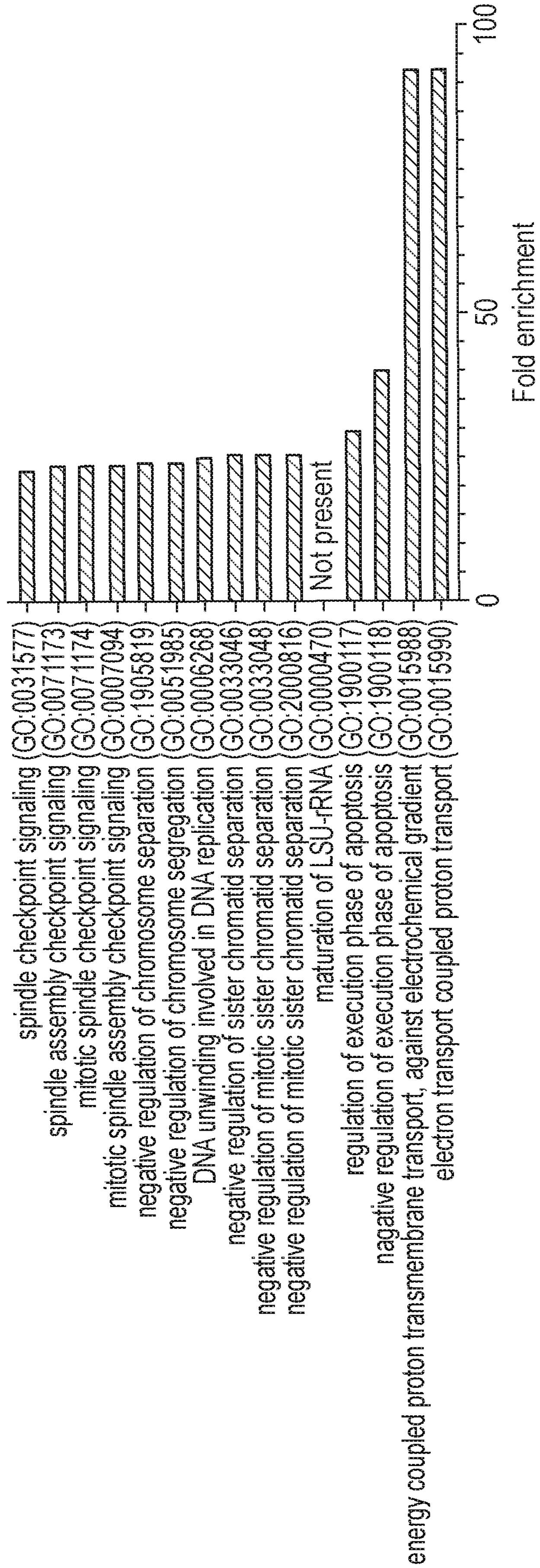
spindle checkpoint signaling (GO:0031577)
spindle assembly checkpoint signaling (GO:0071173)
mitotic spindle checkpoint signaling (GO:0071174)
mitotic spindle assembly checkpoint signaling (GO:0007094)
negative regulation of chromosome separation (GO:1905819)
negative regulation of chromosome segregation (GO:0051985)
DNA unwinding involved in DNA replication (GO:0006268)
negative regulation of sister chromatid separation (GO:0033046)
negative regulation of mitotic sister chromatid separation (GO:0033048)
negative regulation of mitotic sister chromatid separation (GO:2000816)
maturation of LSU-rRNA (GO:0000470)
regulation of execution phase of apoptosis (GO:1900117)
nagative regulation of execution phase of apoptosis (GO:1900118)
energy coupled proton transmembrane transport, against electrochemical gradient (GO:0015988)
electron transport coupled proton transport (GO:0015990)
Not present
0
50
100
Fold enrichment

Fig. 22b

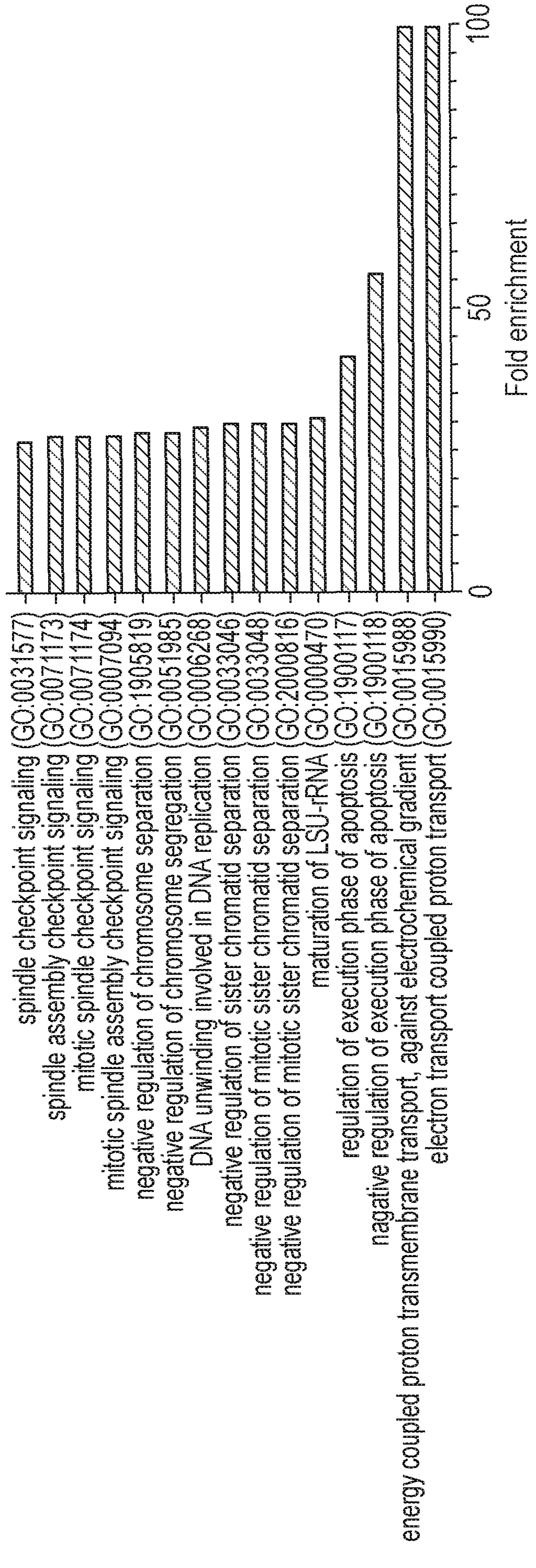
spindle checkpoint signaling (GO:0031577)
spindle assembly checkpoint signaling (GO:0071173)
mitotic spindle checkpoint signaling (GO:0071174)
mitotic spindle assembly checkpoint signaling (GO:0007094)
negative regulation of chromosome separation (GO:1905819)
negative regulation of chromosome segregation (GO:0051985)
DNA unwinding involved in DNA replication (GO:0006268)
negative regulation of sister chromatid separation (GO:0033046)
negative regulation of mitotic sister chromatid separation (GO:0033048)
negative regulation of mitotic sister chromatid separation (GO:2000816)
maturation of LSU-rRNA (GO:0000470)
regulation of execution phase of apoptosis (GO:1900117)
nagative regulation of execution phase of apoptosis (GO:1900118)
energy coupled proton transmembrane transport, against electrochemical gradient (GO:0015988)
electron transport coupled proton transport (GO:0015990)
0
50
100
Fold enrichment

Fig. 23

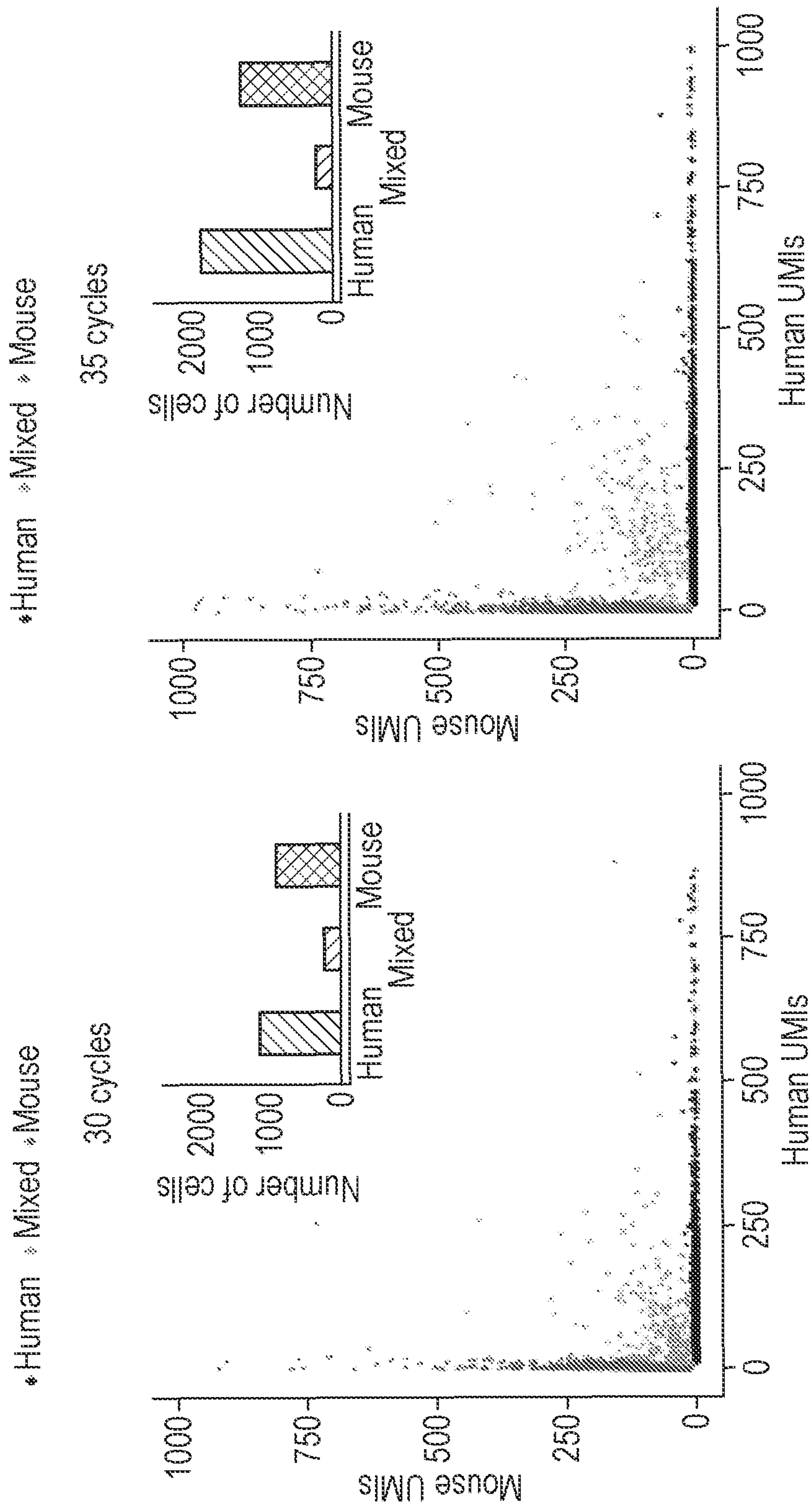
Human
Mixed
Mouse
30 cycles
35 cycles
Number of cells
2000
1000
0
Human
Mixed
Mouse
Mouse UMIs
1000
750
500
250
0
250
500
750
1000
Human UMIs

Fig. 24a

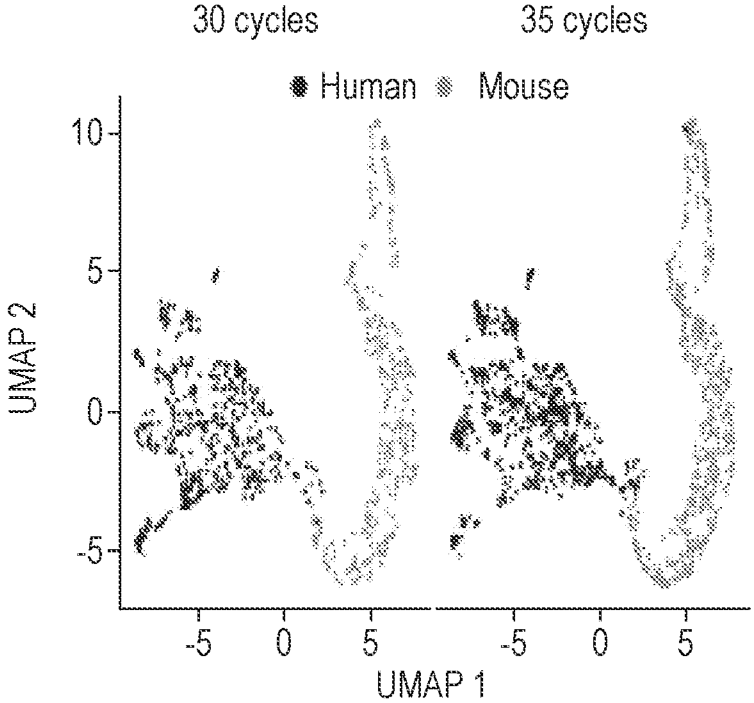


Fig. 24b

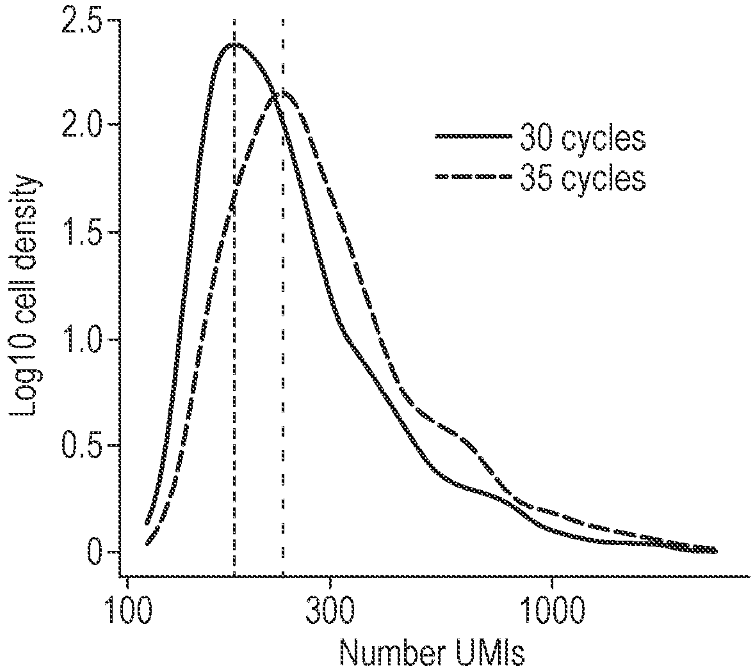

Fig. 25a

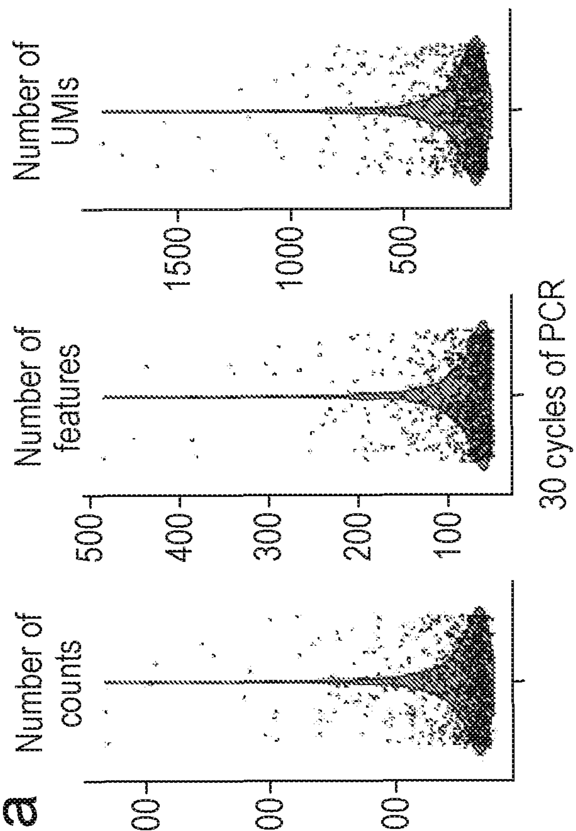


Fig. 25b

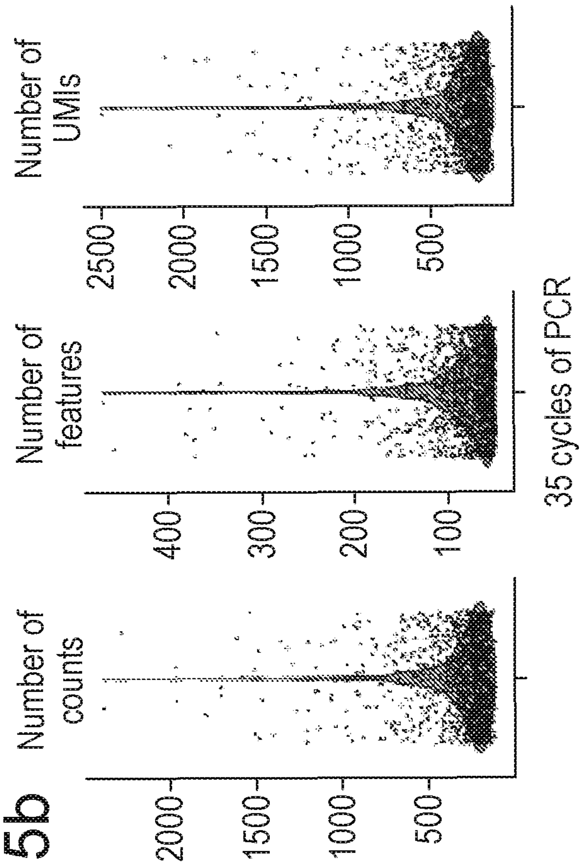


Fig. 25c

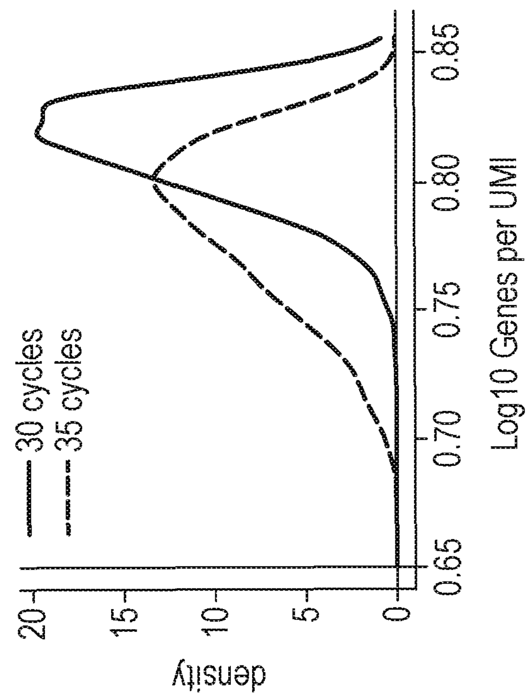

# Fig. 26a

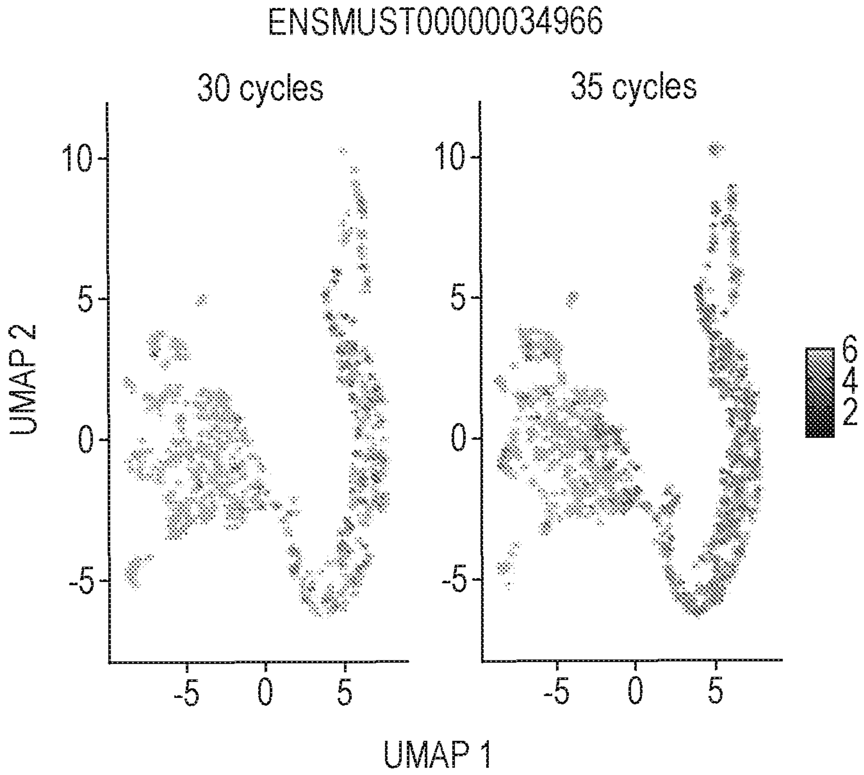


# Fig. 26b

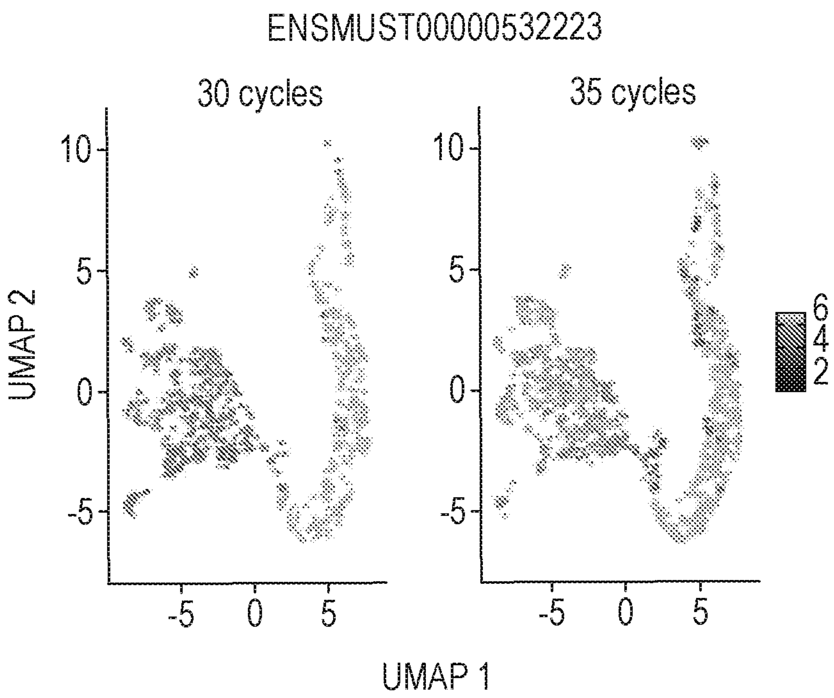

Fig. 27a

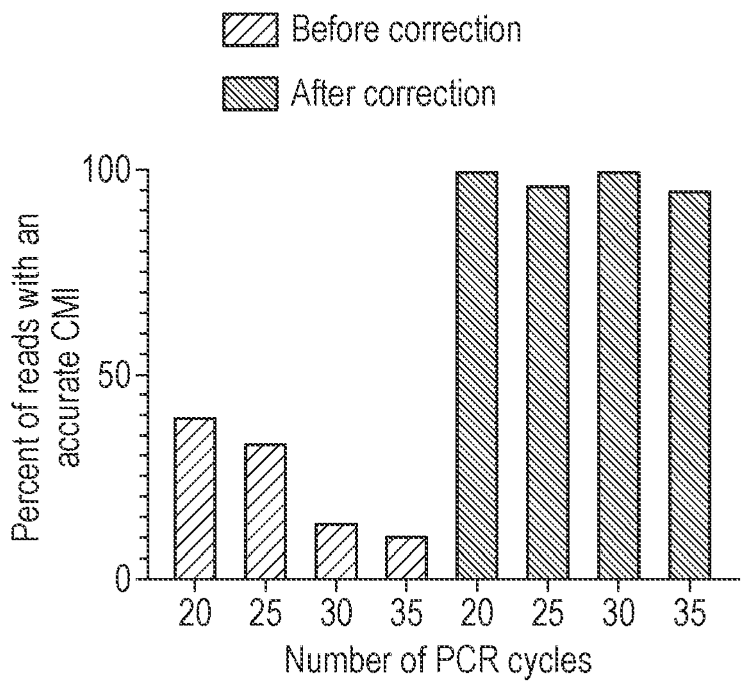


Fig. 27b

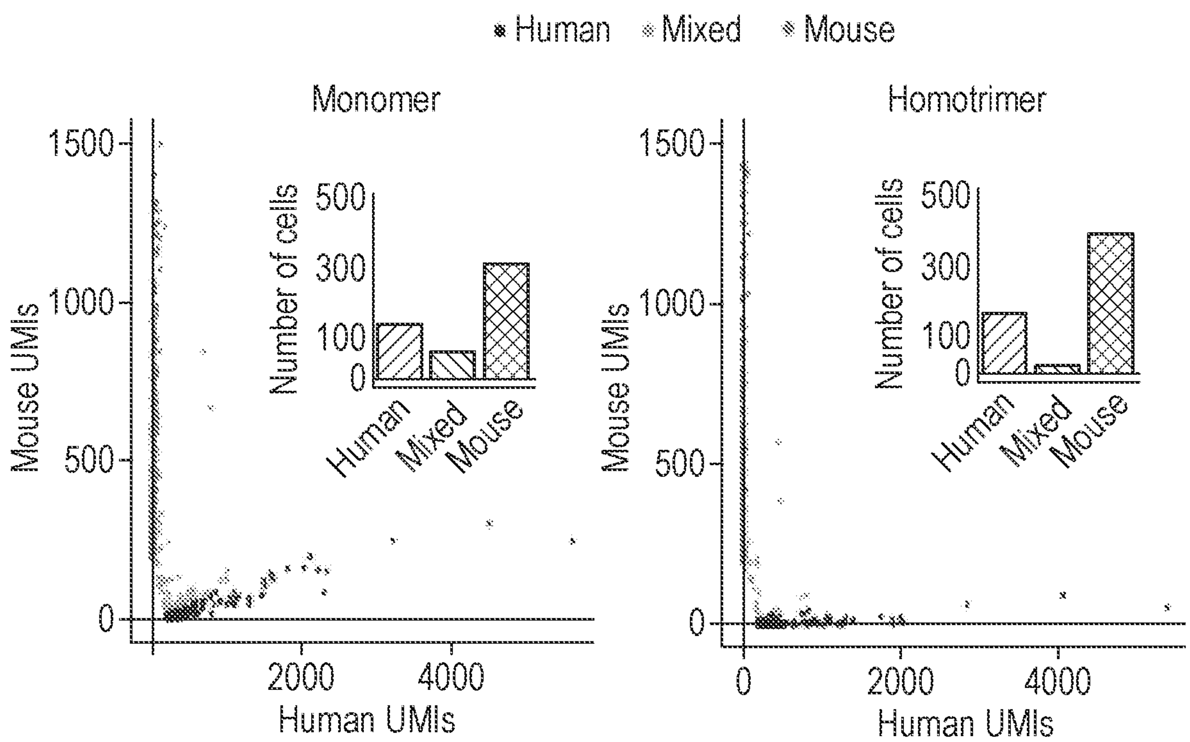

Fig. 28

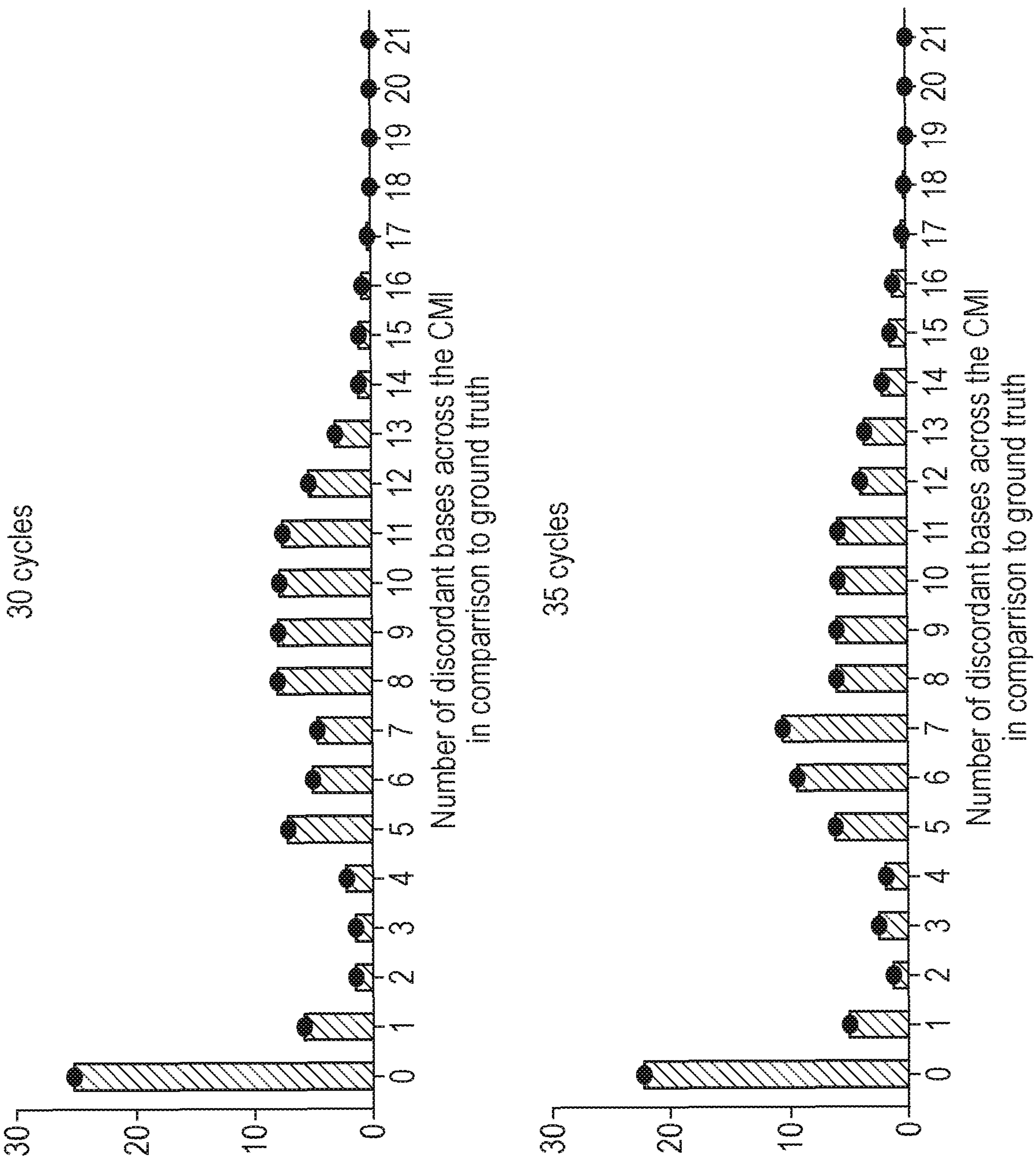

30 cycles
Percent of reads
30
20
10
0
0 1 2 3 4 5 6 7 8 9 10 11 12 13 14 15 16 17 18 19 20 21
Number of discordant bases across the CMI
in comparison to ground truth
35 cycles
Percent of reads
30
20
10
0
0 1 2 3 4 5 6 7 8 9 10 11 12 13 14 15 16 17 18 19 20 21
Number of discordant bases across the CMI
in comparison to ground truth

Fig. 29a

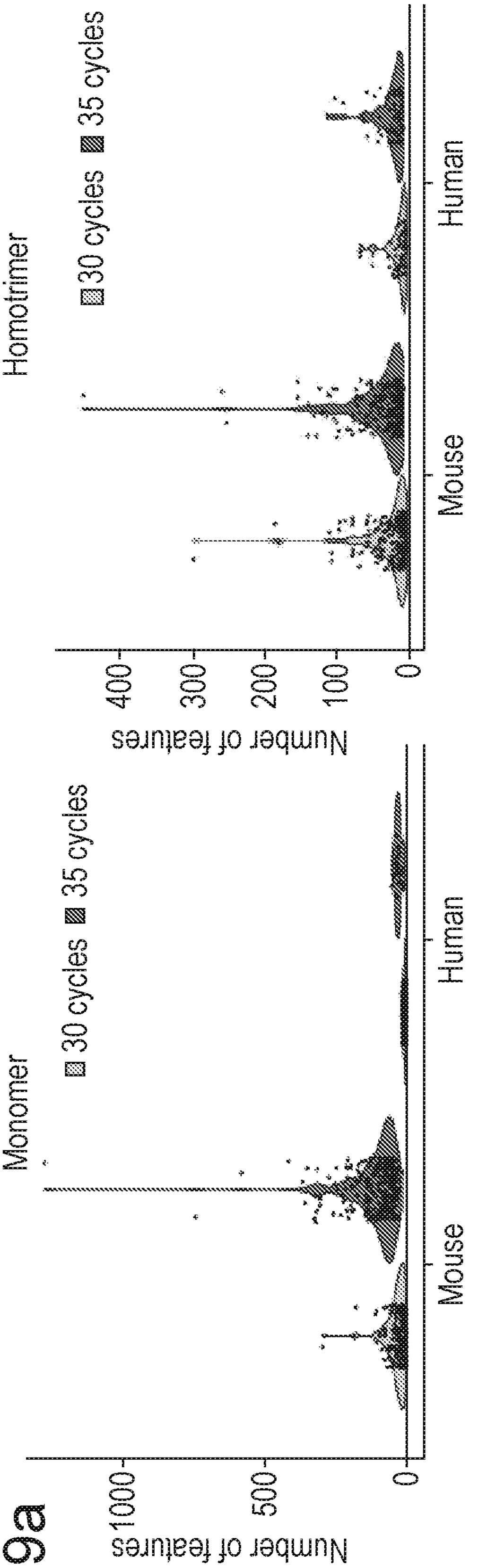


Fig. 29b

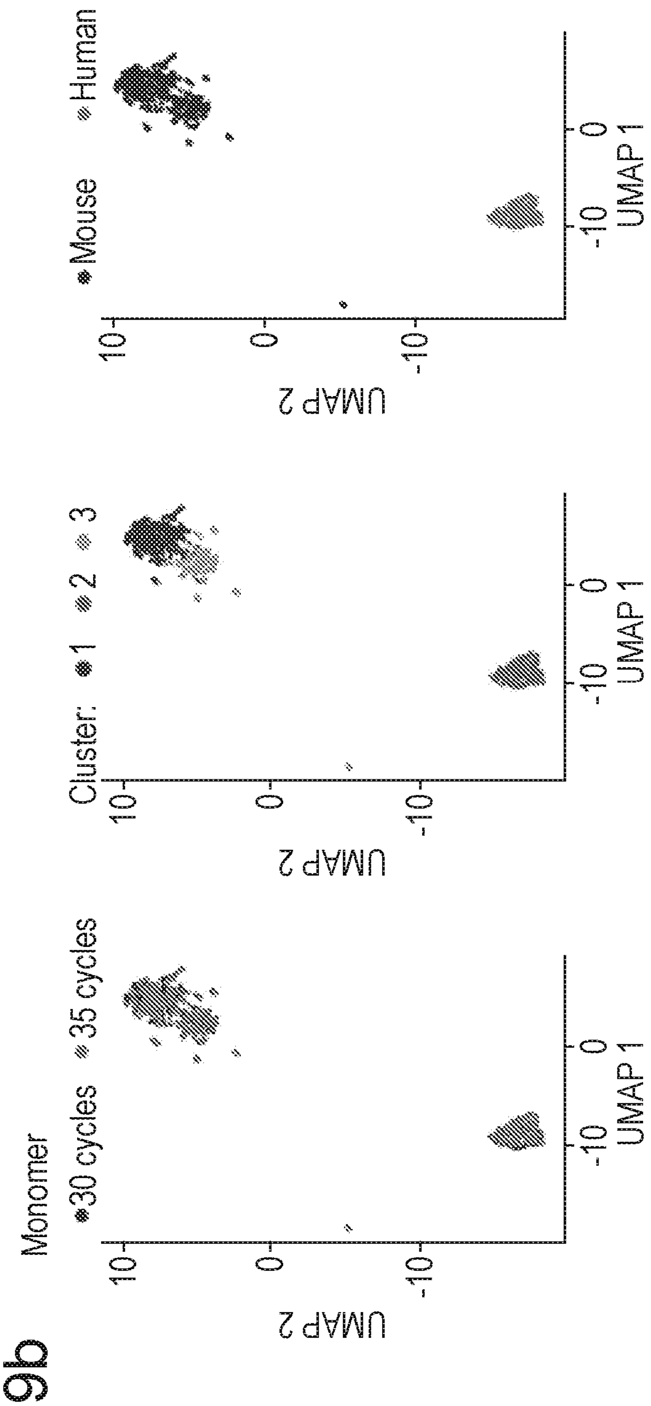

Fig. 29c

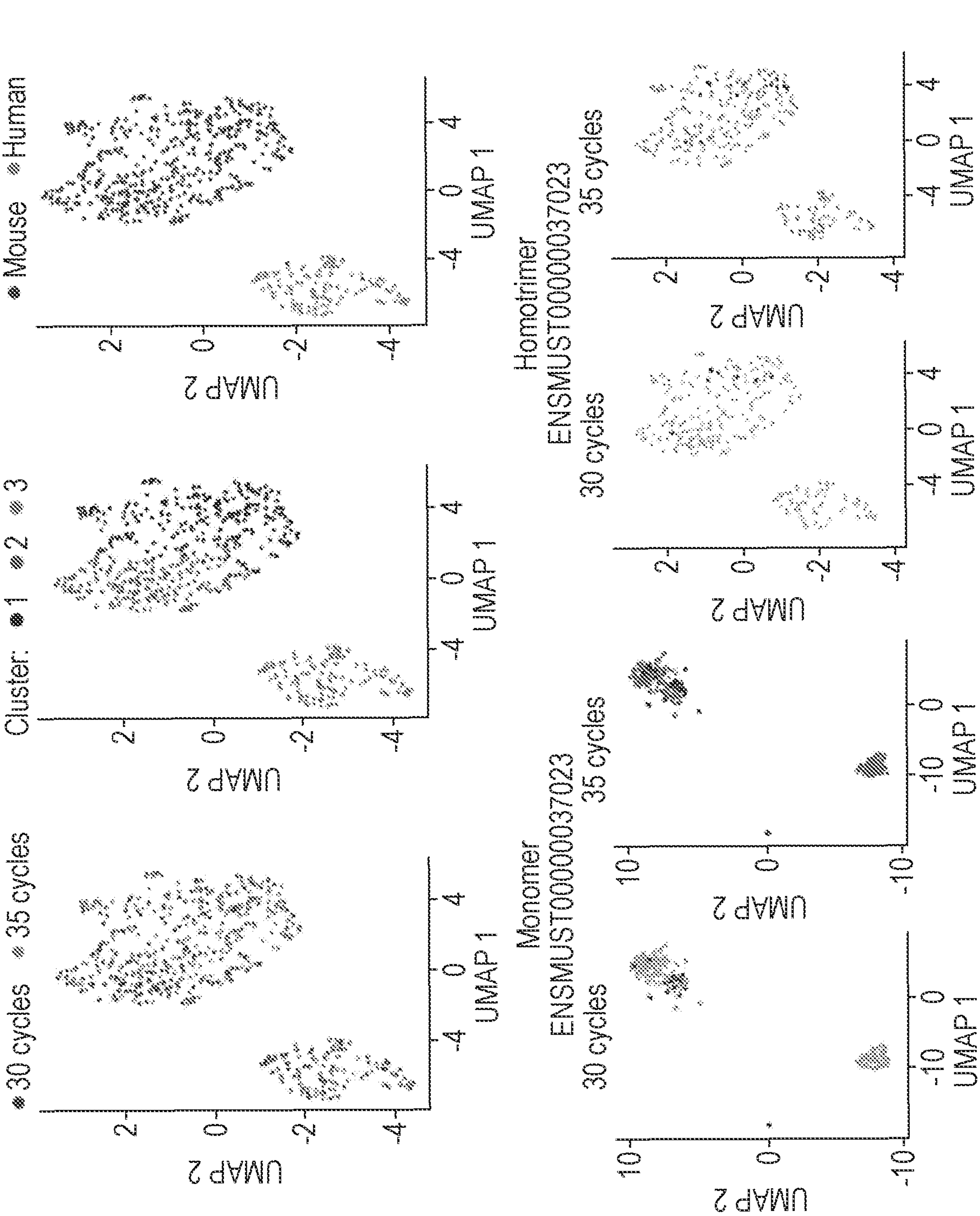
Homotrimer
30 cycles
35 cycles
Cluster:
1
2
3
Mouse
Human
UMAP 2
UMAP 1
Monomer
ENSMUST00000037023
Homotrimer
ENSMUST00000037023

Fig. 30a

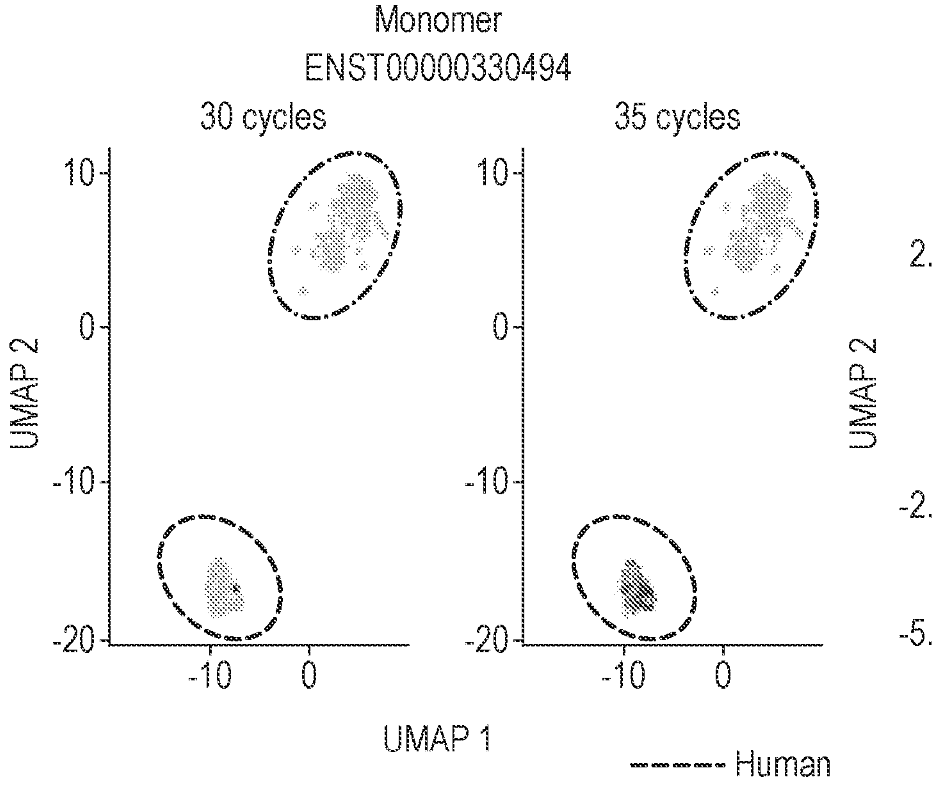


Fig. 30b

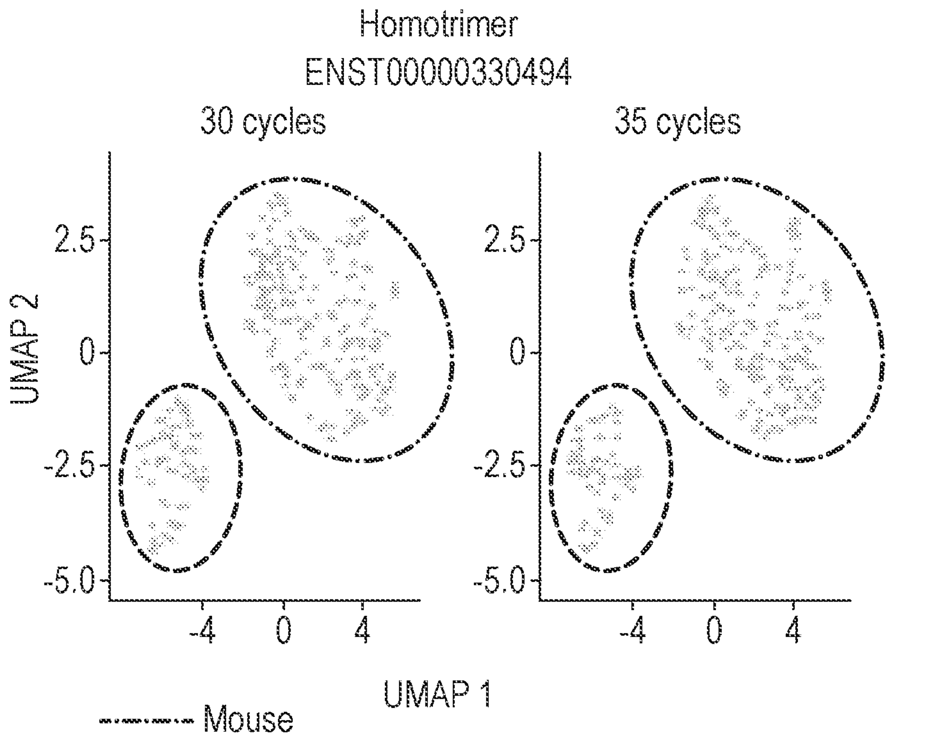

Fig. 31

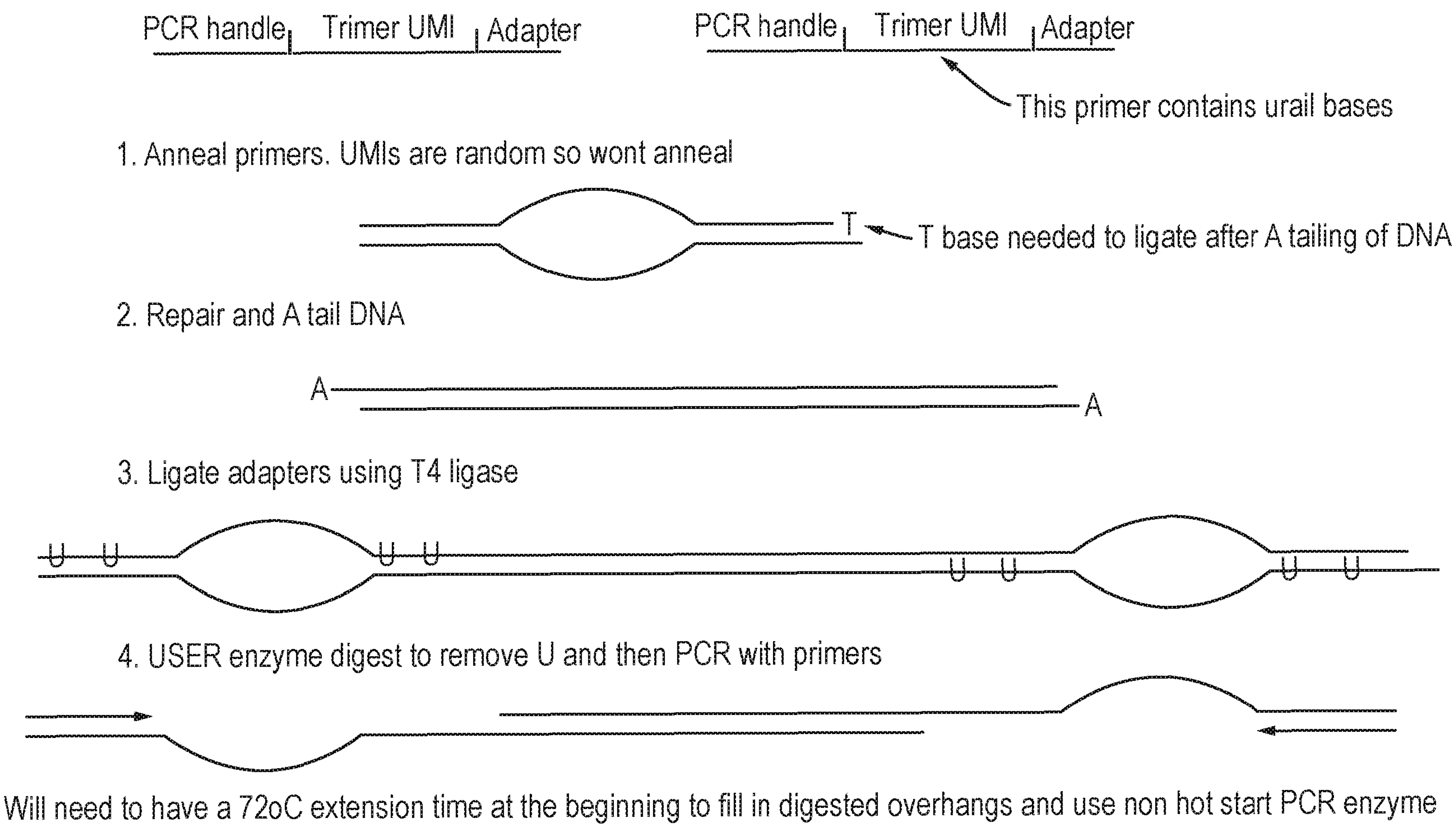
PCR handle
Trimer UMI
Adapter
PCR handle
Trimer UMI
Adapter
This primer contains urail bases
1. Anneal primers. UMIs are random so wont anneal
T
T base needed to ligate after A tailing of DNA
2. Repair and A tail DNA
A
A
3. Ligate adapters using T4 ligase
U
U
U
U
U
U
U
U
4. USER enzyme digest to remove U and then PCR with primers
Will need to have a 72oC extension time at the beginning to fill in digested overhangs and use non hot start PCR enzyme

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022118027 A
- WO 2021229230 A


### Non-patent literature cited in the description


- **BRAKENHOFF, R.H et al.** Chimeric cDNA clones: a novel PCR artifact. *Nucleic Acids Res*, 1991, vol. 19, 1949
- **RUANO, G** ; **KIDD, K.K**. Modeling of heteroduplex formation during PCR from mixtures of DNA templates. *PCR Methods Appl*, 1992, vol. 2, 112-116
- **LEBRIGAND, K et al.** High throughput error corrected Nanopore single cell transcriptome sequencing. *Nat Commun*, 2020, vol. 11, 4025
- **PHILPOTT, M et al.** Nanopore sequencing of single-cell transcriptomes with scCOLOR-seq. *Nat Biotechnol*, 2021, vol. 39, 1517-1520
- **KARST, S.M et al.** High-accuracy long-read amplicon sequences using unique molecular identifiers with Nanopore or PacBio sequencing. *Nat Methods*, 2021, vol. 18, 165-169
- **STOLTENBURG, R et al.** SELEX: A (r)evolutionary Method to Generate High-Affinity Nucleic Acid Ligands. *Biomol Eng*, 2007, vol. 24, 381-403
- **TUERK, C** ; **GOLD, L**. Systematic evolution of ligands by exponential enrichment: RNA ligands to bacteriophage T4 DNA polymerase. *Science*, 1990, vol. 249, 505-510
- **BOCK, L.C et al.** Selection of single-stranded DNA molecules that bind and inhibit human thrombin. *Nature*, 1992, vol. 355, 564-566
- **BEREZOVSKI, M et al.** Non-SELEX Selection of Aptamers. *J Am Chem Soc*, 2006, vol. 128, 1410-1411
- **SMITH, T et al.** UMI-tools: modeling sequencing errors in Unique Molecular Identifiers to improve quantification accuracy. *Genome Res*, 2017, vol. 27, 491-499
- **SMITH, T et al.** I. UMI-tools: modeling sequencing errors in Unique Molecular Identifiers to improve quantification accuracy. *Genome Res*, 2017, vol. 27, 491-499
- **J.A et al.** pRESTO: a toolkit for processing high-throughput sequencing raw reads of lymphocyte receptor repertoires. *Bioinformatics*, 2014, vol. 30, 1930-1932
- **COCQUET, J et al.** Reverse transcriptase template switching and false alternative transcripts. *Genomics*, 2006, vol. 88, 127-131
- **ZHU, Y.Y et al.** Reverse transcriptase template switching: a SMART approach for full-length cDNA library construction. *BioTechniques*, 2001, vol. 30, 892-897
- **MACOSKO, E.Z et al.** Highly Parallel Genome-wide Expression Profiling of Individual Cells Using Nanoliter Droplets. *Cell*, 2015, vol. 161, 1202-1214
- **CRIBBS, A et al.** CGAT-core: a python framework for building scalable, reproducible computational biology workflows [version 1; peer review: 1 approved, 1 approved with reservations. *F1000Research*, 2019, vol. 8
- **ANDREWS, S**. *FastQC: a quality control tool for high throughput sequence data*, 2010
- **EWELS, P et al.** MultiQC: summarize analysis results for multiple tools and samples in a single report. *Bioinformatics*, 2016, vol. 32, 3047-3048
- **LI, H et al.** The Sequence Alignment/Map format and SAMtools. *Bioinformatics*, 2009, vol. 25, 2078-2079
- **LIAO, Y et al.** featureCounts: an efficient general purpose program for assigning sequence reads to genomic features. *Bioinformatics*, 2014, vol. 30, 923-930
- **KIM, D et al.** Graph-based genome alignment and genotyping with HISAT2 and HISAT-genotype. *Nat Biotechnol*, 2019, vol. 37, 907-915
- **LI, H**. Minimap2: pairwise alignment for nucleotide sequences. *Bioinformatics*, 2018, vol. 34, 3094-3100